# FASCICULE DE BREVET EUROPEEN

(11) **EP 1 773 873 B1**
(45) Date de publication et mention de la délivrance du brevet: **13.03.2013**
(21) Numéro de dépôt: 05771139.2
(22) Date de dépôt: 04.05.2005
(51) Int. Cl.: C07K 14/31, G01N 33/569, A61K 39/085

(54) **NOUVEAUX POLYPEPTIDES POUR LA MISE EN EVIDENCE IN VITRO ET LA PREVENTION DES INFECTIONS STAPHYLOCOCCIQUES SUR PROTHESES ARTICULAIRES ET AUTRES MATERIALS ETRANGERS IMPLANTES**
NEUE POLYPEPTIDE ZUR IN-VITRO-ISOLIERUNG UND PRÄVENTION VON STAPHYLOKOKKENINFEKTIONEN AN GELENKPROTHESEN UND ANDEREN IMPLANTIERTEN FREMDKÖRPERN
NOVEL POLYPEPTIDES FOR ISOLATING IN VITRO AND PREVENTING STAPHYLOCCOCAL INFECTIONS ON JOINT PROSTHESES AND OTHER IMPLANTED FOREIGN MATERIALS

(30) Priorité: 08.06.2004 FR 0406154; 28.04.2005 FR 0504287
(43) Date de publication de la demande: 18.04.2007
(73) Titulaire: InGen Biosciences, 91380 Chilly Mazarin (FR)
(72) Inventeur: ARIE, Jean-Philippe, F-33000 BORDEAUX (FR)
(74) Mandataire: Vial, Lionel
(86) Numéro de dépôt international: PCT/FR2005/001120
(87) Numéro de publication internationale: WO 2006/005825

(56) Documents cités:
- WO-A-02/094868
- WO-A-02/094868
- WO-A-03/011899
- WO-A-03/011899
- US-A1- 2003 186 275
- US-A1- 2003 186 275
- US-A1- 2004 147 734
- US-B1- 6 380 370
- US-B1- 6 380 370
- US-B1- 6 703 492
- US-B1- 6 703 492
- DATABASE EMBL 22 juillet 1997 (1997-07-22), XP002299072 Database accession no. U71377
- DATABASE UniProt 7 juin 2004 (2004-06-07), XP002436345 Database accession no. Q8CQD9
- DATABASE WPI Section Ch, Week 200307 Derwent Publications Ltd., London, GB; Class B04, AN 2003-075410 XP002299074 & WO 02/059148 A2 (CISTEM BIOTECHNOLOGIES GMBH) 1 août 2002 (2002-08-01)
- DATABASE WPI Section Ch, Week 200133 Derwent Publications Ltd., London, GB; Class B04, AN 2001-316495 XP002299073 & WO 01/34809 A2 (GLAXO GROUP LTD) 17 mai 2001 (2001-05-17)
- DATABASE EMBL 22 July 1997 (1997-07-22), XP002299072 Database accession no. U71377
- DATABASE UniProt 1 March 2003 (2003-03-01), XP002347980 Database accession no. Q8CPQ1
- DATABASE WPI Section Ch, Week 200133 Derwent Publications Ltd., London, GB; Class B04, AN 2001-316495 XP002299073 & WO 01/34809 A2 (GLAXO GROUP LTD) 17 May 2001 (2001-05-17)
- DATABASE WPI Section Ch, Week 200307 Derwent Publications Ltd., London, GB; Class B04, AN 2003-075410 XP002299074 & WO 02/059148 A2 (CISTEM BIOTECHNOLOGIES GMBH) 1 August 2002 (2002-08-01)

## Description

Nouveaux polypeptides pour la mise en évidence *in vitro* et la prévention des infections staphylococciques sur prothèses articulaires et autres matériels étrangers implantés.

La présente invention concerne un outil de diagnostic sérologique des infections staphylococciques sur prothèses articulaires (par exemple : infection sur prothèse de hanche, coude, genou, cheville,...) et, plus généralement, des infections staphylococciques sur matériel étranger implanté dans le corps.

L'invention comprend, en particulier, l'identification de nouveaux polynucléotides et polypeptides, leur production, leur optimisation et leur utilisation, d'une part, dans le domaine du diagnostic des infections sur matériel étranger (prothèses articulaires par exemple) impliquant *Staphylococcus aureus* et/ou *Staphylococcus epidermidis* et, d'autre part, dans le domaine de la production de vaccins vis-à-vis de ces différents agents.

Les infections sur prothèses articulaires constituent un problème majeur de santé publique. Des centaines de milliers de prothèses articulaires sont posées annuellement dans le monde et des millions de personnes en portent une ou plusieurs (Lew et Waldvogel. 1997. Osteomyelitis. N Engl J Med 336:999-1007). Aux Etats-Unis, on estime qu'environ 430 000 prothèses totales de hanche (en abrégé, PTH) et de genou (en abrégé, PTG) sont posées chaque année (Berbari et al., 1998, Clin Infect. Dis., 27:1247-1254). En Norvège et en Suède, seuls pays où a été mis en place un registre des prothèses, 250 000 PTH ont été posées en 10 ans, entre 1987 et 1996 (Lidgren. 2001. Joint prosthetic infections: a success story. Acta Othop Scand 72:553-556). En France, d'après la Société Française d'Orthopédie, le nombre de. prothèses posées par an avoisiné 100 000 pour les PTH et 25 000 pour les PTG (Agence nationale d'accréditation et d'évaluation en santé, 2000). Ces chiffres devraient encore progresser dans le futur du fait du vieillissement des populations (pose de PTH) et des problèmes de surpoids (pose de PTG) dans les pays industrialisés.

Malgré des progrès considérables enregistrés au cours de ces dernières années, les infections sur prothèses articulaires (en abrégé, IPA) restent une complication fréquente, avec des taux de 0,3 à 1,8% pour les PTH (Berbari et al., 1998 [déjà cité]; Lidgren, 2001 [déjà cité]) et de 0, 5 à 5% pour les PTG (Johnson et Bannister. 1986. The outcome of infected arthroplasty of the knee. J Bone Joint Surg 68:289-291 ; Bengtson et Knutson. 1991. The infected knee arthroplasty. A 6-year follow up of 357 cases. Acta Othop Scand 62:301-311 ; Eveillard *et al.* 2002. Risque infectieux après implantation de prothèses de genou. Etude des infections profondes pour une série continue de 210 prothèses totales de genou en première intention. *B E H* n°13) . Les taux sont encore plus élevés lorsqu'il s'agit de reprises. L'infection peut survenir dans un délai très variable après la pose de la prothèse. Dans une grande étude américaine ayant porté sur plus de 25 000 patients porteurs d'une PTH ou d'une PTG, le délai moyen entre la pose de la prothèse et le diagnostic d'infection était de 512 jours, avec des délais extrêmes de 3 jours à près de 20 ans, et la distribution des délais était la suivante : 20% dans les 3 mois après la pose, 40% entre 3 mois et 2 ans, et 40% au-delà de 2 ans (Berbari et al., 1998 [déjà cité]) .

L'IPA est une complication redoutable, ayant pour conséquences la nécessité d'une ou plusieurs ré-interventions associées à une antibiothérapie au long cours, mais aussi un handicap fonctionnel prolongé, un risque d'amputation, voire de décès (Segawa. et al. 1999. Infection after total knee arthroplasty. J Bone Joint Surg Am 81:1434-1445). L'impact socio-économique d'une IPA est également très important, avec un coût estimé de plus de 50 000 dollars par épisode (Sculco. 1995. The economic impact of infected joint arthroplasty. Orthopedies 18:871-873).

### Les staphylocoques

Les bactéries du genre *Staphylococcus* sont des coques (cocci) à Gram positif, groupés en amas ayant la forme de grappes de raisin, immobiles, non sporulés, catalase positive et oxydase négative. Observés par Pasteur en 1879 dans un pus de furoncle, les staphylocoques doivent leur nom à Ogston (1881) qui les a mis en évidence dans des abcès aigus et chroniques.

On distingue généralement *S*. *aureus* (plus communément appelé staphylocoque doré) des autres espèces de staphylocoques. A la différence de S. aureus, ces dernières espèces ne produisent pas d'enzyme de type coagulase et sont, pour cette raison, regroupées sous la dénomination "staphylocoques à coagulase négative" (en abrégé, SCN).

### Habitat des staphylocoques

Le réservoir naturel de *S. aureus* est l'homme et les animaux à sang chaud. Le site de colonisation préférentielle de *S. aureus* chez l'homme est la muqueuse nasale : jusqu'à 30% des adultes hébergent S. aureus de façon permanente dans leurs narines, et 50% de façon intermittente. A partir de ce site de portage, *S. aureus* colonise la peau et, en particulier, les zones humides (aisselles, périnée) et les mains. *S. aureus* est aussi retrouvé en petites quantités dans l'intestin. Enfin, l'environnement immédiat de l'homme est également une source de contamination potentielle, du fait de la persistance de *S. aureus* après son élimination dans le milieu extérieur.

Les SCN représentent les principaux commensaux de la peau avec les corynébactéries et les propionibactéries. La densité de colonisation est plus importante au niveau des zones humides comme la partie antérieure des narines, le périnée, les creux axillaires et les plis inguinaux. La transmission intra- ou interhumaine s'opère généralement par contact direct (manuportage). Plus rarement, elle peut être indirecte à partir d'une source environnementale (vêtements, draps, matériels médicaux).

Présents normalement et en grandes quantités au niveau de la peau et des muqueuses, *S. epidermidis* et les autres SCN sont susceptibles de contaminer les prélèvements superficiels ou les prélèvements obtenus par ponction transcutanée comme les hémocultures, voire les prélèvements per-opératoires profonds réalisés au cours d'un geste chirurgical. De ce fait, on ne retient généralement la responsabilité d'un SCN au cours d'une infection que lorsque cette bactérie a été retrouvée à plusieurs reprises à partir de prélèvements réalisés de façon indépendante.

### Rôle des staphylocoques en pathologie humaine

*S. aureus* est responsable d'infections très diverses. Les plus fréquentes sont les infections cutanéo-muqueuses telles que folliculites, impétigo, furoncles, anthrax, panaris, cellulites, sinusites ou otites. Ces infections peuvent se compliquer par extension loco-régionale ou par diffusion hématogène des bactéries. *S. aureus* est ainsi à l'origine de septicémies, d'endocardites, de pneumopathies, d'ostéomyélites, d'arthrites et de méningites. Ces infections peuvent engager le pronostic vital par elles-mêmes (par exemple : atteinte d'une valve cardiaque) ou en cas de choc toxique associé.

*S. aureus* a longtemps été considéré comme le seul staphylocoque pathogène chez l'homme, tandis que les SCN étaient considérés comme de simples contaminants. Ce n'est que récemment qu'a été établi le rôle majeur des SCN en pathologie humaine, en particulier chez des patients porteurs de prothèses articulaires, de valves cardiaques artificielles ou de dispositifs implantables tels que cathéters vasculaires ou shunts de dérivation du liquide céphalo-rachidien.

*S. epidermidis* est, de loin, le SCN le plus fréquemment isolé dans les infections sur matériel étranger. Parmi les autres SCN en cause dans ce type d'infections, on peut citer *S. capitis, S. caprae, S. haemolyticus, S. lugdunensis, S. schleiferi, S. simulans* et *S. warneri.*

Les staphylocoques sont les principaux agents des IPA et des autres infections sur matériels étrangers.

Les staphylocoques sont les germes les plus fréquemment retrouvés dans les IPA et les autres infections sur matériels étrangers, puisqu'ils représentent jusqu'à 75% des bactéries isolées, *S. aureus* et *S. epidermidis* étant les espèces prévalentes (Lew et Waldvogel. 1997 [déjà cité]). Le groupe des SCN, au premier rang desquels figure *S. epidermidis,* est à lui seul responsable de 20 à 40% des cas et se range en 1^{ère} ou en 2^{ème} position derrière *S. aureus,* selon les études.

En dehors de *S. epidermidis,* les SCN prévalents sont *S. capitis*, *S. caprae*, *S*. *haemolyticus, S. lugdunensis, S. schleiferi, S. simulas* et *S. warneri.* Parmi ces espèces, *S. capitis, S. caprae* et *S lugdunensis*, sont les principaux SCN autres que *S. epidermidis* responsables d'infections sur prothèses articulaires et, plus généralement, d'infections ostéo-articulaires sur matériel étranger (prothèses articulaires, matériels d'ostéosynthèse) (Rupp et Archer. 1994. Coagulase-negative staphylococci: pathogens associated with medical progress. Clin Infect Dis 19:231-243 ; Crichton et al. 1995. Subspecies discrimination of staphylococci from revision arhtroplasties by ribotyping. J Hosp Infect 30:139-147; Blanc et al. 1999. Infection after total hip replacement by Staphylococcus caprae. Case report and review of the literature. Pathol Biol 47:409-413 ; Sampathkurnar et al. 2000. Prosthetic joint infection due to Staphylococcus lugdunensis. Mayo Clinic Proc 75:511-512; Weightman et al. 2000. Bone and prosthetic joint infection with Staphylococcus lugdunensis. J Infect 40:98-99).

D'une façon générale, les IPA et autres infections sur matériel étranger dues à *S. aureus* sont plus souvent aiguës et suppuratives, en rapport avec la production de nombreuses enzymes et toxines par cette espèce (Nair et al. 2000. Advances in our understanding of the bone and joint pathology caused by Staphylococcus aureus infection. Rheumatology (Oxford) 39:821-834), tandis que les infections à SCN évoluent à bas bruit et sont plutôt chroniques (Von Eiff et al. 2002. Pathogenesis of infections due to coagulase-negative staphylococci. Lancet Infect Dis 2:677-585). Cependant, cette règle est loin d'être toujours respectée : les infections à *S. aureus* prennent souvent un aspect chronique, alors que les infections dues à des SCN tels que *S. lugdunensis* peuvent évoluer sur un mode aigu (Sampathkumar *et al*. 2000 [déjà cité]; Weightman *et al.* 2000 [déjà cité]).

### Particularités des infections staphylococciques sur matériel étranger avec formation de biofilm

Les infections staphylococciques sur matériel étranger se distinguent des infections habituelles par l'organisation des bactéries sous forme de biofilm (Costerton, et al. 1999. Bacterial biofilms: a common cause of persistent infections. Science 284:1318-22). Ce processus a été décrit chez de nombreux staphylocoques incluant *S. aureus,* mais a surtout été étudié chez *S. epidermidis* (Von Eiff *et al.* 2002 [déjà cité]).

Le biofilm est un édifice tri-dimensionnel complexe associé au matériel étranger et où les cellules bactériennes sont enveloppées dans une matrice extracellulaire de nature polysaccharidique appelée *slime* ou glycocalyx (Davey et O'Toole. 2000. Miorobial biofilms: from ecology to molecular genefics. Microbiology and Molecular Biology Reviews 64:847-867). Cette structure particulière peut être formée de bactéries provenant de la même espèce ou d'espèces différentes (Costerton et al. 1995. Microbial biofilms. Ann Rev Med 49:711-745 ; Watnick et Kolter. 2000. Biofilms, city of microbes. J Bacteriol 182:2675-2679). En comparaison de leurs congénères vivant sous forme libre (ou "planctonique"), ces bactéries sont dans un état de quiescence marqué par une faible activité métabolique (Yao et al. 2005. Genomewide analysis of gene expression in Staphylococcus epidermidis biofilms: insights into the pathophysiology of S. epidermidis biofilms and the role of phenol-soluble modulins in formation of biofilms. J Infect Dis 191:289-298).

Les infections sur matériel étranger associées à la formation de biofilm présentent un certain nombre de caractéristiques qui les distinguent totalement des infections tissulaires classiques. Ces infections sont le plus souvent pauci-bactériennes (faible nombre de bactéries) et volontiers poly-microbiennes (association de plusieurs espèces ; par exemple : *S. aureus* et *S. epidermidis* ou *S*. *epidermidis* et un ou plusieurs autres SCN (Costerton *et al.* 1995 [déjà cité] ; Watnick et Kolter. 2000 [déjà cité]). Les bactéries ont un métabolisme très ralenti qui les maintient dans un état proche de la dormance, et les gènes qu'elles expriment sont différents de ceux mis en action chez les formes planctoniques (Yao *et al. 2005* [déjà cité]). L'état de dormance des bactéries et la présence du biofilm réduisent considérablement la réaction inflammatoire et l'attraction des cellules immunitaires au site de l'infection (Vuong et al. 2004. Crucial role for exogolysaccharide modification in bacterial biofilm formation, immune evasion, and virulence. J. Biol. Chem. 279, 52:54881-54886 ; Fux et al. 2003. Bacterial biofilm: a diagnostic and therapeutic challenge. Expert Rev Anti Infect Ther. 1:667-83). Enfin, pour les mêmes raisons, les bactéries sont en grande partie protégées de l'action des antibiotiques (Costerton *et al*. 1995 [déjà cité]).

### Méthodes actuelles de diagnostic des infections staphylococciques au laboratoire

### Diagnostic bactériologique

Le diagnostic bactériologique d'infection staphylococcique est, aujourd'hui, quasi exclusivement direct, par mise en évidence de la bactérie à partir de prélèvements pertinents.

Le diagnostic repose sur les principales étapes suivantes :
(1) Le prélèvement : aseptique (pour diminuer le risque de contamination par un simple staphylocoque commensal de la peau) et en dehors de toute antibiothérapie (après arrêt ou avant le début du traitement antibiotique).
(2) L'examen microscopique permet la recherche de cocci réguliers, à Gram positif, groupés en amas. Cet examen est très peu sensible et ne fournit aucune indication sur l'espèce en cause.
(3) La culture sur gélose ordinaire dans la majorité des cas ou sur milieu de culture sélectif, type milieu de CHAPMAN (qui contient 7 % de NaCl, du mannitol et un indicateur de pH) si le prélèvement est potentiellement contaminé par d'autres bactéries.
(4) L'identification de la bactérie repose sur la mise en évidence des caractères suivants :
   - catalase (différence avec le streptocoque),
   - fermentation du glucose en anaérobiose (différence avec le microcoque),
   - coagulase (différence avec les SCN),
   - DNase thermostable (qui signe l'espèce *S*. *aureus*),
   - minigalerie d'identification (manuelle ou automatisée) (identification des SCN au rang d'espèce),
   - éventuellement, identification moléculaire (par exemple : séquençage du gène sodA) (identification des SCN au rang d'espèce).
(5) Le diagnostic est complété par la mesure de la sensibilité aux antibiotiques (antibiogramme) étant donné la fréquence des résistances chez les staphylocoques, notamment chez les souches hospitalières. Le profil de sensibilité aux antibiotiques est également un élément intéressant, bien qu'imparfait, dans un but de comparaison de souches entre elles (pour un véritable typage, il est nécessaire de recourir à des techniques moléculaires de type électrophorèse en champs pulsés ou "multilocus séquence typing").

Le problème majeur concerne l'interprétation du résultat des cultures, puisque les staphylocoques sont des contaminants fréquents. La règle généralement appliquée est la suivante pour établir un diagnostic d'infection :
- *S. aureus :* au moins un prélèvement pertinent positif (par exemple : une hémoculture),
- SCN : au moins deux prélèvements pertinents et indépendants (par exemple : deux hémocultures prélevées à deux moments différents) positifs au même germe (c'est à dire, en pratique courante, une bactérie de même espèce et de même sensibilité aux antibiotiques).

Ce seuil de deux prélèvements positifs pour les SCN a été récemment remis en question dans le cadre des IPA car jugé trop peu spécifique, et certains auteurs retiennent désormais un seuil de trois prélèvements per-opératoires positifs pour faire un diagnostic d'IPA à SCN (Atkins et al. 1998. Prospective evaluation of criteria for microbiological diagnosis of prosthetic-joint infection at révision arthroplasty. J Clin Microbiol 36:2932-2939).

### Diagnostic sérologique

Le diagnostic indirect d'infection staphylococcique par recherche des anticorps circulants est aujourd'hui très peu développé et ne concerne que le diagnostic de certaines infections sévères à *S. aureus* hors matériel étranger : endocardites, septicémies, infections ostéo-articulaires hématogénes (Sôderquist et al. 1993. Staphylococcal a-toxin in septicaemic patients; detection in serum, antibody response and production in isolated strains. Serodiagn Immunother Infect Dis 5:139-144 ; Nordin et al. 1995. Antibody response in patients with osteomyelitis of the mandible. Oral Surg Oral Med Oral Pathol Oral Radiol Endod 79:429-435 ; Kanclerski et al. 1996. Serum antibody response to Staphylococcus aureus enterotoxins and TSST-1 in patients with septicaemia. J Med Microbiol 44:171-177 ; Colque-Navarro et al. 1998. Antibody response in Staphylococcus aureus septicemia - a prospective study. J Med Microbiol 47:217-225 ; Colque-Navarro & Mölby. 1999. Usefulness of staphylococcal serology. J Med Microbiol 48:107-109 ; Ellis et al. 2003. Role of staphylococcal enterotoxin A in a fatal case of endocarditis. J Med Microbiol 52:109-112).

Les tests actuels reposent sur la recherche d'anticorps dirigés contre des antigènes spécifiques de *S. aureus* ou produits à partir de *S. aureus :* anticorps anti-α-toxine (ou antistaphylolysines alpha), anti-acide β-ribitol téichoïque, anti-entérotoxines et anti-toxine 1 de choc toxique staphylococcique (anti-TSST1), et anticorps anti-capsulaires ; la détection des anticorps se fait par inhibition d'hémolyse, électrosynérèse ou ELISA (Bornstein et al. 1992. Immune response to staphylococcal toxins and ribitol teichoic acid in Staphylococcus aureus infections. Med Microbiol Lett 1:111-119 ; Christensson et al. 1993. Diagnosing Staphylococcus aureus endocarditis by detecting antibodies against S. aureus capsular polysaccharides types 5 and 8. J Infect Dis 163:530-533 ; Kanclerski *et al*, 1996 [déjà cité]). Ces tests sont globalement peu sensibles et peu spécifiques, et donnent des résultats variables selon les souches en cause, notamment en fonction des toxines qu'elles produisent (par exemple : réponse plus forte pour les souches entérotoxines B et/ou C positives que pour les souches entérotoxine A et/ou TSST1 positives) (Kanclerski *et al*. 1996 [déjà cité]). Enfin, il n'existe pas de tests permettant d'identifier la classe des anticorps produits, et notamment s'il s'agit d'IgG ou d'IgA.

Aussi bien *S. aureus* que *S*. *epidermidis* ou les autres SCN sont des bactéries commensales fréquentes de l'homme (membrane nasale pour *S*. *aureus* et peau pour *S. epidermidis* et les autres SCN) et, identifier des antigènes révélant un processus infectieux est difficile. Cette difficulté est attestée par des études dans le laboratoire de la Déposante où de nombreux antigènes staphylococciques ne sont pas des marqueurs d'infection, les témoins répondant dans les mêmes proportions que les malades avérés.

### Diagnostic et suivi des IPA staphylococciques

Il est essentiel de pouvoir diagnostiquer une IPA avec certitude. En effet, le traitement d'une IPA, lourd et coûteux, est associé à un retentissement fonctionnel important et n'est pas dénué de risque vital. En général, le traitement consiste en un débridement chirurgical extensif et méticuleux associé à une antibiothérapie adaptée et prolongée ; les principales options chirurgicales sont l'échange en un temps de la prothèse, le débridement avec rétention de la prothèse et la réimplantation de la prothèse en deux temps, ce qui nécessite des temps d'hospitalisation plus longs (Garvin et Hanssen. 1995. Current concepts review. Infection after total hip arthroplasty. Past, présent, and future. J Borce Joint Surg 77-A:1576-1588). La mortalité associée à l'intervention chirurgicale pour IPA est de 0,4 à 1,2% pour les patients de 65 ans et de 2 à 7% pour les patients de 80 ans (Lentino. Prosthetic joint infections: bane of orthopedists, challenge for infectious disease specialists. Clin Infect Dis 36:1157-1161).

Le risque d'échec infectieux est élevé après réintervention sur IPA, allant en moyenne de 10 à 40% selon la localisation, la gravité des lésions et le type de traitement chirurgical. L'échec n'est pas toujours infectieux et, d'autre part, peut impliquer un autre germe que celui à l'origine du premier épisode d'IPA (germe associé non retrouvé dans les prélèvements per-opératoires ou germe inoculé accidentellement au cours de la reprise). Un test de suivi sérologique permettrait de faire un diagnostic précoce d'échec, en en précisant la nature infectieuse et le germe en cause.

En dehors du cas des IPA aiguës ou suraiguës, il n'existe pas, à l'heure actuelle, de tests permettant de faire un diagnostic pré-opératoire fiable d'IPA et, en particulier, de différencier une IPA d'un descellement aseptique, qui est traité par un simple échange de prothèse en un seul temps et qui ne nécessite aucune antibiothérapie. Les signes cliniques sont trompeurs : douleur et inflammation ne sont pas spécifiques d'infection, et la fièvre est le plus souvent absente. La radiographie simple est peu sensible et les anomalies signant l'infection d'apparition est tardive. Les marqueurs biologiques d'inflammation, tels que vitesse de sédimentation (en abrégé, VS) ou protéine C réactive (en abrégé, CRP), sont peu sensibles et peu spécifiques.

Aujourd'hui, le diagnostic de certitude d'IPA ne peut donc être établi que par la culture de prélèvements per-opératoires ou de liquide d'aspiration impliquant un geste chirurgical sous anesthésie générale (dans certaines situations, il est également possible de réaliser des prélèvements de type "true-cut" sans abord chirurgical, mais sous anesthésie générale).

Cette stratégie présente plusieurs défauts :
- il n'est pas possible, à l'heure actuelle, de faire un diagnostic d'IPA pré-opératoire, et encore moins de connaître le ou les germes en cause,
- les techniques de mise en culture des prélèvements per-opératoires ne sont pas standardisées et des études récentes montrent que les techniques usuelles manquent de sensibilité (Tunney et al. 1999. Détection of prosthetic hip infection at revision arthroplasty by immunofluorescence microscopy and PCR amplification of the bacterial 16S rRNA gene. J Clin Microbiol 37:3281-3290),
- les délais de culture sont particulièrement longs dans les formes chroniques,
- l'interprétation du résultat des cultures reste controversé : le seuil d'au moins trois prélèvements indépendants positifs proposé par le groupe OSIRIS d'Oxford offre une excellente spécificité (Atkins *et al*. 1998 [déjà cité]), mais pourrait manquer de sensibilité, notamment en cas de préparation sub-optimale des échantillons.

Aucune des méthodes de diagnostic actuelles n'a recours à l'utilisation d'antigène purifié pour mettre en évidence une infection staphylococcique sur prothèse articulaire et les méthodes sérologiques actuelles ne permettent ni de réaliser des suivis thérapeutiques ni de réaliser des diagnostics post-implantatoires.

Il existe donc, dans ce domaine, un très grand besoin en une technique de diagnostic sérologique afin de réaliser des tests rapides, peu coûteux et non invasifs. Idéalement ces tests devraient permettre d'identifier le germe ou les germes en cause et, en particulier, devraient permettre de distinguer *S*. *aureus,* dont le pouvoir de destruction tissulaire est très important, des autres staphylocoques.

L'utilisation de ces tests pourrait également permettre un diagnostic précoce d'IPA et, donc, une prise en charge chirurgicale moins délabrante à un stade d'infection débutante. Ceci pourrait être réalisé par un suivi sérologique longitudinal, c'est-à-dire un suivi sur une période de temps substantielle, des patients après pose de prothèse (diagnostic post-implantatoire), notamment chez les patients présentant un risque élevé d'IPA.

Enfin, dans l'avenir, en cas de développement d'approches vaccinales visant à prévenir les IPA staphylococciques notamment, mais non exclusivement, celles dues à *S*. *aureus,* de tels tests seront indispensables pour détecter les échecs de vaccination.

La présente invention a pour objectif de résoudre les problèmes posés par l'absence de tests sérologique permettant la mise en évidence d'une infection à staphylocoques sur matériel étranger, notamment sur prothèse articulaire, par détection d'anticorps circulants.

Elle a, en outre, pour objectif de permettre la détection d'anticorps circulants vis-à-vis de bactéries qui se trouvent en état de dormance et protégées du système immunitaire par leur présence au sein d'un biofilm. Elle a, de plus, pour objectif de permettre le diagnostic d'infections staphylococciques poly-microbiennes (c'est à dire impliquant plusieurs espèces différentes).

L'invention a, pour objectif supplémentaire, de permettre une analyse plus précise et plus complète grâce à la détection des différents isotypes d'anticorps spécifiques des polypeptides.

### Définitions

Les définitions suivantes sont données afin de faciliter la compréhension de certains termes utilisés dans cette description.

On entend par "polynucléotide", un polyribonucléotide ou un polydésoxyribonucléotide qui peut être un ADN ou un ARN modifié ou non.

Le terme polynucléotide inclut, sans limitation, un ADN simple brin ou double brin, un ADN composé d'un mélange d'une ou plusieurs région(s) simple brin et d'une ou plusieurs région(s) double brin, un ADN qui est un mélange de régions simple brin, double brin et/ou triple brin, un ARN simple brin ou double brin, un ARN composé d'un mélange d'une ou plusieurs région(s) simple brin et d'une ou plusieurs région(s) double brin et les molécules hybrides comprenant un ADN et un ARN qui peuvent comprendre des régions simple brin, double brin et/ou triple brin ou un mélange de régions simple brin et double brin. Le terme polynucléotide peut aussi comprendre un ARN et/ou un ADN comprenant une ou plusieurs régions triple brin. Les brins dans de telles régions peuvent provenir de la même molécule ou de molécules différentes. Par conséquent, les ADN ou ARN avec des squelettes modifiés pour la stabilité ou d'autres raisons sont inclus dans le terme polynucléotides. On entend également par polynucléotide, les ADN et ARN contenant une ou plusieurs bases modifiées. On entend par base modifiée, par exemple, les bases inhabituelles telles que l'inosine. Le terme polynucléotide vise également les polynucléotides de forme modifiée chimiquement, enzymatiquement ou métaboliquement. Les polynucléotides comprennent également les polynucléotides courts tels que les oligonucléotides.

On entend par "polypeptide", un peptide, un oligopeptide, un oligomère ou une protéine comprenant au moins deux acides aminés joints l'un à l'autre par une liaison peptidique normale ou modifiée.

Le terme polypeptide comprend les chaînes courtes, appelées peptides, oligopeptides et oligomères, et les chaînes longues, appelées protéines.

Un polypeptide peut être composé d'acides aminés autres que les 20 acides aminés codés par les gènes humains. Un polypeptide peut également être composé d'acides aminés modifiés par des processus naturels, tels que le processus de maturation post-traductionnel ou par des procédés chimiques, qui sont bien connus de l'homme du métier. Le même type de modification peut être présent à plusieurs endroits du polypeptide et n'importe où dans le polypeptide : dans le squelette peptidique, dans la chaîne d'acides aminés ou encore aux extrémités carboxy- ou amino-terminales.

Un polypeptide peut être ramifié suite à une ubiquitination ou être cyclique avec ou sans ramification. Ce type de modifications peut être le résultat de processus de post-traduction naturel ou synthétique, qui sont bien connus de l'homme du métier.

On entend, par exemple, par modifications d'un polypeptide, l'acétylation, l'acylation, l'ADP-ribosylation, l'amidation, la fixation covalente de flavine, la fixation covalente d'un hème, la fixation covalente d'un nucléotide ou d'un dérivé nucléotidique, la fixation covalente d'un lipide ou d'un dérivé lipidique, la fixation covalente d'un phosphatidylinositol, la réticulation covalente ou non-covalente, la cyclisation, la formation de pont disulfure, la déméthylation, la formation de cystéine, la formation de pyroglutamate, la formylation, la gamma-carboxylation, la glycosylation, la formation d'ancre au GPI, l'hydroxylation, l'iodisation, la méthylation, la myristoylation, l'oxydation, le processus protéolytique, la phosphorylation, la prénylation, la racémisation, la sénéloylation, la sulfatation, l'addition d'acides aminés telles que l'arginylation ou l'ubiquitination (PROTEINS STRUCTURE AND MOLECULAR PROPERTIES, 2nd Ed., T.E. Creighton, W.H. Freeman and Company, New York (1993) and Wold, F., Posttranslational Protein Modifications: Perspectives and Prospects, pgs 1-12 in POSTTRANSLATIONAL COVALENT MODIFICATION OF PROTEINS, B.C. Johnson, Ed., Academic Press, New York (1983); Seifter et al., Meth. Enzymol. 182:626-646 (1990) and Rattan et al., Protein Synthesis: Posttranslational Modifications and Aging, Ann. N.Y. Acad. Sci. 663:48-62 (1992)).

On entend par "pourcentage d'identité" entre deux séquences polynucléotidiques ou polypeptidiques le pourcentage de nucléotides ou d'acides aminés identiques entre les deux séquences à comparer, obtenu après le meilleur alignement, ce pourcentage étant purement statistique et les différences entre les deux séquences étant réparties au hasard et sur toute leur longueur. Les comparaisons entre deux séquences polynucléotidiques ou polypeptidiques sont traditionnellement réalisées en comparant ces séquences après les avoir alignées de manière optimale, ladite comparaison étant réalisée par segment ou par "fenêtre de comparaison" pour identifier et comparer les régions locales de similarité de séquence. Cette comparaison peut être réalisée grâce à un programme, par exemple le programme EMBOSS-Needle (alignement global Needleman-Wunsch) à l'aide de la matrice BLOSUM62/Open Gap 10.0 et Extension Penalty de 0,5 (Needleman et Wunsch (1970). J Mol. Biol. 48, 443-453 et Kruskal, J.B. (1983), An overview of sequence comparison, In D. Sankoff and J.B. Kruskal, (ed), Time warps, strind edits and macromolecules : the theory and practice of sequence comparison, pp. 1-44 Addison Wesley).

Le pourcentage d'identité est calculé en déterminant le nombre de positions identiques pour lesquelles le nucléotide ou l'acide aminé est identique entre les deux séquences, en divisant ce nombre de positions identiques par le nombre total de positions dans la fenêtre de comparaison et en multipliant le résultat obtenu par 100.

Un polynucléotide ayant, par exemple, une identité d'au moins 95% avec le polynucléotide ID SEQ N°1 est donc un polynucléotide comportant, au plus, 5 nucléotides modifiés sur 100 nucléotides, par rapport à ladite séquence. En d'autres termes jusqu'à 5% des nucléotides de la séquence ID SEQ N°1 peuvent être délétés ou substitués par un autre nucléotide, ou jusqu'à 5% du nombre total de nucléotides de la séquence ID SEQ N°1 peuvent être insérés dans ladite séquence. Ces modifications peuvent être positionnées aux extrémités 3' et/ou 5', ou à un endroit quelconque entre ces extrémités, en un seul ou plusieurs emplacements.

Par analogie, un polypeptide ayant une identité d'au moins 95% avec le polypeptide ID SEQ N°2 est un polypeptide comportant, au plus, 5 acides aminés modifiés sur 100 acides aminés, par rapport à ladite séquence. En d'autres termes jusqu'à 5% des acides aminés dans la séquence ID SEQ N°2 peuvent être délétés ou substitués par un autre acide aminé ou jusqu'à 5% du nombre total d'acides aminés de la séquence ID SEQ N°2 peuvent être insérés dans ladite séquence. Ces altérations de la séquence peuvent être situées aux positions amino et/ou carboxy-terminales de la séquence d'acides aminés ou à un endroit quelconque entre ces positions terminales, en un ou plusieurs emplacements. (Computational Molecular Biology, Lesk, A.M., ed, Oxford University Press, New York, 1988; Biocomputing: Informatics and Genome Projects, Smith, D.W., ed., Academic Press, New York, 1993 ; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987).

La "similarité" est, quant à elle, calculée de la même façon que l'identité, excepté que les acides aminés non identiques, mais présentant des caractéristiques physico-chimiques communes, sont considérés comme identiques.

On entend par "cellule hôte", une cellule qui a été transformée ou transfectée, ou est capable de transformation ou de transfection, par une séquence polynucléotidique exogène.

On entend par "milieu de culture", le milieu dans lequel on purifie le polypeptide de l'invention. Ce milieu peut être constitué par le milieu extracellulaire et/ou le lysat cellulaire. Des techniques bien connues de l'homme du métier permettent également à ce dernier de redonner la conformation active au polypeptide, si la conformation dudit polypeptide a été altérée lors de l'isolation ou de la purification.

On entend par "fonction", l'activité biologique d'un polypeptide ou d'un polynucléotide.

La fonction d'un polypeptide conforme à l'invention est celle d'un antigène de *Staphylococcus epidermidis* et/ou de *Staphylococcus aureus*, et la fonction d'un polynucléotide conforme à l'invention est celle de coder ce polypeptide. La fonction d'une combinaison d'antigènes est de permettre d'établir un diagnostic d'une infection sur matériel étranger et, en particulier, sur prothèse ostéoarticulaire, mais aussi de réaliser des suivis post-implantatoire, des suivis thérapeutiques et, enfin, de préciser s'il s'agit d'une infection active ou aiguë par, par exemple, l'utilisation de la détection de différents isotypes d'anticorps.

On entend par "antigène", tout composé qui, seul ou en association avec un adjuvant ou porteur, est capable d'induire une réponse immunitaire spécifique. Cette définition comprend également tout composé présentant une analogie structurale avec ledit antigène capable d'induire une réponse immunologique dirigée contre ledit antigène.

On entend par "analogie structurale" une analogie aussi bien de la structure primaire (séquence) que de la structure secondaire (éléments structuraux), de la structure tertiaire (structure tridimensionnelle) ou de la structure quaternaire (association de plusieurs polypeptides dans un même complexe) .(BIOCHElISTRY, 4ème Ed, L. Stryer, New York, 1995).

On entend par "variant" d'un polynucléotide dit initial ou d'un polypeptide dit initial, respectivement, un polynucléotide ou un polypeptide qui en diffère par au moins un nucléotide ou un acide aminé, mais qui garde les mêmes propriétés intrinsèques, c'est-à-dire la même fonction.

Une différence dans la séquence polynucléotidique du variant peut altérer ou non la séquence d'acides aminés du polypeptide qu'il code, par rapport à un polypeptide initial. Néanmoins, par définition, ces variants doivent conférer la même fonction que la séquence polynucléotidique initiale, par exemple, coder un polypeptide ayant une fonction antigénique.

Le polynucléotide ou le polypeptide variant diffère généralement du polynucléotide initial ou du polypeptide initial par une (ou plusieurs) substitution(s), addtion (s), délétion (s), fusion (s) ou troncation(s) ou plusieurs de ces modifications, prises en combinaison. Un variant non-naturel d'un polynucléotide initial ou d'un polypeptide initial peut être obtenu, par exemple, par mutagenèse dirigée ou par synthèse directe.

On définit comme "séquence polynucléotidique complémentaire à la séquence polynucléotidique", un polynucléotide qui peut être hybridé avec cette séquence polynucléotidique dans des conditions stringentes.

On entend généralement, mais pas nécessairement, par "conditions stringentes" les conditions chimiques qui permettent une hybridation lorsque les séquences polynucléotidiques ont une identité d'au moins 80%.

Ces conditions peuvent être obtenues selon les méthodes bien connues de l'homme du métier.

On entend par "anticorps", les anticorps monoclonaux, polyclonaux, chimériques, simple chaîne, humanisés ainsi que les fragments Fab, incluant les produits d'un Fab ou d'une banque d'expression d'immunoglobulines. Il est aussi possible d'utiliser à la place des anticorps d'autres molécules immunospécifiques par exemple des récepteurs de cellules T (TCR) comme récemment décrit (Li et al. 2005. Directed evolution of human T-cell receptors with picomolar affinities by phage display. Nat .Biotechnol. 23:349-54) ou des molécules sélectionnées pour leur fixation spécifique, par exemple par évolution dirigée. (voir par exemple Conrad et Scheller. 2005. Considération on antibody-phage display methodology. Comb Chem High Throughput Screen. 8:117-26).

Un anticorps immunospécifique peut être obtenu par administration à un animal d'un polypeptide donné suivie d'une récupération des anticorps produits par ledit animal par extraction depuis ses fluides corporels. On peut également administrer à l'animal un variant dudit polypeptide, ou des cellules hôtes exprimant ce polypeptide.

Le terme "immunospécifique" appliqué au terme anticorps, vis-à-vis d'un polypeptide donné, signifie que l'anticorps possède une meilleure affinité pour ce polypeptide que pour d'autres polypeptides connus de l'art antérieur.

Par sérum "positif", on entend un sérum contenant des anticorps, produits à la suite d'une infection à *S. epidermidis* ou *S. aureus* sur prothèse articulaire, identifiés par leur liaison avec les polypeptides (antigènes) de l'invention.

On entend par "sensibilité" la proportion de malades infectés, diagnostiqués selon l'art antérieur et donnés positifs par le diagnostic selon l'invention.

On entend par "spécificité" la proportion de donneurs de sang, testés comme témoins, soumis au diagnostic selon l'invention et donnés négatifs par le diagnostic selon l'invention.

La présente invention répond aux objectifs qu'elle s'est fixés plus haut en apportant de nouveaux polynucléotides et de nouveaux polypeptides, et ainsi que des fragments qui se sont révélés plus pertinents et/ou plus sensibles et/ou plus spécifiques que la protéine entière.

La demande décrit l'utilisation, *in vitro,* de l'une au moins des protéines de séquence ID SEQ N°2, 4, 6, 8, 10, 12, 14, 16, 18, 20, 22, 24, 26, 28, 30, 32, 34 ou 36, d'une partie ou de variants de cette protéine, de cellules hôtes comprenant des vecteurs incluant un polynucléotide codant pour l'une au moins desdites protéines ou un variant desdites protéines, dans la production d'anticorps et le domaine du diagnostic *in vitro* de *Staphylococcus epidermidis* et/ou *S. aureus*. On décrit également un kit de diagnostic et une composition pharmaceutique.

### Utilisation des polypeptides

De nombreuses séquences polypeptidiques et nucléotidiques dérivées de Staphylococcus epidermidis sont décrites dans l'art antérieur et notamment dans les demandes de brevet WO 01/34809 et US2004147734 et le brevet US6380370 qui proposent également leur utilisation dans des méthodes de prévention, de diagnostic ou de traitement d'infections bactériennes à Staphylococcus epidermidis.

Le laboratoire de la Déposante connaît des polypeptides issus de *S. epidermidis* ou *S. aureus* qui ne permettent pas de pronostiquer une infection sur matériel étranger, notamment sur prothèse articulaire (par exemple : la protéine "Thimet oligopeptidase-like protein" (Q8CPS6) recombinante de *S. epidermidis*).

La Déposante a cependant identifié, de façon tout à fait inattendue, qu'un tel pronostic était possible en utilisant d'autres polypeptides issus de *S. epidermidis* ou *S. aureus* qu'elle a spécifiquement identifiés et, même, des fragments de polypeptides issus de *S. epidermidis* ou *S. aureus* qui, complets, ne permettent pas ce pronostic.

L'identification des polypeptides selon l'invention est le résultat d'un crible et d'études approfondies que les séquences issues des programmes de génomique de *S. epidermidis* ou *S. aureus* ne laissaient pas envisager.

Ainsi, la demande décrit l'utilisation, dans la production d'anticorps et dans le domaine du diagnostic *in vitro* d'infections à *Staphylococcus epidermidis et*/*ou S. aureus,* d'au moins un polypeptide comprenant :
- la séquence d'acides aminés ID SEQ N°2 (appelée protéine E4), codée par la séquence polynucléotidique ID SEQ N°1 ; ou
- la séquence d'acides aminés ID SEQ N°4 (appelée protéine 2B6), codée par la séquence polynucléotidique ID SEQ N°3 ; ou
- la séquence d'acides aminés ID SEQ N°6 (appelée protéine F2), codée par la séquence polynucléotidique ID SEQ N°5 ; ou
- la séquence d'acides aminés ID SEQ N°8 (appelée protéine 3F7), codée par la séquence polynucléotidique ID SEQ N°7 ; ou
- la séquence d'acides aminés ID SEQ N°10 (appelée protéine 2D6B1), codée par la séquence polynucléotidique ID SEQ N°9 ; ou
- la séquence d'acides aminés ID SEQ N°12 (appelée protéine JR7), codée par la séquence polynucléotidique ID SEQ N°11 ; ou
- la séquence d'acides aminés ID SEQ N°14 (appelée protéine JR12), codée par la séquence polynucléotidique ID SEQ N°13 ; ou
- la séquence d'acides aminés ID SEQ N°16 (appelée protéine JR5), codée par la séquence polynucléotidique ID SEQ N°15 ; ou
- la séquence d'acides aminés ID SEQ N°18 (appelée protéine 3A7), codée par la séquence polynucléotidique ID SEQ N°17 ; ou
- la séquence d'acides aminés ID SEQ N°20 (appelée protéine 3B6), codée par la séquence polynucléotidique ID SEQ N°19 ; ou
- la séquence d'acides aminés ID SEQ N°22 (appelée protéine 3D5), codée par la séquence polynucléotidique ID SEQ N°21 ; ou
- la séquence d'acides aminés ID SEQ N°24 (appelée protéine 3E5), codée par la séquence polynucléotidique ID SEQ N°23 ; ou
- la séquence d'acides aminés ID SEQ N°26 (appelée protéine 3F3), codée par la séquence polynucléotidique ID SEQ N°25 ; ou
- la séquence d'acides aminés ID SEQ N°28 (appelée protéine 3G3), codée par la séquence polynucléotidique ID SEQ N°27 ; ou
- la séquence d'acides aminés ID SEQ N°30 (appelée protéine 3H3), codée par la séquence polynucléotidique ID SEQ N°29 ; ou
- la séquence d'acides aminés ID SEQ N°32 (appelée protéine 3H4), codée par la séquence polynucléotidique ID SEQ N°31 ; ou
- la séquence d'acides aminés ID SEQ N°34 (appelée protéine 3D7), codée par la séquence polynucléotidique ID SEQ N°33 ; ou
- la séquence d'acides aminés ID SEQ N°36 (appelée protéine 3C7), codée par la séquence polynucléotidique ID SEQ N°35.

On décrit également l'utilisation d'au moins un polypeptide comprenant :
a) une partie de la séquence d'acides aminés ID SEQ N°2, 4, 6, 8, 10, 12, 14, 16, 18, 20, 22, 24, 26, 28, 30, 32, 34 ou 36 ayant la même fonction que ladite séquence, ou
b) une séquence d'acides aminés ayant au moins 60% d'identité, de préférence au moins 80% d'identité, et mieux au moins 90% d'identité, avec l'une des séquences d'acides aminés ID SEQ N°2, 4, 6, 8, 10, 12, 14, 16, 18, 20, 22, 24, 26, 28, 30, 32, 34 ou 36, ou avec la partie de séquence définie sous a), et ayant la même fonction que ladite séquence.

Ainsi, des polypeptides peuvent comprendre des variants des séquences d'acides aminés ID SEQ N°2, 4, 6, 8, 10, 12, 14, 16, 18, 20, 22, 24, 26, 28, 30, 32, 34 ou 36.

La séquence polypeptidique ID SEQ N°4, appelée protéine 2B6, codée par la séquence ID SEQ N°3, comprend les acides aminés 1 à 184 de la séquence ID SEQ N°2 et a la même fonction que la séquence ID SEQ N°2.

La séquence polypeptidique ID SEQ N°6 (appelée protéine F2), codée par la séquence polynucléotidique ID SEQ N°5, présente 60% d'identité et 78% de similarité avec la séquence ID SEQ N°2.

D'autres variants des séquences ID SEQ N°2, 4 et 6 sont, par exemple, une partie de la protéine AtlC (3F7 (ID SEQ N°8) par exemple) codée par la bactérie *Staphylococcus caprae.*

Grâce aux polypeptides définis ci-dessus, l'invention permet la détection d'anticorps naturellement produits lors d'infections à staphylocoques sur prothèse articulaire.

Les polypeptides décrits peuvent être utilisés pour détecter, périodiquement, *in vitro,* les anticorps dirigés contre *S. epidermidis* et/ou *S. aureus* et ainsi suivre l'évolution de la pathologie et de l'effet d'un traitement appliqué à un patient.

Les échantillons biologiques testés peuvent être du sang, de l'urine, de la salive, du liquide de ponction de sérologie (par exemple liquide céphalo-rachidien, liquide pleural ou liquide articulaire) ou l'un de leurs constituants (par exemple, le sérum).

Le diagnostic *in vitro* d'une infection à *Staphylococcus epidermidis* et/ou *S. aureus* est effectué, par exemple, par des tests classiques de réactions immunologiques, tels que ELISA ou Western Blot. Ces tests utilisent l'un des polypeptides selon l'invention qui vient se fixer, de manière spécifique, aux anticorps sériques contre *Staphylococcus epidermidis et*/*ou* aureus présents dans les échantillons biologiques.

### Utilisation de vecteurs d'expression et de cellules hôtes

La présente invention a aussi pour objet l'utilisation d'un polypeptide préparé par culture d'une cellule hôte comprenant un vecteur recombinant ayant, inséré, un polynucléotide codant pour ledit polypeptide.

De nombreux systèmes d'expression peuvent être utilisés comme, par exemple, les chromosomes, les épisomes, les virus dérivés. Plus particulièrement, les vecteurs recombinants utilisés peuvent être dérivés de plasmides bactériens, de transposons, d'épisome de levure, d'éléments d'insertion, d'éléments chromosomiques de levures, de virus tels que les baculovirus, les *papillonna virus* comme SV40, les *vaccinia virus,* les adénovirus, les *fox pox virus,* les *pseudorab.ies virus,* les rétrovirus.

Ces vecteurs recombinants peuvent également être des dérivés de cosmides ou de phagemides. La séquence polynucléotidique peut être insérée dans le vecteur recombinant d'expression par les méthodes bien connues de l'homme du métier.

Le vecteur recombinant peut comprendre des séquences polynucléotidiques de contrôle de la régulation de l'expression du polynucléotide ainsi que des séquences polynucléotidiques permettant l'expression et la transcription d'un polynucléotide selon l'invention et la traduction d'un polypeptide selon l'invention, ces séquences étant choisies en fonction des cellules hôtes mises en oeuvre.

L'introduction du vecteur recombinant dans une cellule hôte peut être effectuée selon les méthodes bien connues de l'homme du métier telles que la transfection par phosphate de calcium, la transfection par lipides cationiques, l'électroporation, la transduction ou l'infection.

Les cellules hôtes peuvent être, par exemple, des cellules bactériennes, telles que des cellules de streptocoques, de staphylocoques, d'*Escherichia coli* ou de *Bacillus subtilis* ; des cellules de champignons, telles que des cellules de levure et des cellules *d'Aspergillus ;* des cellules de *Streptomyces* ; des cellules d'insectes, telles que des cellules de *Drosophilia S2* et de *Spodoptera Sf9 ;* des cellules animales, telles que les cellules CHO, COS, HeLa, C127, BHK, HEK 293 ou encore des cellules végétales.

Le polypeptide peut être purifié à partir des cellules hôtes, selon les méthodes bien connues de l'homme du métier, telles que la précipitation à l'aide d'agents chaotropiques comme les sels, en particulier le sulfate d'ammonium, l'éthanol, l'acétone ou l'acide trichloroacétique, ou de moyens tels que l'extraction à l'acide, la chromatographie échangeuse d'ions, la chromatographie sur phosphocellulose, la chromatographie par interaction hydrophobe, la chromatographie d'affinité, la chromatographie sur hydroxylapatite ou les chromatographies d'exclusion.

### Utilisation des polynucléotides

On décrit également l'utilisation, dans la production d'anticorps et dans le diagnostic d'une infection à *Staphylococcus epidermidis et*/*ou aureus,* d'au moins un polynucléotide comprenant la séquence polynucléotidique ID SEQ N°1, 3, 5, 7, 9 11, 13, 15, 17, 19, 21, 23, 25, 27, 29, 31, 33 ou 35, codant, respectivement, pour la protéine 2, 4, 6, 8, 10, 12, 14, 16, 18, 20, 22, 24, 26, 28, 30, 32, 34 ou 36.

Est également décrite l'utilisation d'au moins un polynucléotide comprenant :
a) une partie de la séquence ID SEQ N°1, 3, 5, 7, 9, 11, 13, 15, 17, 19, 21, 23, 25, 27, 29, 31, 33 ou 35 et ayant la même fonction que ladite séquence, ou
b) une séquence polynucléotidique ayant au moins 60% d'identité, de préférence au moins 80% d'identité, et mieux au moins 90% d'identité, avec la séquence polynucléotidique ID SEQ N°1, 3, 5, 7, 9, 11, 13, 15, 17, 19, 21, 23, 25, 27, 29, 31, 33 ou 35 , ou avec la partie de séquence telle que définie sous a), et ayant la même fonction que ladite séquence, ou
c) une séquence polynucléotidique complémentaire à la séquence polynucléotidique ID SEQ N°1, 3, 5, 7, 9, 11, 13, 15, 17, 19, 21, 23, 25, 27, 29, 31, 33 ou 35 ou à la partie de séquence définie sous a) ou à la séquence définie sous b).

La séquence polynucléotidique ID SEQ N°3 comprend les nucléotides 1 à 552 de la séquence ID SEQ N°1 et a la même fonction que ladite séquence.

La séquence polynucléotidique ID SEQ N°5 (appelée F2 et extraite à partir du génome de *Staphylococcus aureus*) est un variant de la séquence ID SEQ N°1.

D'autres variants sont, par exemple, des séquences polynucléotidiques codant pour des protéines de la famille des autolysines (par exemple, la séquence ID SEQ N°7, variant de la séquence ID SEQ n°1, codant pour une partie de la protéine AtlC, appelée 3F7 et présente dans la bactérie *Staphylococcus caprae*). Ainsi, des polynucléotides peuvent comprendre des variants de l'une des séquences polynucléotidiques ID SEQ N°1, 3, 5, 7, 9, 11, 13, 15, 17, 19, 21, 23, 25, 27, 29, 31, 33 ou 35.

Les polynucléotides de l'invention peuvent être obtenus par les méthodes standard de synthèse d'ADN ou d'ARN.

Les polynucléotides conformes à l'invention peuvent également comprendre des séquences polynucléotidiques comme les séquences 5' et/ou 3' non-codantes, telles que, par exemple, des séquences transcrites, des séquences non-traduites, des séquences signal d'épissage, des séquences polyadénylées, des séquences de liaison avec des ribosomes ou encore des séquences qui stabilisent l'ARNm.

### Utilisations des anticorps selon l'invention

La demande vise également l'utilisation d'anticorps, selon l'invention, pour détecter, *in vitro,* dans des échantillons biologiques la présence d'antigènes de *S. epidermidis* et/ou *S. aureus.*

La demande vise, en outre, l'utilisation d'anticorps selon l'invention, pour détecter, périodiquement, *in vitro,* les antigènes de *S. epidermidis* et/ou *S. aureus* et ainsi suivre l'évolution de la pathologie et de l'effet d'un traitement appliqué à un patient.

Les anticorps immunospécifiques peuvent être obtenus par administration d'un polypeptide selon l'invention, d'un de ses fragments, d'un analogue ou d'un fragment épitopique ou d'une cellule exprimant ce polypeptide, à un mammifère, de préférence non humain, selon les méthodes bien connues de l'homme du métier.

Pour la préparation d'anticorps monoclonaux, on peut utiliser des méthodes usuelles de production d'anticorps, à partir de lignées cellulaires, telles que la technique des hybridomes, la technique des triomes, la technique des hybridomes de cellules B humaines et la technique des hybridomes EBV.

### Isotypes d'anticorps et affinité

Les immunoglobulines (ou anticorps) sont constituées de deux chaînes polypeptidiques différentes : deux chaînes légères (L) d'isotypes [kappa] ou [lambda], et de deux chaînes lourdes (H) d'isotypes [gamma], [alpha], [mu], [delta] ou [epsilon]. Ces quatre chaînes sont liées covalemment par des ponts disulfures. Les deux types de chaînes H ou L possèdent des régions qui contribuent à la fixation des antigènes et sont très variables d'une immunoglobuline à une autre. Ces régions déterminent l'affinité des anticorps pour leur ligand.

Les isotypes des chaînes lourdes permettent de définir la classe de l'immunoglobuline ; il existe donc 5 isotypes d'anticorps (IgA, IgM, IgD, IgE et IgG). Des sous-classes, par exemple IgG1, IgG2, IgG3 et IgG4 sont définies par des différences de séquence de la chaîne lourde. Les différentes classes d'immunoglobulines ont des propriétés physicochimiques propres et leur synthèse dépend directement des phases et des niveaux d'activation de la réponse immunitaire.

Chez l'homme, les IgG représentent la principale classe d'immunoglobulines : leur concentration sérique chez l'adulte varie de 8 à 16 g/l. Leur demi-vie plasmatique est d'environ 3 semaines.

Les IgA constituent la deuxième classe d'immunoglobulines sériques, après les IgG, en terme de concentration (2 à 4 g/l). En revanche, elles représentent la classe prépondérante des immunoglobulines dans les sécrétions (sécrétions respiratoires, salivaires, digestives..., lait, colostrum, larmes). Sur le plan structural, les IgA ont la particularité de se présenter sous plusieurs formes moléculaires :
- dans le sérum, les IgA peuvent se présenter sous forme de monomères (forme prépondérante) ou sous forme de dimères associés à une chaîne J (chaîne de jonction). La chaîne J est un peptide riche en cystéine de 137 acides aminés (poids moléculaire : 15 000 Da), d'origine plasmocytaire ; la sous-classe IgA1 est prépondérante dans le sérum.
- dans les sécrétions, les IgA, appelées IgA sécrétoires, sont sous forme de dimères ; elles sont donc associées à une chaîne J, mais elles comportent également un composant sécrétoire. Les IgA produites par les lymphocytes B sont captées par les cellules épithéliales, par un récepteur (polyIgR) situé au pôle basal de la cellule. C'est pendant le transfert de l'IgA à travers la cellule épithéliale, vers le pôle apical de la cellule, que le composant sécrétoire (poids moléculaire : 70 000 Da), ou pièce sécrétoire, est ajouté. Le rôle de la pièce sécrétoire est de protéger les IgA sécrétoires vis-à-vis des enzymes protéolytiques présents dans les sécrétions.

Comme les IgA, les IgM peuvent se présenter sous 2 formes moléculaires distinctes :
- une forme monomérique : c'est la forme sous laquelle les IgM sont synthétisées et insérées dans la membrane du lymphocytes B ;
- une forme pentamérique : c'est la forme sous laquelle les IgM sont sécrétées. Les 5 monomères de bases sont reliés par des ponts disulfures. De plus, une chaîne J relie l'extrémité de 2 monomères.

Les IgD représentent moins de 1 % des immunoglobulines sériques. Elles sont habituellement coexprimées avec les IgM à la surface des lymphocytes B où elles semblent jouer un rôle de récepteur pour les antigènes.

Les IgE ne sont présentes, chez un sujet normal, qu'à l'état de traces.

### Cinétique d'apparition des anticorps et affinité

La cinétique d'apparition d'anticorps spécifiques et d'un isotype donné est aujourd'hui encore mal comprise. D'une façon générale les cellules B dites naïves synthétisent différents IgM qui constituent un large répertoire de molécules capables de fixer des antigènes (Steven A. Frank., Immunology and Evolution of Infectious Disease. (2002), Princeton University Press, Princeton, USA). Ainsi lors de la première exposition à un antigène, celui-ci va se fixer avec une faible affinité à différents IgM du système immunitaire. Cette interaction va néanmoins stimuler la division de la cellule B naïve correspondante. La division est d'autant plus rapide que l'affinité de l'IgM est forte ce qui permet une sélection initiale des meilleurs clones. De plus, lors de la division les réarrangements génétiques permettent l'augmentation de l'affinité des anticorps. La région constante dès immunoglobulines change également afin de produire des IgG dans le système circulatoire et des IgA à la surface des muqueuses (Steven et Frank 2002. Immunology and Evolution of Infectious Disease. Princeton University Press, Princeton, USA).

Il peut donc être déduit par l'homme de l'art que la présence d'une ou plusieurs classes d'anticorps et l'affinité particulière des anticorps ont différentes implications d'un point de vue du diagnostic médical dans la différentiation des infections récurrentes ou non, d'une infection active, aiguë, chronique, latente, et récente.

### Les kits

Des kits de diagnostic *in vitro* peuvent compendre au moins l'un des polypeptides conformes à l'invention, ou au moins l'un des polynucléotides codant pour lesdits polypeptides, ainsi que des kits de diagnostic *in vitro* comprenant au moins l'un des anticorps conformes à l'invention.

### Les vaccins

La présente description porte également ou une composition pharmaceutique, utilisable comme vaccin, renfermant à titre de principe actif au moins un polypeptide selon l'invention ou un polynucléotide ou un vecteur recombinant ou une cellule hôte selon l'invention.

### Partie expérimentale

### A) Protocoles d'obtention des antigènes

### A.1. Clonage de la séquence codant pour les polypeptides ID SEQ N°2, 4, 6, 8, 10, 12, 14, 16, 18, 20, 22, 24, 26, 28, 30, 32, 34 ou 36

Les gènes codant pour les séquences des polypeptides, qui sont des antigènes, sont obtenus par amplification par PCR à partir de l'ADN génomique des bactéries *Staphylococcus epidermidis* (souche WHO 12, ATCC 12228), *Staphylococcus caprae* (ATCC 35538) ou *Staphylococcus aureus* (souche MU50, ATCC 700699 ou MW2) en utilisant, respectivement, des amorces spécifiques des séquences ID SEQ N°1, 3, 5, 7, 9, 11, 13, 15, 17, 19, 21, 23, 25, 27, 29, 31, 33 ou 35. On entend par amorces spécifiques de courtes séquences nucléotidiques capables de s'hybrider de façon spécifique, grâce à la complémentarité des bases, sur le brin d'ADN ou sur son brin complémentaire ; ces amorces sont choisies par l'homme de l'art de façon à encadrer la séquence d'ADN et à l'amplifier spécifiquement (par exemple ID SEQ N°1, 3, 5, 7, 9, 11, 13, 15, 17, 19, 21, 23, 25, 27, 29, 31, 33 ou 35).

Le fragment correspondant, ainsi amplifié, est cloné dans un vecteur selon des techniques classiques bien connues de l'homme du métier. Ce vecteur permet la production des protéines clonées sous contrôle d'un promoteur inductible par l'isopropyl-thiogalactoside (IPTG). Les protéines clonées correspondent aux polypeptides ID SEQ N°2, 4, 6, 8, 10, 12, 14, 16, 18, 20, 22, 24, 26, 28, 30, 32, 34 ou 36.

### A.2. Expression des protéines

Une souche d'*Escherichia coli* est transformée par les vecteurs d'expression précédemment décrits. Les bactéries sélectionnées sont mises à cultiver une nuit à 30°C sous agitation dans 30 ml de milieu Luria Bertani (LB, J. Miller, "A short Course in Bacterial Genetics", Cold Spring Harbor Laboratory Press, 1992) contenant de l'ampicilline à une concentration finale de 100 µg/ml. Le lendemain, la culture est diluée au 1/50^{ème} dans un volume final de 1 litre de milieu LB additionné d'ampicilline à une concentration finale de 100 µg/ml qui est incubée à 30°C sous agitation. Lorsque la turbidité de la culture atteint une valeur d'absorbance à 600 nm (en abrégé, A600) d'environ 0,7, la production de la protéine est induite par l'isopropyl-thiogalactoside (en abrégé, IPTG) à une concentration finale de 0,1 mM. Les bactéries sont récoltées par centrifugation (10 minutes à 1400xg et à 4°C) lorsque la turbidité de la culture atteint une A600 d'environ 1,5.

### A.3. Purification des protéines

Après centrifugation, les cellules sont remises en suspension dans un tampon Tris-HCl 20 mM à pH 8,0 contenant du saccharose à 0,5 mM, puis traitées par du lysozyme (0,2 g/l) en présence d'acide éthylènediaminotétraacétique (EDTA) 12,5 mM, de DNase, RNase et PMSF. La suspension est incubée 30 minutes à 4°C puis centrifugée 10 minutes à 4°C à 15500xg. Le culot est congelé à -20°C pendant au moins une nuit.

### A.4. Exemple : purification de 3C7 (ID SEQ N°36)

Après décongélation, les bactéries sont reprises dans un tampon Mes 25 mM à pH 6,0, puis soniquées 4 fois 20 secondes dans la glace. Après centrifugation à 15500xg à 4°C pendant 30 minutes, le surnageant est filtré sur membrane de porosité 0,22 µm. Le filtrat est alors déposé sur une colonne échangeuse de cation (par exemple, SP-Sépharose 12 ml, Amersham Biosciences). Après lavage de la colonne, la protéine est éluée par un gradient linéaire de 0 à 1 M de NaCl dans du tampon Mes 25 mM à pH 6,0 en 20 volumes de colonne Les fractions contenant la protéine sont rassemblées et les protéines sont précipitées par du sulfate d'ammonium à une concentration finale de 0,6 g/l. La solution est laissée au moins une nuit à 4°C puis centrifugée 30 minutes à 20800xg. Le culot est alors repris dans un volume le plus faible possible (en général 300 µl de tampon Na₂HPO_{4/}NaH₂PO₄ 50 mM pH 8,0 contenant du NaCl 100 mM puis déposé sur une colonne de gel filtration, par exemple Superdex HR75-10/30, Amersham). Les fractions éluées contenant la protéine sont rassemblées et du glycérol est ajouté jusqu'à une concentration finale de 20%. Les protéines purifiées sont alors stockées à -20°C jusqu'à leur utilisation dans les tests.

Les concentrations des protéines sont déterminées spectrophotométriquement à partir des coefficients d'absorption calculés par la méthode de Pace (Pace et al. 1995. How to measure and predict the molar absorption coefficient of a protein. Protein Science 4, 2411-2423). La pureté des protéines est vérifiée par analyse par électrophorèse SDS-PAGE et par spectrométrie de masse.

### B) Test de diagnostic in vitro

Il a été utilisé des sérums provenant de patients ayant eu une infection ostéoarticulaire documentée (collection du laboratoire) :
- à *Staphylococcus epidermidis* (au moins trois prélèvements per-opératoires positifs) ;
- à *Staphylococcus aureus* (au moins un prélèvement per-opératoire positif) ;
- à des bactéries autres que des staphylocoques (au moins trois prélèvements per-opératoires positifs).

Les sérums témoins correspondaient à des sérums de donneurs de sang (collection du laboratoire).

### Exemple B1 : Protocole du test pour les polypeptides de séquence ID SEQ N°2, 4, 6, 8, 10, 12, 14, 16, 18, 20, 22, 24, 26, 28, 30, 32 et 34 (ELISA)

La fixation des anticorps présents dans les sérums a été évaluée par des tests ELISA. Les plaques ELISA sont laissées pendant une nuit à 4°C en présence de 0,5 µg d'antigène purifié (protéine recombinante E4) dans du "tampon salin phosphate" (PBS). Après quatre lavages par du PBS contenant du polyoxyéthylène sorbitan (Tween) à 0,05%, les plaques sont saturées une heure à 37°C par du PBS-Tween contenant 5% de lait demi-écrémé (250 µl par puits). Quatre nouveaux lavages sont réalisés, puis 100 µl de chaque sérum positif, à la dilution adéquate (soit 1/3000^{ème} pour E4) dans du tampon PBS-Tween contenant 5% de lait demi-écrémé, sont ajoutés dans chaque puits. La plaque est ensuite laissée à 25°C pendant 30 minutes. Après quatre nouveaux lavages, des anticorps (secondaires) anti-immunoglobulines G, M, ou A ou simultanément anti-immunoglobulines G et/ou A et/ou M, humaines de chèvre marqués à la phosphatase alcaline (par exemple 170-6462, Biorad) sont ajoutés pendant 30 minutes à 25°C après avoir été dilués selon le protocole du fournisseur dans du tampon PBS-Tween contenant 5% de lait demi écrémé. Quatre nouveaux lavages sont réalisés puis 100 µl de substrat (phosphate de p-nitrophényle), pNPP, par exemple A-3469, Sigma) sont ajoutés. L'absorbance à 405 nm de chacun des puits est mesurée après une incubation de 30 minutes à 37°C.

### Résultats et interprétation

Les essais ont été réalisés sur des protéines recombinantes issues de purifications indépendantes.

Des résultats types sont présentés dans le tableau 1 (résultats pour les polypeptides E4, F2 et 2D6-B1 avec des anticorps secondaires reconnaissant les immunoglobulines totales (Ig G, A et M) présentes dans les sérums des patients).

**Tableau 1 : Résultats (ELISA) obtenus en utilisant des anti-IgGAM comme anticorps secondaires**

| | Polypeptide testé | | |
|---|---|---|---|
| | E4 | F2 | 2D6B1 |
| Proportion de sérums "positifs" parmi les 15 sérums de malades infectés par *S*. *epidermidis* diagnostiqués selon l'art antérieur et soumis au diagnostic selon l'invention | 47,0% | 50, 0% | 40,0% |
| Proportion de sérums "positifs" parmi les 16 sérums de malades infectés par *S*. *aureus* diagnostiqués selon l'art antérieur et soumis au diagnostic selon l'invention | 26,0% | 25,0% | 25,0% |
| Proportion de sérums "négatifs" parmi les 96 sérums de donneurs de sang testés comme témoins | 95,0% | 98,0% | 96,8% |

D'après le tableau 1, on constate que les polypeptides de l'invention peuvent être utilisés pour la mise en évidence d'infections à staphylocoques sur prothèses articulaires.

### Exemple B2 : Résultats obtenus par l'utilisation de la détection d'isotype d'anticorps dans les sérums (ELISA)

L'utilisation de certains polypeptides, par exemple 2D6-B1 (tableau 2), pour la détection d'immunoglobulines d'un isotype particulier est particulièrement pertinente.

**Tableau 2 : Exemples de résultats (ELISA) en utilisant des anti-IgA comme anticorps secondaires**

| Polypeptide testé | 2D6B1 |
|---|---|
| Proportion de sérums "positifs" parmi les 15 sérums de malades infectés par *S. epidermidis* diagnostiqués selon l'art antérieur et soumis au diagnostic selon l'invention | 47,0% |
| Proportion de sérums "positifs" parmi les 16 sérums de malades infectés par *S. aureus* diagnostiqués selon l'art antérieur et soumis au diagnostic selon l'invention | 31,0% |
| Proportion de sérums "négatifs" parmi les 96 sérums de donneurs de sang testés comme témoins | 97,0% |

Il en découle que les polypeptides peuvent être utilisés pour mettre en évidence la présence de certains isotypes d'anticorps lors d'infections à staphylocoques sur des prothèses articulaires.

### Exemple B3 : combinaison de polypeptides pour le diagnostic in vitro d'IPA

L'utilisation conjointe de plusieurs antigènes et/ou la détection de plusieurs isotypes d'anticorps permet d'augmenter la sensibilité de la méthode (Tableaux 3 et 4).

Le polypeptide 2B6 est très spécifique des infections à *S. epidermidis.* Le polypeptide 2D6-B1, au contraire de 2B6, est peu spécifique d'une espèce et des résultats "positifs" peuvent être observés avec des sérums de patients ayant une IPA à d'autres espèces de Staphylocoque (par exemple *S. Iugdunensis*)*.* Ce polypeptide est donc "spécifique" du genre staphylocoques et non d'une espèce particulière. Il est donc possible, par l'utilisation de différents polypeptides (ou par la détection de différents isotypes d'anticorps), de réaliser des distinction d'espèces.

**Tableau 3 : Résultats (ELISA) obtenus en utilisant des combinaisons de différents polypeptides, pour la détection d'immunoglobulines IgG, A ou M**

| Polypeptide testé | 2B6 | 2D6B1 | 2B6 ou 2D6B1 |
|---|---|---|---|
| Proportion de sérums "positifs" parmi les 15 sérums de malades infectés par *S. epidermidis* diagnostiqués selon l'art antérieur et soumis au diagnostic selon l'invention | 67,0% | 40,0% | 87,0% |
| Proportion de sérums "positifs" parmi les 16 sérums de malades infectés par *S. aureus* diagnostiqués selon l'art antérieur et soumis au diagnostic selon l'invention | 11,0% | 25,0% | 37,5% |
| Proportion de sérums "négatifs" parmi les 96 sérums de donneurs de sang testés comme témoins | 99,0% | 96,8% | 96,0% |

**Tableau 4 : Résultats (ELISA) obtenus en utilisant 2D6B1 comme polypeptide et en détectant les anticorps grâce à différents anticorps secondaires**

| Anticorps secondaire utilisé | IgA | IgG | IgG ou A |
|---|---|---|---|
| Proportion de sérums "positifs" parmi les 15 sérums de malades infectés par *S. epidermidis* diagnostiqués selon l'art antérieur et soumis au diagnostic selon l'invention | 47,0% | 40,0% | 67,0% |
| Proportion de sérums "positifs" parmi les 16 sérums de malades infectés par *S. aureus* diagnostiqués selon l'art antérieur et soumis au diagnostic selon l'invention | 31,0% | 25,0% | 37,0% |
| Proportion de sérums "négatifs" parmi les 96 sérums de donneurs de sang testés comme témoins | 97,0% | 96,0% | 96,0% |

### Exemple B4 : Identification d'IPA non diagnostiquées par culture de prélèvements per-opératoires

Ces essais ont été réalisés en utilisant des sérums provenant de patients opérés pour suspicion d'infection staphylococcique sur prothèse ostéoarticulaire, mais considérés comme bactériologiquement non infectés d'après les résultats de culture des échantillons profonds per-opératoires réalisés au cours de la révision de la prothèse (seulement 1 ou 2 prélèvements per-opératoires positifs à SCN).

Le tableau 5 présente un exemple de résultats types obtenus par détection des immunoglobulines totales anti-2D6B1 chez 7 patients ayant eu 1 ou 2 prélèvements per-opératoires positifs à *S. epidermidis* (collection du laboratoire). Les sérums témoins correspondaient à des sérums de donneurs de sang (collection du laboratoire).

**Tableau 5**

| | Type de prothèse | Valeur ELISA |
|---|---|---|
| Patient 1 | PTG | 2,026 |
| Patient 2 | PTH | 0,151 |
| Patient 3 | PTG | 0,302 |
| Patient 4 | PTH | 0,970 |
| Patient 5 | PTG | 2,237 |
| Patient 6 | PTH | 0,369 |
| Patient 7 | PTH | 0,465 |

Au cours de la même analyse, 3 des 89 sérums témoins (donneurs de sang) avaient une valeur ELISA ≥ 0,900 (seuil donnant une spécificité de 96,6%) et aucun n'avait une valeur ELISA ≥1,600 (seuil donnant une spécificité de 100,0%). La réponse observée était donc significative chez le patient 4 et hautement significative chez les patients 1 et 5.

Il est donc démontré qu'il est possible de détecter une réponse anticorps significative vis-à-vis des polypeptides selon l'invention au cours d'infections ostéoarticulaires sur prothèses, non diagnostiquées par culture d'échantillons per-opératoires.

### Exemple B5 : Protocole du test pour les polypeptides ID SEQ N°12, 14 et 16 (Western Blot)

Pour ces Western Blot, des sérums provenant de patients ayant eu une infection documentée à *S. aureus* ou *S. epidermidis* (collection du laboratoire) ont été utilisés.

Les sérums témoins correspondaient à des sérums de donneurs de sang (collection du laboratoire) et de patients porteurs d'autres infections (collection du laboratoire). La fixation éventuelle des anticorps présents dans ces sérums a été évaluée par des tests en Western Blot sur des extraits totaux de bactéries produisant les polypeptides ID SEQ N°12, 14 et 16.

La membrane de nitrocellulose sur laquelle les protéines de l'extrait sont transférées est saturée pendant 30 minutes à l'aide d'une solution de "tampon salin phosphate" (PBS) contenant 3% de lait demi écrémé. Après trois lavages par du PBS contenant du polyoxyéthylène sorbitan (Tween) à 0,05%, la membrane est mise en présence du sérum testé à la dilution adéquate (soit 1/300^{ème}) dans du tampon PBS contenant 3% de lait demi-écrémé, pendant 45 minutes. Après trois nouveaux lavages, des anticorps (secondaires) anti-immunoglobulines G, M ou A ou simultanément anti-immmunoglobulines G et/ou A et/ou M humaines de chèvre, marqués à la phosphatase alcaline (par exemple 170-6462, Biorad), sont ajoutés, pendant une période de 30 minutes après avoir été dilués, selon le protocole du fournisseur, dans du tampon PBS contenant 3% de lait demi écrémé. Trois nouveaux lavages sont réalisés puis du 5-bromo-4-chloro-3-indolyl-phosphate et du nitroblue tetrazolium sont ajoutés selon les indications du fournisseur jusqu'à l'obtention du résultat. Un résultat "positif" correspond à une précipitation du substrat sur la membrane à la position des polypeptides-ID SEQ N°12, 14 ou 16.

Les résultats à ces tests démontrent l'existence d'une réponse anticorps significative au cours des infections ostéoarticulaires sur matériel étranger vis-à-vis des polypeptides ID SEQ N°12, 14 ou 16 et la pertinence de ces polypeptides et de leurs associations/combinaisons pour le diagnostic sérologique de ce type d'infection. De fait, la combinaison des polypeptides ID SEQ N°12, 14 ou 16 est, elle aussi, pertinente.

Des résultats similaires ont été obtenus pour l'ensemble des polypeptides (polypeptides ID SEQ N°2, 4, 6, 8, 10, 12, 14, 16, 18, 20, 22, 24, 26, 28, 30, 32 et 34).

Il est donc démontré, d'une part, l'existence d'une réponse anticorps (IgG et/ou IgA) significative au cours des infections ostéoarticulaires sur matériel étranger et, d'autre part, que les polypeptides décrits leurs associations/combinaisons sont pertinents pour le diagnostic sérologique de ce type d'infection. De fait, la combinaison d'au moins deux des polypeptides ID SEQ N°2, 4, 6, 8, 10, 12, 14, 16, 18, 20, 22, 24, 26, 28, 30, 32 et 34 est, elle aussi, pertinente. De même, la combinaison des résultats obtenus par la détection des différents isotypes d'anticorps dans les sérums, pour chacun des polypeptides, est elle aussi pertinente.

### C) Utilisation des polypeptides selon l'invention pour le suivi thérapeutique (tableau 6)

Chez des patients en convalescence, la constatation de la diminution, au cours du temps, des valeurs ELISA, obtenues en détectant les immunoglobulines (anticorps) présents dans les sérums, par leur fixation aux antigènes 2B6 ou 2D6-B1 indique leur guérison.

**Tableau 6 : Evolution en fonction de l'anticorps secondaire utilisé**

| Valeur ELISA | | | | | | | | |
|---|---|---|---|---|---|---|---|---|
| Patient 1 | | | | | | Patient 1 | | |
| | | | | | | +1an | | |
| | 2D6B1 | 2B6 | | | | | 2D6B1 | 2B6 |
| anti-IgGAM | 0,99 | 2,3 (+) | | | | anti-IgGAM | 0,44 | 0,74 |
| anti-IgG | 0,4 | 0,51 (+) | | | | anti-IgG | 0,27 | 0,24 |
| anti-IgA | 0,66 | 0,34 | | | | anti-IgA | 0,31 | 0,31 |
| | | | | | | | | |

| Patient 2 | | | | | | Patient 2 | | |
|---|---|---|---|---|---|---|---|---|
| | | | | | | +3mois | | |
| | 2D6B1 | 2B6 | | | | | 2D6B1 | 2B6 |
| anti-IgGAM | 1,03 | 1,43 (+) | | | | anti-IgGAM | 0,44 | 0,74 |
| anti-IgG | 0,5 | 0,46 (+) | | | | anti-IgG | 0,27 | 0,24 |
| anti-IgA | 1,06 (+) | 0,2 | | | | anti-IgA | 0,31 | 0,31 |
| | | | | | | | | |

| Patient 3 | | | | | | Patient 3 | | |
|---|---|---|---|---|---|---|---|---|
| | | | | | | +6mois | | |
| | 2D6B1 | 2B6 | | | | | 2D6B1 | 2B6 |
| anti-IgGAM | 2,27 (+) | 1,6 (+) | | | | anti-IgGAM | 2,41 (+) | 0,86 |
| anti-IgG | 0,94 (+) | 0,5 (+) | | | | anti-IgG | 0,87 (+) | 0,22 |
| anti-IgA | 0,14 | 0,19 | | | | anti-IgA | 0,15 | 0,19 |

| | | | | | | | | |
|---|---|---|---|---|---|---|---|---|
| **Les valeurs ELISA notées (+) sont considérées positives** | | | | | | | | |

Dans le tableau 6, les individus initialement positifs selon l'invention deviennent négatifs.

L'utilisation des polypeptides selon l'invention permet donc de réaliser des suivis thérapeutiques, c'est-à-dire (i) d'évaluer l'effet d'un traitement et (ii) de suivre l'évolution post-opératoire (ou post-implantatoire) d'un patient.

### D) Optimisation des antigènes et des tests de diagnostic in vitro

La recherche de fragments afin d'améliorer, d'une part, les performances des antigènes et, d'autre part, la production des polypeptides est particulièrement utile pour leur utilisation industrielle et la pertinence des tests.

### E) Combinaison multiparamétrique et évaluation par syndrome.

L'utilisation conjointe de plusieurs des polypeptides décrits, permet en outre :
(i) d'augmenter la sensibilité de la détection ;
(ii) d'évaluer la possibilité de la présence de plusieurs germes (par exemple par l'utilisation d'antigène spécifique d'un germe ou d'une famille de germes) ;
(iii) de préciser le génotype de la souche ;
(iv) de déterminer l'évolution de la pathologie (par l'utilisation de certains antigènes et d'isotypes d'anticorps) ;
(v) de réaliser des suivis thérapeutiques à moindre coût ; et
(vi) de réaliser des diagnostics par syndrome.

En conclusion, la présente invention décrit des polypeptides optimisés quant à leur sensibilité et à leur spécificité, permettant de les utiliser comme sonde (antigène) dans des tests. Elle permet, sans intervention chirurgicale, le diagnostic d'infections staphylococciques, sur matériel étranger, tel que prothèses ostéo-articulaires, notamment d'infections non diagnostiquées par culture de prélèvements per-opératoires profonds.

## Revendications

1. Utilisation pour détecter, *in vitro,* dans des échantillons biologiques, la présence d'anticorps produits à la suite d'une infection à *Staphylococcus epidermidis* et/ou *aureus* d'un polypeptide de séquence d'acides aminés ID SEQ N°10.

2. Utilisation pour détecter, *in vitro*, dans des échantillons biologiques, la présence d'anticorps produits à la suite d'une infection à *Staphylococcus epidermidis* et/ou *aureus* d'un polypeptide consistant en :
a) une partie de la séquence d'acides aminés telle que définie selon la revendication 1 et ayant la même fonction que ladite séquence, ou
b) une séquence d'acides aminés ayant au moins 60% d'identité avec la séquence d'acides aminés telle que définie selon la revendication 1 ou avec une partie de la séquence telle que définie sous a), et ayant la même fonction que ladite séquence,
étant entendu que, par « ayant la même fonction que ladite séquence », on entend une fonction d'antigène de *Staphylococcus epidermidis* et/ou *aureus*.

3. Utilisation selon la revendication 1 ou 2, **caractérisée en ce que** le polypeptide est préparé par culture d'une cellule hôte comprenant un vecteur recombinant ayant, inséré, un polynucléotide codant pour ledit polypeptide et par isolement dudit polypeptide du milieu de culture.

4. Utilisation selon la revendication 3, quand elle dépend de la revendication 1, **caractérisée en ce que** le polynucléotide est de séquence ID SEQ N°9.

5. Utilisation pour détecter, *in vitro*, dans des échantillons biologiques, la présence d'anticorps produits à la suite d'une infection à *Staphylococcus epidermidis* et/ou *aureus*, d'un polynucléotide de séquence ID SEQ N°9.

6. Utilisation pour détecter, *in vitro,* dans des échantillons biologiques, la présence d'anticorps produits à la suite d'une infection à *Staphylococcus epidermidis* et/ou *aureus*, d'un polynucléotide consistant en :
a) une partie de la séquence telle que définie selon la revendication 5 et ayant la même fonction que ladite séquence, ou
b) une séquence polynucléotidique ayant au moins 60% d'identité avec la séquence polynucléotidique telle que définie selon la revendication 5 ou avec une partie de la séquence telle que définie sous a), et ayant la même fonction que ladite séquence, ou
c) une séquence polynucléotidique complémentaire à une des séquences polynucléotidiques telles que définies selon la revendication 5 ou selon la revendication 6,
étant entendu que, par « ayant la même fonction que ladite séquence », on entend une fonction de codage d'un polypeptide ayant lui-même une fonction d'antigène de *Staphylococcus epidermidis* et/ou *aureus*.

7. Utilisation selon l'une quelconque des revendications 1 à 6 **caractérisée en ce que** ladite infection à *Staphylococcus epidermidis* et/ou aureus est sur un matériel étranger implanté dans le corps.

## Claims

1. Use of a polypeptide of amino acid sequence SEQ ID No. 10 for the *in vitro* detection, in biological samples, of the presence of antibodies produced following a *Staphylococcus epidermidis* and/or aureus infection.

2. Use of a polypeptide comprising:
a) part of the amino acid sequence as defined according to claim 1 and having the same function as said sequence, or
b) an amino acid sequence having at least 60% identity with the amino acid sequence as defined according to claim 1 or with part of the sequence as defined under a), and having the same function as said sequence,
for the *in vitro* detection, in biological samples, of the presence of antibodies produced following a *Staphylococcus epidermidis* and/or *aureus* infection,
it being understood that "having the same function as said sequence" is understood as meaning a *Staphylococcus epidermidis* and/or aureus antigen function.

3. Use according to claim 1 or 2, **characterised in that** the polypeptide is prepared by culturing a host cell comprising a recombinant vector having, inserted therein, a polynucleotide coding for said polypeptide, and by isolating said polypeptide from the culture medium.

4. Use according to claim 3, when dependent on claim 1, **characterised in that** the polynucleotide is of sequence SEQ ID No. 9.

5. Use of a polypeptide of sequence SEQ ID No. 9 for the *in vitro* detection, in biological samples, of the presence of antibodies produced following a *Staphylococcus epidermidis* and/or aureus infection.

6. Use of a polynucleotide comprising:
a) part of the sequence as defined according to claim 5 and having the same function as said sequence, or
b) a polynucleotide sequence having at least 60% identity with the polynucleotide sequence as defined according to claim 5 or with part of the sequence as defined under a), and having the same function as said sequence, or
c) a polynucleotide sequence that is complementary to one of the polynucleotide sequences as defined according to claim 5 or according to claim 6,
for the *in vitro* detection, in biological samples, of the presence of antibodies produced following a *Staphylococcus epidermidis* and/or *aureus* infection, it being understood that "having the same function as said sequence" is understood as meaning a function of coding for a polypeptide that itself has a *Staphylococcus epidermidis* and/or *aureus* antigen function.

7. Use according to any one of claims 1 to 6, **characterised in that** said *Staphylococcus epidermidis* and/or *aureus* infection is on a foreign material implanted in the body.

## Patentansprüche

1. Verwendung von einem Polypeptid der Aminosäurensequenz ID SEQ N°10, um *in vitro* das Vorhandensein von Antikörpern in biologischen Proben zu detektieren, die als Folge einer *Staphylococcus epidermis* und/oder *aureus* Infektion erzeugt werden.

2. Verwendung von einem Polypeptid, um *in vitro* das Vorhandensein von Antikörpern in biologischen Proben zu detektieren, die als Folge einer *Staphylococcus epidermis* und/oder *aureus* Infektion erzeugt werden, wobei das Polypeptid besteht aus:
a) einem Teil der Aminosäuresequenz, wie sie nach Anspruch 1 definiert ist, und die die gleiche Funktion wie die genannte Sequenz hat, oder
b) einer Aminosäuresequenz, die mindestens eine 60%ige Identität mit der Aminosäuresequenz, wie sie nach Anspruch 1 definiert ist, hat oder einer Aminosäuresequenz mit einem Teil der Sequenz, wie sie unter a) definiert ist, und die die gleiche Funktion wie die genannte Sequenz hat,
wobei klargestellt wird, dass unter "die gleiche Funktion wie die genannte Sequenz habend" eine Funktion von einem Antigen von *Staphylococcus epidermis* und/oder *aureus* verstanden wird.

3. Verwendung nach Anspruch 1 oder Anspruch 2, **dadurch gekennzeichnet, dass** das Polypeptid durch Kultur einer Wirtzelle, die einen rekombinierenden Vektor umfasst, in den ein für das Polypeptid kodierendes Polynukleotid eingefügt ist, und durch Isolierung des Polypeptids aus dem Kulturmedium bereitgestellt wird.

4. Verwendung nach Anspruch 3, wenn er von Anspruch 1 abhängt, **dadurch gekennzeichnet, dass** das Polynukleotid von einer Sequenz ID SEQ N°9 ist.

5. Verwendung von einem Polynukleotid der Sequenz ID SEQ N°9, um in *vitro* das Vorhandensein von Antikörpern in biologischen Proben zu detektieren, die als Folge einer *Staphylococcus epidermis* und/oder *aureus* Infektion erzeugt werden.

6. Verwendung von einem Polynukleotid, um *in vitro* das Vorhandensein von Antikörpern in biologischen Proben zu detektieren, die als Folge einer *Staphylococcus epidermis* und/oder *aureus* Infektion erzeugt werden, wobei das Polypeptid besteht aus:
a) einem Teil der Sequenz, wie sie nach Anspruch 5 definiert ist und die gleiche Funktion wie die Sequenz hat, oder
b) einer Polynukleotidsequenz, die mindestens eine 60%ige identität mit der Polynukleotidsequenz, wie sie im Anspruch 5 definiert ist, hat oder einer Polynukleotidsequenz mit einem Teil der Sequenz, wie sie unter a) definiert ist und die die gleiche Funktion wie die Sequenz hat, oder
c) einer Polynukleotidsequenz, die komplementär zu einer der Polynukleotidsequenzen, wie sie nach dem Anspruch 5 oder dem Anspruch 6 definiert sind, ist
wobei klargestellt wird, dass unter "die gleiche Funktion wie die genannte Sequenz habend" eine Kodierungsfunktion für ein Polypeptid verstanden wird, das selbst eine Funktion von einem Antigen von *Staphylococcus epidermis* und/oder *aureus* hat.

7. Verwendung nach einem beliebigen der Ansprüche 1 bis 6, **dadurch gekennzeichnet, dass** die *Staphylococcus epidermis* und/oder *aureus* infektion auf einem in den Körper implantierten Fremdmaterial ist.


### LISTAGE DE SEQUENCES

<110> ABAG
<120> Nouveaux polypeptides pour la mise en évidence in vitro et la prévention des infections staphylococciques sur prothèses articulaires et autres matériels étrangers implantés.
<130> B3875
<160> 36
<170> PatentIn version 3.1
<210> 1
   <211> 1485
   <212> DNA
   <213> Staphylococcus epidermidis
<220>
   <221> CDS
   <222> (1)..(1485)
   <223>
<400> 1
   gga act aat aat aaa tta act gtg tct gct aat cgt ggt gtt gct caa       48
   Gly Thr Asn Asn Lys Leu Thr Val Ser Ala Asn Arg Gly Val Ala Gln
   1               5                   10                  15
   att aaa cca aca aat aat ggc tta tat aca act gtt tat gac agt aaa       96
   Ile Lys Pro Thr Asn Asn Gly Leu Tyr Thr Thr Val Tyr Asp Ser Lys
               20                  25                  30
   ggt cat aag act gat caa gta caa aaa act cta tcc gtt act aaa act      144
   Gly His Lys Thr Asp Gln Val Gln Lys Thr Leu Ser Val Thr Lys Thr
           35                  40                  45
   gca aca tta gga aat aac aaa ttc tat tta gtt gaa gac tac aat agc      192
   Ala Thr Leu Gly Asn Asn Lys Phe Tyr Leu Val Glu Asp Tyr Asn Ser
       50                  55                  60
   ggt aaa aaa tac ggt tgg gtt aaa caa ggt gat gtt gtt tat aac act      240
   Gly Lys Lys Tyr Gly Trp Val Lys Gln Gly Asp Val Val Tyr Asn Thr
   65                  70                  75                  80
   gct aag gca cca gta aaa gtg aat caa aca tat aat gtt aaa gca ggg      288
   Ala Lys Ala Pro Val Lys Val Asn Gln Thr Tyr Asn Val Lys Ala Gly
                   85                  90                  95
   tca aca ctt tac aca gtt cct tgg ggt aca cca aaa caa gtt gct agc      336
   Ser Thr Leu Tyr Thr Val Pro Trp Gly Thr Pro Lys Gln Val Ala Ser
               100                 105                 110
   aaa gta tct ggt act gga aat caa aca ttt aaa gca act aaa cag caa      384
   Lys Val Ser Gly Thr Gly Asn Gln Thr Phe Lys Ala Thr Lys Gln Gln
           115                 120                 125
   caa att gat aaa gca acg tat ctt tat ggt aca gtg aat ggt aaa tct      432
   Gln Ile Asp Lys Ala Thr Tyr Leu Tyr Gly Thr Val Asn Gly Lys Ser
       130                 135                 140
   ggt tgg att agt aaa tat tac tta act aca gca tct aaa cct agc aat      480
   Gly Trp Ile Ser Lys Tyr Tyr Leu Thr Thr Ala Ser Lys Pro Ser Asn
   145                 150                 155                 160
   cca act aaa cct tca aca aac aac caa tta aca gtg act aac aat agt      528
   Pro Thr Lys Pro Ser Thr Asn Asn Gln Leu Thr Val Thr Asn Asn Ser
                   165                 170                 175
   ggt gtt gct caa atc aat gca aaa aat agt ggc tta tat act aca gtt      576
   Gly Val Ala Gln Ile Asn Ala Lys Asn Ser Gly Leu Tyr Thr Thr Val
               180                 185                 190
   tat gac act aaa gga aag aca aca aat caa atc caa cgt aca ttg tca      624
   Tyr Asp Thr Lys Gly Lys Thr Thr Asn Gln Ile Gln Arg Thr Leu Ser
           195                 200                 205
   gtg acg aaa gct gcc aca ctt ggt gat aaa aaa ttc tat ctt gtt ggt      672
   Val Thr Lys Ala Ala Thr Leu Gly Asp Lys Lys Phe Tyr Leu Val Gly
       210                 215                 220
   gat tat aat act ggt aca aat tat ggt tgg gta aaa caa gat gag gtc      720
   Asp Tyr Asn Thr Gly Thr Asn Tyr Gly Trp Val Lys Gln Asp Glu Val
   225                 230                 235                 240
   att tac aac aca gct aaa tca cct gta aaa atc aat caa aca tac aac      768
   Ile Tyr Asn Thr Ala Lys Ser Pro Val Lys Ile Asn Gln Thr Tyr Asn
                   245                 250                 255
   gtc aaa cct ggt gtt aaa tta cac aca gta cct tgg ggc aca tat aat      816
   Val Lys Pro Gly Val Lys Leu His Thr Val Pro Trp Gly Thr Tyr Asn
               260                 265                 270
   caa gtg gct gga aca gtt tca ggt aaa ggc gat caa act ttt aaa gca      864
   Gln Val Ala Gly Thr Val Ser Gly Lys Gly Asp Gln Thr Phe Lys Ala
           275                 280                 285
   act aaa caa caa caa att gat aaa gca aca tat ctt tat ggt aca gtg      912
   Thr Lys Gln Gln Gln Ile Asp Lys Ala Thr Tyr Leu Tyr Gly Thr Val
       290                 295                 300
   aac ggt aaa tct ggt tgg att agt aaa tac tat tta act gca cca tca      960
   Asn Gly Lys Ser Gly Trp Ile Ser Lys Tyr Tyr Leu Thr Ala Pro Ser
   305                 310                 315                 320
   aaa gtt caa gct ttg tct act caa tca aca cca gca cct aaa caa gta     1008
   Lys Val Gln Ala Leu Ser Thr Gln Ser Thr Pro Ala Pro Lys Gln Val
                   325                 330                 335
   aaa cca tct aca caa act gta aat caa att gct caa gtg aaa gct aat     1056
   Lys Pro Ser Thr Gln Thr Val Asn Gln Ile Ala Gln Val Lys Ala Asn
               340                 345                 350
   aat tct gga ata aga gca tct gta tat gat aaa aca gcc aaa agt ggt     1104
   Asn Ser Gly Ile Arg Ala Ser Val Tyr Asp Lys Thr Ala Lys Ser Gly
           355                 360                 365
   acg aaa tac gct aac cgt aca ttc ctt atc aat aaa caa cgt act caa     1152
   Thr Lys Tyr Ala Asn Arg Thr Phe Leu Ile Asn Lys Gln Arg Thr Gln
       370                 375                 380
   ggt aat aac acg tat gta cta ctt caa gat gga aca agt aat act cca     1200
   Gly Asn Asn Thr Tyr Val Leu Leu Gln Asp Gly Thr Ser Asn Thr Pro
   385                 390                 395                 400
   tta gga tgg gta aac att aat gat gtg aca act caa aat atc gga aaa     1248
   Leu Gly Trp Val Asn Ile Asn Asp Val Thr Thr Gln Asn Ile Gly Lys
                   405                 410                 415
   caa act cag tct ata ggt aaa tat tca gta aaa cct aca aat aat ggt     1296
   Gln Thr Gln Ser Ile Gly Lys Tyr Ser Val Lys Pro Thr Asn Asn Gly
               420                 425                 430
   cta tat tct att gct tgg ggt act aaa aac caa caa tta cta gca cct     1344
   Leu Tyr Ser Ile Ala Trp Gly Thr Lys Asn Gln Gln Leu Leu Ala Pro
           435                 440                 445
   aat acg cta gct aat caa gca ttt aat gct tcc aaa gct gtt tac gtt     1392
   Asn Thr Leu Ala Asn Gln Ala Phe Asn Ala Ser Lys Ala Val Tyr Val
       450                 455                 460
   ggt aaa gat tta tat cta tac ggt aca gtc aat aac aga aca gga tgg     1440
   Gly Lys Asp Leu Tyr Leu Tyr Gly Thr Val Asn Asn Arg Thr Gly Trp
   465                 470                 475                 480
   att gct gct aag gat tta atc caa aac agt act gac gct caa tca         1485
   Ile Ala Ala Lys Asp Leu Ile Gln Asn Ser Thr Asp Ala Gln Ser
                   485                 490                 495
<210> 2
   <211> 495
   <212> PRT
   <213> Staphylococcus epidermidis
<400> 2
   Gly Thr Asn Asn Lys Leu Thr Val Ser Ala Asn Arg Gly Val Ala Gln
   1               5                   10                  15
   Ile Lys Pro Thr Asn Asn Gly Leu Tyr Thr Thr Val Tyr Asp Ser Lys
               20                  25                  30
   Gly His Lys Thr Asp Gln Val Gln Lys Thr Leu Ser Val Thr Lys Thr
           35                  40                  45
   Ala Thr Leu Gly Asn Asn Lys Phe Tyr Leu Val Glu Asp Tyr Asn Ser
       50                  55                  60
   Gly Lys Lys Tyr Gly Trp Val Lys Gln Gly Asp Val Val Tyr Asn Thr
   65                  70                  75                  80
   Ala Lys Ala Pro Val Lys Val Asn Gln Thr Tyr Asn Val Lys Ala Gly
                   85                  90                  95
   Ser Thr Leu Tyr Thr Val Pro Trp Gly Thr Pro Lys Gln Val Ala Ser
               100                 105                 110
   Lys Val Ser Gly Thr Gly Asn Gln Thr Phe Lys Ala Thr Lys Gln Gln
           115                 120                 125
   Gln Ile Asp Lys Ala Thr Tyr Leu Tyr Gly Thr Val Asn Gly Lys Ser
       130                 135                 140
   Gly Trp Ile Ser Lys Tyr Tyr Leu Thr Thr Ala Ser Lys Pro Ser Asn
   145                 150                 155                 160
   Pro Thr Lys Pro Ser Thr Asn Asn Gln Leu Thr Val Thr Asn Asn Ser
                   165                 170                 175
   Gly Val Ala Gln Ile Asn Ala Lys Asn Ser Gly Leu Tyr Thr Thr Val
               180                 185                 190
   Tyr Asp Thr Lys Gly Lys Thr Thr Asn Gln Ile Gln Arg Thr Leu Ser
           195                 200                 205
   Val Thr Lys Ala Ala Thr Leu Gly Asp Lys Lys Phe Tyr Leu Val Gly
       210                 215                 220
   Asp Tyr Asn Thr Gly Thr Asn Tyr Gly Trp Val Lys Gln Asp Glu Val
   225 230 235 240
   Ile Tyr Asn Thr Ala Lys Ser Pro Val Lys Ile Asn Gln Thr Tyr Asn
   245 250 255
   Val Lys Pro Gly Val Lys Leu His Thr Val Pro Trp Gly Thr Tyr Asn
   260 265 270
   Gln Val Ala Gly Thr Val Ser Gly Lys Gly Asp Gln Thr Phe Lys Ala
   275 280 285
   Thr Lys Gln Gln Gln Ile Asp Lys Ala Thr Tyr Leu Tyr Gly Thr Val
   290 295 300
   Asn Gly Lys Ser Gly Trp Ile Ser Lys Tyr Tyr Leu Thr Ala Pro Ser
   305 310 315 320
   Lys Val Gln Ala Leu Ser Thr Gln Ser Thr Pro Ala Pro Lys Gln Val
   325 330 335
   Lys Pro Ser Thr Gln Thr Val Asn Gln Ile Ala Gln Val Lys Ala Asn
   340 345 350
   Asn Ser Gly Ile Arg Ala Ser Val Tyr Asp Lys Thr Ala Lys Ser Gly
   355 360 365
   Thr Lys Tyr Ala Asn Arg Thr Phe Leu Ile Asn Lys Gln Arg Thr Gln
   370 375 380
   Gly Asn Asn Thr Tyr Val Leu Leu Gln Asp Gly Thr Ser Asn Thr Pro
   385 390 395 400
   Leu Gly Trp Val Asn Ile Asn Asp Val Thr Thr Gln Asn Ile Gly Lys
   405 410 415
   Gln Thr Gln Ser Ile Gly Lys Tyr Ser Val Lys Pro Thr Asn Asn Gly
   420 425 430
   Leu Tyr Ser Ile Ala Trp Gly Thr Lys Asn Gln Gln Leu Leu Ala Pro
   435 440 445
   Asn Thr Leu Ala Asn Gln Ala Phe Asn Ala Ser Lys Ala Val Tyr Val
   450 455 460
   Gly Lys Asp Leu Tyr Leu Tyr Gly Thr Val Asn Asn Arg Thr Gly Trp
   465                 470                 475                 480
   Ile Ala Ala Lys Asp Leu Ile Gln Asn Ser Thr Asp Ala Gln Ser
                   485                 490                 495
<210> 3
   <211> 561
   <212> DNA
   <213> Staphylococcus epidermidis
<220>
   <221> CDS
   <222> (1)..(561)
   <223>
<400> 3
   gga act aat aat aaa tta act gtg tct gct aat cgt ggt gtt gct caa      48
   Gly Thr Asn Asn Lys Leu Thr Val Ser Ala Asn Arg Gly Val Ala Gln
   1               5                   10                  15
   att aaa cca aca aat aat ggc tta tat aca act gtt tat gac agt aaa      96
   Ile Lys Pro Thr Asn Asn Gly Leu Tyr Thr Thr Val Tyr Asp Ser Lys
               20                  25                  30
   ggt cat aag act gat caa gta caa aaa act cta tcc gtt act aaa act     144
   Gly His Lys Thr Asp Gln Val Gln Lys Thr Leu Ser Val Thr Lys Thr
           35                  40                  45
   gca aca tta gga aat aac aaa ttc tat tta gtt gaa gac tac aat agc     192
   Ala Thr Leu Gly Asn Asn Lys Phe Tyr Leu Val Glu Asp Tyr Asn Ser
       50                  55                  60
   ggt aaa aaa tac ggt tgg gtt aaa caa ggt gat gtt gtt tat aac act     240
   Gly Lys Lys Tyr Gly Trp Val Lys Gln Gly Asp Val Val Tyr Asn Thr
   65                  70                  75                  80
   gct aag gca cca gta aaa gtg aat caa aca tat aat gtt aaa gca ggg     288
   Ala Lys Ala Pro Val Lys Val Asn Gln Thr Tyr Asn Val Lys Ala Gly
                   85                  90                  95
   tca aca ctt tac aca gtt cct tgg ggt aca cca aaa caa gtt gct agc     336
   Ser Thr Leu Tyr Thr Val Pro Trp Gly Thr Pro Lys Gln Val Ala Ser
               100                 105                 110
   aaa gta tct ggt act gga aat caa aca ttt aaa gca act aaa cag caa     384
   Lys Val Ser Gly Thr Gly Asn Gln Thr Phe Lys Ala Thr Lys Gln Gln
           115                 120                 125
   caa att gat aaa gca acg tat ctt tat ggt aca gtg aat ggt aaa tct        432
   Gln Ile Asp Lys Ala Thr Tyr Leu Tyr Gly Thr Val Asn Gly Lys Ser
       130                 135                 140
   ggt tgg att agt aaa tat tac tta act aca gca tct aaa cct agc aat        480
   Gly Trp Ile Ser Lys Tyr Tyr Leu Thr Thr Ala Ser Lys Pro Ser Asn
   145                 150                 155                 160
   cca act aaa cct tca aca aac aac caa tta aca gtg act aac aat agt        528
   Pro Thr Lys Pro Ser Thr Asn Asn Gln Leu Thr Val Thr Asn Asn Ser
                   165                 170                 175
   ggt gtt gct caa atc aat gca aaa aat agt ggc                            561
   Gly Val Ala Gln Ile Asn Ala Lys Asn Ser Gly
               180                 185
<210> 4
   <211> 187
   <212> PRT
   <213> Staphylococcus epidermidis
<400> 4
   Gly Thr Asn Asn Lys Leu Thr Val Ser Ala Asn Arg Gly Val Ala Gln
   1               5                   10                  15
   Ile Lys Pro Thr Asn Asn Gly Leu Tyr Thr Thr Val Tyr Asp Ser Lys
               20                  25                  30
   Gly His Lys Thr Asp Gln Val Gln Lys Thr Leu Ser Val Thr Lys Thr
           35                  40                  45
   Ala Thr Leu Gly Asn Asn Lys Phe Tyr Leu Val Glu Asp Tyr Asn Ser
       50                  55                  60
   Gly Lys Lys Tyr Gly Trp Val Lys Gln Gly Asp Val Val Tyr Asn Thr
   65                  70                  75                  80
   Ala Lys Ala Pro Val Lys Val Asn Gln Thr Tyr Asn Val Lys Ala Gly
                   85                  90                  95
   Ser Thr Leu Tyr Thr Val Pro Trp Gly Thr Pro Lys Gln Val Ala Ser
               100                 105                 110
   Lys Val Ser Gly Thr Gly Asn Gln Thr Phe Lys Ala Thr Lys Gln Gln
           115                 120                 125
   Gln Ile Asp Lys Ala Thr Tyr Leu Tyr Gly Thr Val Asn Gly Lys Ser
       130                 135                 140
   Gly Trp Ile Ser Lys Tyr Tyr Leu Thr Thr Ala Ser Lys Pro Ser Asn
   145                 150                 155                 160
   Pro Thr Lys Pro Ser Thr Asn Asn Gln Leu Thr Val Thr Asn Asn Ser
                   165                 170                 175
   Gly Val Ala Gln Ile Asn Ala Lys Asn Ser Gly
               180                 185
<210> 5
   <211> 1524
   <212> DNA
   <213> Staphylococcus aureus
<220>
   <221> CDS
   <222> (1)..(1524)
   <223>
<400> 5
   tca act ggt aaa tta aca gtt gct gca aac aat ggt gtc gca caa atc      48
   Ser Thr Gly Lys Leu Thr Val Ala Ala Asn Asn Gly Val Ala Gln Ile
   1               5                   10                  15
   aaa cca aca aat agt ggt tta tat act act gta tac gac aaa act ggt      96
   Lys Pro Thr Asn Ser Gly Leu Tyr Thr Thr Val Tyr Asp Lys Thr Gly
               20                  25                  30
   aaa gca act aat gaa gtt caa aaa aca ttt gct gta tct aaa aca gct     144
   Lys Ala Thr Asn Glu Val Gln Lys Thr Phe Ala Val Ser Lys Thr Ala
           35                  40                  45
   aca tta ggt aat caa aaa ttc tat ctt gtt caa gat tac aat tct ggt     192
   Thr Leu Gly Asn Gln Lys Phe Tyr Leu Val Gln Asp Tyr Asn Ser Gly
       50                  55                  60
   aat aaa ttt ggt tgg gtt aaa gaa ggc gat gtg gtt tac aac aca gct     240
   Asn Lys Phe Gly Trp Val Lys Glu Gly Asp Val Val Tyr Asn Thr Ala
   65                  70                  75                  80
   aaa tca cct gta aat gta aat caa tca tat tca atc aaa cct ggt acg     288
   Lys Ser Pro Val Asn Val Asn Gln Ser Tyr Ser Ile Lys Pro Gly Thr
                   85                  90                  95
   aaa ctt tat aca gta cct tgg ggt aca tct aaa caa gtt gct ggt agt      336
   Lys Leu Tyr Thr Val Pro Trp Gly Thr Ser Lys Gln Val Ala Gly Ser
               100                 105                 110
   gtg tct ggc tct gga aac caa aca ttt aag gct tca aag caa caa caa      384
   Val Ser Gly Ser Gly Asn Gln Thr Phe Lys Ala Ser Lys Gln Gln Gln
           115                 120                 125
   att gat aaa tca att tat tta tat ggc tct gtg aat ggt aaa tct ggt      432
   Ile Asp Lys Ser Ile Tyr Leu Tyr Gly Ser Val Asn Gly Lys Ser Gly
       130                 135                 140
   tgg gta agt aaa gca tat tta gtt gat act gct aaa cct acg cct aca      480
   Trp Val Ser Lys Ala Tyr Leu Val Asp Thr Ala Lys Pro Thr Pro Thr
   145                 150                 155                 160
   cca aca cct aag cca tca aca cct aca aca aat aat aaa tta aca gtt      528
   Pro Thr Pro Lys Pro Ser Thr Pro Thr Thr Asn Asn Lys Leu Thr Val
                   165                 170                 175
   tca tca tta aac ggt gtt gct caa att aat gct aaa aac aat ggc tta      576
   Ser Ser Leu Asn Gly Val Ala Gln Ile Asn Ala Lys Asn Asn Gly Leu
               180                 185                 190
   ttc act aca gtt tat gac aaa act ggt aag cca acg aaa gaa gtt caa      624
   Phe Thr Thr Val Tyr Asp Lys Thr Gly Lys Pro Thr Lys Glu Val Gln
           195                 200                 205
   aaa aca ttt gct gta aca aaa gaa gca agt tta ggt gga aac aaa ttc      672
   Lys Thr Phe Ala Val Thr Lys Glu Ala Ser Leu Gly Gly Asn Lys Phe
       210                 215                 220
   tac tta gtt aaa gat tac aat agt cca act tta att ggt tgg gtt aaa      720
   Tyr Leu Val Lys Asp Tyr Asn Ser Pro Thr Leu Ile Gly Trp Val Lys
   225                 230                 235                 240
   caa ggt gac gtt att tat aac aat gca aaa tca cct gta aat gta atg      768
   Gln Gly Asp Val Ile Tyr Asn Asn Ala Lys Ser Pro Val Asn Val Met
                   245                 250                 255
   caa aca tat aca gta aaa cca ggc act aaa tta tat tca gta cct tgg      816
   Gln Thr Tyr Thr Val Lys Pro Gly Thr Lys Leu Tyr Ser Val Pro Trp
               260                 265                 270
   ggc act tat aaa caa gaa gct ggt gca gtt tct ggt aca ggt aac caa      864
   Gly Thr Tyr Lys Gln Glu Ala Gly Ala Val Ser Gly Thr Gly Asn Gln
           275                 280                 285
   act ttt aaa gcg act aag caa caa caa att gat aaa tct atc tat tta      912
   Thr Phe Lys Ala Thr Lys Gln Gln Gln Ile Asp Lys Ser Ile Tyr Leu
       290                 295                 300
   ttt gga act gta aat ggt aaa tct ggt tgg gta agt aaa gca tat tta      960
   Phe Gly Thr Val Asn Gly Lys Ser Gly Trp Val Ser Lys Ala Tyr Leu
   305                 310                 315                 320
   gct gta cct gct gca cct aaa aaa gca gta gca caa cca aaa aca gct     1008
   Ala Val Pro Ala Ala Pro Lys Lys Ala Val Ala Gln Pro Lys Thr Ala
                   325                 330                 335
   gta aaa gct tat act gtt act aaa cca caa acg act caa aca gtt agc      1056
   Val Lys Ala Tyr Thr Val Thr Lys Pro Gln Thr Thr Gln Thr Val Ser
               340                 345                 350
   aag att gct caa gtt aaa cca aac aac act ggt att cgt gct tct gtt      1104
   Lys Ile Ala Gln Val Lys Pro Asn Asn Thr Gly Ile Arg Ala Ser Val
           355                 360                 365
   tat gaa aaa aca gcg aaa aac ggt gcg aaa tat gca gac cgt acg ttc      1152
   Tyr Glu Lys Thr Ala Lys Asn Gly Ala Lys Tyr Ala Asp Arg Thr Phe
       370                 375                 380
   tat gta aca aaa gag cgt gct cat ggt aat gaa acg tat gta tta tta      1200
   Tyr Val Thr Lys Glu Arg Ala His Gly Asn Glu Thr Tyr Val Leu Leu
   385                 390                 395                 400
   aac aat aca agc cat aac atc cca tta ggt tgg ttc aat gta aaa gac      1248
   Asn Asn Thr Ser His Asn Ile Pro Leu Gly Trp Phe Asn Val Lys Asp
                   405                 410                 415
   tta aat gtt caa aac tta ggc aaa gaa gtt aaa acg act caa aaa tat      1296
   Leu Asn Val Gln Asn Leu Gly Lys Glu Val Lys Thr Thr Gln Lys Tyr
               420                 425                 430
   act gtt aat aaa tca aat aac ggc tta tca atg gtt cct tgg ggt act      1344
   Thr Val Asn Lys Ser Asn Asn Gly Leu Ser Met Val Pro Trp Gly Thr
           435                 440                 445
   aaa aac caa gtc att tta aca ggc aat aac att gct caa ggt aca ttt      1392
   Lys Asn Gln Val Ile Leu Thr Gly Asn Asn Ile Ala Gln Gly Thr Phe
       450                 455                 460
   aat gca acg aaa caa gta tct gta ggc aaa gat gtt tat tta tac ggt      1440
   Asn Ala Thr Lys Gln Val Ser Val Gly Lys Asp Val Tyr Leu Tyr Gly
   465                 470                 475                 480
   act att aat aac cgc act ggt tgg gta aat gca aaa gat tta act gca      1488
   Thr Ile Asn Asn Arg Thr Gly Trp Val Asn Ala Lys Asp Leu Thr Ala
                   485                 490                 495
   cca act gct gtt aaa cca act aca tca gct gcc aaa                      1524
   Pro Thr Ala Val Lys Pro Thr Thr Ser Ala Ala Lys
               500                 505
<210> 6
   <211> 508
   <212> PRT
   <213> Staphylococcus aureus
<400> 6
   Ser Thr Gly Lys Leu Thr Val Ala Ala Asn Asn Gly Val Ala Gln Ile
   1               5                   10                  15
   Lys Pro Thr Asn Ser Gly Leu Tyr Thr Thr Val Tyr Asp Lys Thr Gly
               20                  25                  30
   Lys Ala Thr Asn Glu Val Gln Lys Thr Phe Ala Val Ser Lys Thr Ala
           35                  40                  45
   Thr Leu Gly Asn Gln Lys Phe Tyr Leu Val Gln Asp Tyr Asn Ser Gly
       50                  55                  60
   Asn Lys Phe Gly Trp Val Lys Glu Gly Asp Val Val Tyr Asn Thr Ala
   65                  70                  75                  80
   Lys Ser Pro Val Asn Val Asn Gln Ser Tyr Ser Ile Lys Pro Gly Thr
                   85                  90                  95
   Lys Leu Tyr Thr Val Pro Trp Gly Thr Ser Lys Gln Val Ala Gly Ser
               100                 105                 110
   Val Ser Gly Ser Gly Asn Gln Thr Phe Lys Ala Ser Lys Gln Gln Gln
           115                 120                 125
   Ile Asp Lys Ser Ile Tyr Leu Tyr Gly Ser Val Asn Gly Lys Ser Gly
       130                 135                 140
   Trp Val Ser Lys Ala Tyr Leu Val Asp Thr Ala Lys Pro Thr Pro Thr
   145                 150                 155                 160
   Pro Thr Pro Lys Pro Ser Thr Pro Thr Thr Asn Asn Lys Leu Thr Val
                   165                 170                 175
   Ser Ser Leu Asn Gly Val Ala Gln Ile Asn Ala Lys Asn Asn Gly Leu
               180                 185                 190
   Phe Thr Thr Val Tyr Asp Lys Thr Gly Lys Pro Thr Lys Glu Val Gln
           195                 200                 205
   Lys Thr Phe Ala Val Thr Lys Glu Ala Ser Leu Gly Gly Asn Lys Phe
       210                 215                 220
   Tyr Leu Val Lys Asp Tyr Asn Ser Pro Thr Leu Ile Gly Trp Val Lys
   225                 230                 235                 240
   Gln Gly Asp Val Ile Tyr Asn Asn Ala Lys Ser Pro Val Asn Val Met
                   245                 250                 255
   Gln Thr Tyr Thr Val Lys Pro Gly Thr Lys Leu Tyr Ser Val Pro Trp
               260                 265                 270
   Gly Thr Tyr Lys Gln Glu Ala Gly Ala Val Ser Gly Thr Gly Asn Gln
           275                 280                 285
   Thr Phe Lys Ala Thr Lys Gln Gln Gln Ile Asp Lys Ser Ile Tyr Leu
       290                 295                 300
   Phe Gly Thr Val Asn Gly Lys Ser Gly Trp Val Ser Lys Ala Tyr Leu
   305                 310                 315                 320
   Ala Val Pro Ala Ala Pro Lys Lys Ala Val Ala Gln Pro Lys Thr Ala
                   325                 330                 335
   Val Lys Ala Tyr Thr Val Thr Lys Pro Gln Thr Thr Gln Thr Val Ser
               340                 345                 350
   Lys Ile Ala Gln Val Lys Pro Asn Asn Thr Gly Ile Arg Ala Ser Val
           355                 360                 365
   Tyr Glu Lys Thr Ala Lys Asn Gly Ala Lys Tyr Ala Asp Arg Thr Phe
       370                 375                 380
   Tyr Val Thr Lys Glu Arg Ala His Gly Asn Glu Thr Tyr Val Leu Leu
   385                 390                 395                 400
   Asn Asn Thr Ser His Asn Ile Pro Leu Gly Trp Phe Asn Val Lys Asp
                   405                 410                 415
   Leu Asn Val Gln Asn Leu Gly Lys Glu Val Lys Thr Thr Gln Lys Tyr
               420                 425                 430
   Thr Val Asn Lys Ser Asn Asn Gly Leu Ser Met Val Pro Trp Gly Thr
           435                 440                 445
   Lys Asn Gln Val Ile Leu Thr Gly Asn Asn Ile Ala Gln Gly Thr Phe
       450                 455                 460
   Asn Ala Thr Lys Gln Val Ser Val Gly Lys Asp Val Tyr Leu Tyr Gly
   465                 470                 475                 480
   Thr Ile Asn Asn Arg Thr Gly Trp Val Asn Ala Lys Asp Leu Thr Ala
                   485                 490                 495
   Pro Thr Ala Val Lys Pro Thr Thr Ser Ala Ala Lys
               500                     505
<210> 7
   <211> 579
   <212> DNA
   <213> Staphylococcus caprae
<220>
   <221> CDS
   <222> (1) .. (579)
   <223>
<400> 7
   tca aac aac aat aaa tta act gtt tct gct aat agt ggt gtt gct caa       48
   Ser Asn Asn Asn Lys Leu Thr Val Ser Ala Asn Ser Gly Val Ala Gln
   1               5                   10                  15
   att aaa cct agc aat agt ggc ctt tac aca act gtt tat gac gaa aaa       96
   Ile Lys Pro Ser Asn Ser Gly Leu Tyr Thr Thr Val Tyr Asp Glu Lys
               20                  25                  30
   gga cat tca aca gat caa gct caa aaa aca tta tct gtt act aaa tct      144
   Gly His Ser Thr Asp Gln Ala Gln Lys Thr Leu Ser Val Thr Lys Ser
           35                  40                  45
   gca aca ctt ggc aat aat aaa ttc tat tta gtc gaa gat tac aac act      192
   Ala Thr Leu Gly Asn Asn Lys Phe Tyr Leu Val Glu Asp Tyr Asn Thr
       50                  55                  60
   ggt aaa aag tat ggc tgg gta aaa caa ggc gat gtt gtt tat aat aca      240
   Gly Lys Lys Tyr Gly Trp Val Lys Gln Gly Asp Val Val Tyr Asn Thr
   65                  70                  75                  80
   gct aaa tca cct gtt aaa gtt aac caa aca tat aat gta aaa gct ggt      288
   Ala Lys Ser Pro Val Lys Val Asn Gln Thr Tyr Asn Val Lys Ala Gly
                   85                  90                  95
   tca act tta tac aca gta cct tgg ggt acg cct agt caa gta gct tca      336
   Ser Thr Leu Tyr Thr Val Pro Trp Gly Thr Pro Ser Gln Val Ala Ser
               100                 105                 110
   aag gtt tca ggt tct gga aat caa aca ttt aaa gct aca aaa caa caa      384
   Lys Val Ser Gly Ser Gly Asn Gln Thr Phe Lys Ala Thr Lys Gln Gln
           115                 120                 125
   caa att gat aaa gcc att tat tta tac gga aca gtg aac ggt aaa tct      432
   Gln Ile Asp Lys Ala Ile Tyr Leu Tyr Gly Thr Val Asn Gly Lys Ser
       130                 135                 140
   ggt tgg att agt aaa tat tac tta aca aca cct tca tcg tct aat acg        480
   Gly Trp Ile Ser Lys Tyr Tyr Leu Thr Thr Pro Ser Ser Ser Asn Thr
   145                 150                 155                 160
   aaa cca agt aaa cca tcg aca gat aat agt tca agt aat aat aaa tta        528
   Lys Pro Ser Lys Pro Ser Thr Asp Asn Ser Ser Ser Asn Asn Lys Leu
                   165                 170                 175
   act gtt tct gct aac agt ggt gtt gct caa att aaa gct aaa aat aat        576
   Thr Val Ser Ala Asn Ser Gly Val Ala Gln Ile Lys Ala Lys Asn Asn
               180                 185                 190
   ggt                                                                    579
   Gly
<210> 8
   <211> 193
   <212> PRT
   <213> Staphylococcus caprae
<400> 8
   Ser Asn Asn Asn Lys Leu Thr Val Ser Ala Asn Ser Gly Val Ala Gln
   1               5                   10                  15
   Ile Lys Pro Ser Asn Ser Gly Leu Tyr Thr Thr Val Tyr Asp Glu Lys
               20                  25                  30
   Gly His Ser Thr Asp Gln Ala Gln Lys Thr Leu Ser Val Thr Lys Ser
           35                  40                  45
   Ala Thr Leu Gly Asn Asn Lys Phe Tyr Leu Val Glu Asp Tyr Asn Thr
       50                  55                  60
   Gly Lys Lys Tyr Gly Trp Val Lys Gln Gly Asp Val Val Tyr Asn Thr
   65                  70                  75                  80
   Ala Lys Ser Pro Val Lys Val Asn Gln Thr Tyr Asn Val Lys Ala Gly
                   85                  90                  95
   Ser Thr Leu Tyr Thr Val Pro Trp Gly Thr Pro Ser Gln Val Ala Ser
               100                 105                 110
   Lys Val Ser Gly Ser Gly Asn Gln Thr Phe Lys Ala Thr Lys Gln Gln
           115                 120                 125
   Gln Ile Asp Lys Ala Ile Tyr Leu Tyr Gly Thr Val Asn Gly Lys Ser
       130                 135                 140
   Gly Trp Ile Ser Lys Tyr Tyr Leu Thr Thr Pro Ser Ser Ser Asn Thr
   145                 150                 155                 160
   Lys Pro Ser Lys Pro Ser Thr Asp Asn Ser Ser Ser Asn Asn Lys Leu
                   165                 170                 175
   Thr Val Ser Ala Asn Ser Gly Val Ala Gln Ile Lys Ala Lys Asn Asn
               180                 185                 190
   Gly
<210> 9
   <211> 696
   <212> DNA
   <213> Staphylococcus epidermidis
<220>
   <221> CDS
   <222> (1)..(696)
   <223>
<400> 9
   ggt aag cca gga gtt aaa aat cct gat aca ggc gaa gta gtc aca cca       48
   Gly Lys Pro Gly Val Lys Asn Pro Asp Thr Gly Glu Val Val Thr Pro
   1               5                   10                  15
   cca gtg gat gat gtg aca aaa tat ggt cca gtt gat gga gat ccg att       96
   Pro Val Asp Asp Val Thr Lys Tyr Gly Pro Val Asp Gly Asp Pro Ile
               20                  25                  30
   acg tca acg gaa gaa att ccg ttt gat aaa aaa cgc gaa ttt gat cca      144
   Thr Ser Thr Glu Glu Ile Pro Phe Asp Lys Lys Arg Glu Phe Asp Pro
           35                  40                  45
   aac tta gcg cca ggt aca gag aaa gtc gtt caa aaa ggt gaa cca gga      192
   Asn Leu Ala Pro Gly Thr Glu Lys Val Val Gln Lys Gly Glu Pro Gly
       50                  55                  60
   aca aaa aca att aca acg cca aca act aag aac cca tta aca gga gaa      240
   Thr Lys Thr Ile Thr Thr Pro Thr Thr Lys Asn Pro Leu Thr Gly Glu
   65                  70                  75                  80
   aaa gtt ggc gaa ggt gaa cca aca gaa aaa ata aca aaa caa cca gtg      288
   Lys Val Gly Glu Gly Glu Pro Thr Glu Lys Ile Thr Lys Gln Pro Val
                   85                  90                  95
   gat gag att gtt cat tat ggt ggt gaa caa ata cca caa ggt cat aaa      336
   Asp Glu Ile Val His Tyr Gly Gly Glu Gln Ile Pro Gln Gly His Lys
               100                 105                 110
   gat gaa ttt gat cca aat gca cct gta gat agt aaa act gaa gtt cca      384
   Asp Glu Phe Asp Pro Asn Ala Pro Val Asp Ser Lys Thr Glu Val Pro
           115                 120                 125
   ggt aaa cca gga gtt aaa aat cct gat aca ggt gaa gtt gtt acc cca      432
   Gly Lys Pro Gly Val Lys Asn Pro Asp Thr Gly Glu Val Val Thr Pro
       130                 135                 140
   cca gtg gat gat gtg aca aaa tat ggt ccg aaa gtt ggt aat cca atc      480
   Pro Val Asp Asp Val Thr Lys Tyr Gly Pro Lys Val Gly Asn Pro Ile
   145                 150                 155                 160
   aca tca acg gaa gag att cca ttt gat aag aaa cgt gta ttt aat cct      528
   Thr Ser Thr Glu Glu Ile Pro Phe Asp Lys Lys Arg Val Phe Asn Pro
                   165                 170                 175
   gat tta aaa cca ggt gaa gag cgc gtt aaa caa aaa ggt gaa cca gga      576
   Asp Leu Lys Pro Gly Glu Glu Arg Val Lys Gln Lys Gly Glu Pro Gly
               180                 185                 190
   aca aaa aca att aca aca cca ata tta gtt aat cct att aca gga gaa      624
   Thr Lys Thr Ile Thr Thr Pro Ile Leu Val Asn Pro Ile Thr Gly Glu
           195                 200                 205
   aaa gtt ggc gaa ggt aaa tca aca gaa aaa gtc act aaa caa cct gtt      672
   Lys Val Gly Glu Gly Lys Ser Thr Glu Lys Val Thr Lys Gln Pro Val
       210                 215                 220
   gac gaa att gtt gag tat ggt cca                                      696
   Asp Glu Ile Val Glu Tyr Gly Pro
   225                 230
<210> 10
   <211> 232
   <212> PRT
   <213> Staphylococcus epidermidis
<400> 10
   Gly Lys Pro Gly Val Lys Asn Pro Asp Thr Gly Glu Val Val Thr Pro
   1               5                   10                  15
   Pro Val Asp Asp Val Thr Lys Tyr Gly Pro Val Asp Gly Asp Pro Ile
               20                  25                  30
   Thr Ser Thr Glu Glu Ile Pro Phe Asp Lys Lys Arg Glu Phe Asp Pro
           35                  40                  45
   Asn Leu Ala Pro Gly Thr Glu Lys Val Val Gln Lys Gly Glu Pro Gly
       50                  55                  60
   Thr Lys Thr Ile Thr Thr Pro Thr Thr Lys Asn Pro Leu Thr Gly Glu
   65                  70                  75                  80
   Lys Val Gly Glu Gly Glu Pro Thr Glu Lys Ile Thr Lys Gln Pro Val
                   85                  90                  95
   Asp Glu Ile Val His Tyr Gly Gly Glu Gln Ile Pro Gln Gly His Lys
               100                 105                 110
   Asp Glu Phe Asp Pro Asn Ala Pro Val Asp Ser Lys Thr Glu Val Pro
           115                 120                 125
   Gly Lys Pro Gly Val Lys Asn Pro Asp Thr Gly Glu Val Val Thr Pro
       130                 135                 140
   Pro Val Asp Asp Val Thr Lys Tyr Gly Pro Lys Val Gly Asn Pro Ile
   145                 150                 155                 160
   Thr Ser Thr Glu Glu Ile Pro Phe Asp Lys Lys Arg Val Phe Asn Pro
                   165                 170                 175
   Asp Leu Lys Pro Gly Glu Glu Arg Val Lys Gln Lys Gly Glu Pro Gly
               180                 185                 190
   Thr Lys Thr Ile Thr Thr Pro Ile Leu Val Asn Pro Ile Thr Gly Glu
           195                 200                 205
   Lys Val Gly Glu Gly Lys Ser Thr Glu Lys Val Thr Lys Gln Pro Val
       210                 215                 220
   Asp Glu Ile Val Glu Tyr Gly Pro
   225                 230
<210> 11
   <211> 843
   <212> DNA
   <213> Staphylococcus epidermidis
<220>
   <221> CDS
   <222> (1)..(843)
   <223>
<400> 11
   aaa aag aga ttt tta tct ata tgt aca atg aca att gca gcg tta gca      48
   Lys Lys Arg Phe Leu Ser Ile Cys Thr Met Thr Ile Ala Ala Leu Ala
   1               5                   10                  15
   act act aca atg gta aat act tct tat gca aaa acc gat aca gaa agc      96
   Thr Thr Thr Met Val Asn Thr Ser Tyr Ala Lys Thr Asp Thr Glu Ser
               20                  25                  30
   cat aat cat tcc tca ctt ggc aca gaa aac aaa aat gtt tta gat att      144
   His Asn His Ser Ser Leu Gly Thr Glu Asn Lys Asn Val Leu Asp Ile
           35                  40                  45
   aat agt tcg agt cat aat atc aaa cca agt caa aat aaa agt tac cca      192
   Asn Ser Ser Ser His Asn Ile Lys Pro Ser Gln Asn Lys Ser Tyr Pro
       50                  55                  60
   agt gta ata tta cct aat aat aat aga cat caa att ttt aat act aca      240
   Ser Val Ile Leu Pro Asn Asn Asn Arg His Gln Ile Phe Asn Thr Thr
   65                  70                  75                  80
   caa ggt cat tat gat gct gtt agt ttt att tat ata cca ata gat ggt      288
   Gln Gly His Tyr Asp Ala Val Ser Phe Ile Tyr Ile Pro Ile Asp Gly
                   85                  90                  95
   gga tat atg agt ggt tca ggt gtt gtt gta ggt gaa aat gaa ata tta      336
   Gly Tyr Met Ser Gly Ser Gly Val Val Val Gly Glu Asn Glu Ile Leu
               100                 105                 110
   act aat aaa cac gtt gtt aat gga gct aag ggt aat cca aga aat att      384
   Thr Asn Lys His Val Val Asn Gly Ala Lys Gly Asn Pro Arg Asn Ile
           115                 120                 125
   agt gtc cat cct tca gct aaa aat gaa aat gat tat cct aat ggc aaa      432
   Ser Val His Pro Ser Ala Lys Asn Glu Asn Asp Tyr Pro Asn Gly Lys
       130                 135                 140
   ttt gtg ggt caa gaa atc ata ccg tat cct ggt aat agt gat tta gca      480
   Phe Val Gly Gln Glu Ile Ile Pro Tyr Pro Gly Asn Ser Asp Leu Ala
   145                 150                 155                 160
   atc tta aga gtg tca cca aac gaa cat aat caa cat att ggt caa gta      528
   Ile Leu Arg Val Ser Pro Asn Glu His Asn Gln His Ile Gly Gln Val
                   165                 170                 175
   gtt aaa cct gca act ata agt agc aat aca gac act aga att aat gaa      576
   Val Lys Pro Ala Thr Ile Ser Ser Asn Thr Asp Thr Arg Ile Asn Glu
               180                 185                 190
   aac atc act gtt act ggt tac cct ggt gac aaa cca tta gcc aca atg       624
   Asn Ile Thr Val Thr Gly Tyr Pro Gly Asp Lys Pro Leu Ala Thr Met
       195                     200                     205
   tgg gaa agt gta ggt aaa gtt gtc tat att ggt ggc gag gaa tta aga       672
   Trp Glu Ser Val Gly Lys Val Val Tyr Ile Gly Gly Glu Glu Leu Arg
       210                     215                     220
   tat gac cta agt act gta ggt gga aac tct gga tct cca gta ttt aat       720
   Tyr Asp Leu Ser Thr Val Gly Gly Asn Ser Gly Ser Pro Val Phe Asn
   225                     230                     235                 240
   ggt aaa aat caa gtt att gga ata cat tat ggt ggc gta gat aat aaa       768
   Gly Lys Asn Gln Val Ile Gly Ile His Tyr Gly Gly Val Asp Asn Lys
                   245                     250                     255
   tac aat agc agt gtt tat att aat gat ttc gtt caa caa ttc cta aga       816
   Tyr Asn Ser Ser Val Tyr Ile Asn Asp Phe Val Gln Gln Phe Leu Arg
               260                     265                     270
   aac aat ata cct gat ata aat att cag                                   843
   Asn Asn Ile Pro Asp Ile Asn Ile Gln
           275                     280
<210> 12
   <211> 281
   <212> PRT
   <213> Staphylococcus epidermidis
<400> 12
   Lys Lys Arg Phe Leu Ser Ile Cys Thr Met Thr Ile Ala Ala Leu Ala
   1               5                   10                  15
   Thr Thr Thr Met Val Asn Thr Ser Tyr Ala Lys Thr Asp Thr Glu Ser
               20                  25                  30
   His Asn His Ser Ser Leu Gly Thr Glu Asn Lys Asn Val Leu Asp Ile
           35                  40                  45
   Asn Ser Ser Ser His Asn Ile Lys Pro Ser Gln Asn Lys Ser Tyr Pro
       50                  55                  60
   Ser Val Ile Leu Pro Asn Asn Asn Arg His Gln Ile Phe Asn Thr Thr
   65                  70                  75                  80
   Gln Gly His Tyr Asp Ala Val Ser Phe Ile Tyr Ile Pro Ile Asp Gly
                   85                  90                  95
   Gly Tyr Met Ser Gly Ser Gly Val Val Val Gly Glu Asn Glu Ile Leu
               100                 105                 110
   Thr Asn Lys His Val Val Asn Gly Ala Lys Gly Asn Pro Arg Asn Ile
           115                 120                 125
   Ser Val His Pro Ser Ala Lys Asn Glu Asn Asp Tyr Pro Asn Gly Lys
       130                 135                 140
   Phe Val Gly Gln Glu Ile Ile Pro Tyr Pro Gly Asn Ser Asp Leu Ala
   145                 150                 155                 160
   Ile Leu Arg Val Ser Pro Asn Glu His Asn Gln His Ile Gly Gln Val
                   165                 170                 175
   Val Lys Pro Ala Thr Ile Ser Ser Asn Thr Asp Thr Arg Ile Asn Glu
               180                 185                 190
   Asn Ile Thr Val Thr Gly Tyr Pro Gly Asp Lys Pro Leu Ala Thr Met
           195                 200                 205
   Trp Glu Ser Val Gly Lys Val Val Tyr Ile Gly Gly Glu Glu Leu Arg
       210                 215                 220
   Tyr Asp Leu Ser Thr Val Gly Gly Asn Ser Gly Ser Pro Val Phe Asn
   225                 230                 235                 240
   Gly Lys Asn Gln Val Ile Gly Ile His Tyr Gly Gly Val Asp Asn Lys
                   245                 250                 255
   Tyr Asn Ser Ser Val Tyr Ile Asn Asp Phe Val Gln Gln Phe Leu Arg
               260                 265                 270
   Asn Asn Ile Pro Asp Ile Asn Ile Gln
           275                 280
<210> 13
   <211> 1068
   <212> DNA
   <213> Staphylococcus aureus
<220>
   <221> CDS
   <222> (1)..(1068)
   <223>
<400> 13
   aaa ggt aaa ttt tta aaa gtt agt tct tta ttc gtt gca act ttg aca      48
   Lys Gly Lys Phe Leu Lys Val Ser Ser Leu Phe Val Ala Thr Leu Thr
   1               5                   10                  15
   aca gcg aca ctt gtg agt tct cca gca gca aat gcg tta tct tca aaa      96
   Thr Ala Thr Leu Val Ser Ser Pro Ala Ala Asn Ala Leu Ser Ser Lys
               20                  25                  30
   gct atg gac aat cat cca caa caa acg cag aca gac aaa cag caa aca     144
   Ala Met Asp Asn His Pro Gln Gln Thr Gln Thr Asp Lys Gln Gln Thr
           35                  40                  45
   cct aag att caa aaa ggc ggt aac ctt aaa cca tta gaa caa cgt gaa     192
   Pro Lys Ile Gln Lys Gly Gly Asn Leu Lys Pro Leu Glu Gln Arg Glu
       50                  55                  60
   cgc gct aat gtt ata tta cca aat aac gat cgt cac caa atc aca gat     240
   Arg Ala Asn Val Ile Leu Pro Asn Asn Asp Arg His Gln Ile Thr Asp
   65                  70                  75                  80
   aca acg aat ggt cat tat gca cct gtt act tat att caa gtt gaa gca     288
   Thr Thr Asn Gly His Tyr Ala Pro Val Thr Tyr Ile Gln Val Glu Ala
                   85                  90                  95
   cct act ggt aca ttt att gct tct ggt gta gtt gta ggt aaa gat aca     336
   Pro Thr Gly Thr Phe Ile Ala Ser Gly Val Val Val Gly Lys Asp Thr
               100                 105                 110
   ctt tta aca aat aaa cac gtc gta gat gct acg cac ggt gat cct cat     384
   Leu Leu Thr Asn Lys His Val Val Asp Ala Thr His Gly Asp Pro His
           115                 120                 125
   gct tta aaa gca ttc cct tct gca att aac caa gac aat tat cct aat     432
   Ala Leu Lys Ala Phe Pro Ser Ala Ile Asn Gln Asp Asn Tyr Pro Asn
       130                 135                 140
   ggt ggt ttc act gct gaa caa atc act aaa tat tca ggc gaa ggt gat     480
   Gly Gly Phe Thr Ala Glu Gln Ile Thr Lys Tyr Ser Gly Glu Gly Asp
   145                 150                 155                 160
   tta gca atc gtt aaa ttc tcc cct aat gag caa aac aaa cat att ggc     528
   Leu Ala Ile Val Lys Phe Ser Pro Asn Glu Gln Asn Lys His Ile Gly
                   165                 170                 175
   gaa gta gtt aaa cca gca aca atg agt aat aat gct gaa aca caa gtt     576
   Glu Val Val Lys Pro Ala Thr Met Ser Asn Asn Ala Glu Thr Gln Val
               180                 185                 190
   aac caa aat att act gta aca gga tat cct ggt gat aaa cct gtc gca     624
   Asn Gln Asn Ile Thr Val Thr Gly Tyr Pro Gly Asp Lys Pro Val Ala
           195                 200                 205
   aca atg tgg gaa agt aaa gga aaa ata acg tac tta aaa ggt gaa gca      672
   Thr Met Trp Glu Ser Lys Gly Lys Ile Thr Tyr Leu Lys Gly Glu Ala
       210                 215                 220
   atg caa tat gat tta agt aca act ggt ggt aac tca ggt tca cct gta      720
   Met Gln Tyr Asp Leu Ser Thr Thr Gly Gly Asn Ser Gly Ser Pro Val
   225                 230                 235                 240
   ttt aat gaa aaa aat gaa gtc att ggc att cat tgg ggt ggc gtt cca      768
   Phe Asn Glu Lys Asn Glu Val Ile Gly Ile His Trp Gly Gly Val Pro
                   245                 250                 255
   aat caa ttt aac ggt gca gta ttt att aat gaa aat gta cgc aac ttc      816
   Asn Gln Phe Asn Gly Ala Val Phe Ile Asn Glu Asn Val Arg Asn Phe
               260                 265                 270
   tta aaa caa aat att gaa gat atc aat ttc gca aat gat gac cac cct      864
   Leu Lys Gln Asn Ile Glu Asp Ile Asn Phe Ala Asn Asp Asp His Pro
           275                 280                 285
   aac aac cct gat aat cca gac aat cca aat aat ccg gac aat cct aac      912
   Asn Asn Pro Asp Asn Pro Asp Asn Pro Asn Asn Pro Asp Asn Pro Asn
       290                 295                 300
   aac cct gat aac cct aac aac cct gat aat cca gac aat cct aat aat      960
   Asn Pro Asp Asn Pro Asn Asn Pro Asp Asn Pro Asp Asn Pro Asn Asn
   305                 310                 315                 320
   cct gat aac cct aac aac ccg gac aat cca aat aac cct gac caa cct     1008
   Pro Asp Asn Pro Asn Asn Pro Asp Asn Pro Asn Asn Pro Asp Gln Pro
                   325                 330                 335
   aac aac cca aat aac ccg gac aat ggc gat aac aat aat tca gac aac     1056
   Asn Asn Pro Asn Asn Pro Asp Asn Gly Asp Asn Asn Asn Ser Asp Asn
               340                 345                 350
   cct gac gct gca                                                     1068
   Pro Asp Ala Ala
           355
<210> 14
   <211> 356
   <212> PRT
   <213> Staphylococcus aureus
<400> 14
   Lys Gly Lys Phe Leu Lys Val Ser Ser Leu Phe Val Ala Thr Leu Thr
   1               5                   10                  15
   Thr Ala Thr Leu Val Ser Ser Pro Ala Ala Asn Ala Leu Ser Ser Lys
               20                  25                  30
   Ala Met Asp Asn His Pro Gln Gln Thr Gln Thr Asp Lys Gln Gln Thr
   35 40 45
   Pro Lys Ile Gln Lys Gly Gly Asn Leu Lys Pro Leu Glu Gln Arg Glu
   50 55 60
   Arg Ala Asn Val Ile Leu Pro Asn Asn Asp Arg His Gln Ile Thr Asp
   65 70 75 80
   Thr Thr Asn Gly His Tyr Ala Pro Val Thr Tyr Ile Gln Val Glu Ala
   85 90 95
   Pro Thr Gly Thr Phe Ile Ala Ser Gly Val Val Val Gly Lys Asp Thr
   100 105 110
   Leu Leu Thr Asn Lys His Val Val Asp Ala Thr His Gly Asp Pro His
   115 120 125
   Ala Leu Lys Ala Phe Pro Ser Ala Ile Asn Gln Asp Asn Tyr Pro Asn
   130 135 140
   Gly Gly Phe Thr Ala Glu Gln Ile Thr Lys Tyr Ser Gly Glu Gly Asp
   145 150 155 160
   Leu Ala Ile Val Lys Phe Ser Pro Asn Glu Gln Asn Lys His Ile Gly
   165 170 175
   Glu Val Val Lys Pro Ala Thr Met Ser Asn Asn Ala Glu Thr Gln Val
   180 185 190
   Asn Gln Asn Ile Thr Val Thr Gly Tyr Pro Gly Asp Lys Pro Val Ala
   195 200 205
   Thr Met Trp Glu Ser Lys Gly Lys Ile Thr Tyr Leu Lys Gly Glu Ala
   210 215 220
   Met Gln Tyr Asp Leu Ser Thr Thr Gly Gly Asn Ser Gly Ser Pro Val
   225 230 235 240
   Phe Asn Glu Lys Asn Glu Val Ile Gly Ile His Trp Gly Gly Val Pro
   245 250 255
   Asn Gln Phe Asn Gly Ala Val Phe Ile Asn Glu Asn Val Arg Asn Phe
   260 265 270
   Leu Lys Gln Asn Ile Glu Asp Ile Asn Phe Ala Asn Asp Asp His Pro
           275                 280                 285
   Asn Asn Pro Asp Asn Pro Asp Asn Pro Asn Asn Pro Asp Asn Pro Asn
       290                 295                 300
   Asn Pro Asp Asn Pro Asn Asn Pro Asp Asn Pro Asp Asn Pro Asn Asn
   305                 310                 315                 320
   Pro Asp Asn Pro Asn Asn Pro Asp Asn Pro Asn Asn Pro Asp Gln Pro
                   325                 330                 335
   Asn Asn Pro Asn Asn Pro Asp Asn Gly Asp Asn Asn Asn Ser Asp Asn
               340                 345                 350
   Pro Asp Ala Ala
           355
<210> 15
   <211> 1515
   <212> DNA
   <213> Staphylococcus epidermidis
<220>
   <221> CDS
   <222> (1)..(1515)
   <223>
<400> 15
   atg gca aaa caa cca act gcc tta atc atc tta gat ggt ttc gca aat        48
   Met Ala Lys Gln Pro Thr Ala Leu Ile Ile Leu Asp Gly Phe Ala Asn
   1               5                   10                  15
   cgt gaa agt gaa cat ggc aat gca gtt aag caa gca cat aaa cct aat        96
   Arg Glu Ser Glu His Gly Asn Ala Val Lys Gln Ala His Lys Pro Asn
               20                  25                  30
   ttt gat cga tat tat gaa aaa tat cct aca aca caa ata gaa gct agt       144
   Phe Asp Arg Tyr Tyr Glu Lys Tyr Pro Thr Thr Gln Ile Glu Ala Ser
           35                  40                  45
   ggc tta gat gta ggt ctt cct gaa ggt caa atg ggt aac tct gaa gta       192
   Gly Leu Asp Val Gly Leu Pro Glu Gly Gln Met Gly Asn Ser Glu Val
       50                  55                  60
   gga cat atg aat att ggt gca gga cgc atc gta tat caa agt tta act      240
   Gly His Met Asn Ile Gly Ala Gly Arg Ile Val Tyr Gln Ser Leu Thr
   65                  70                  75                  80
   cgt att aat aaa tcg att gaa gac gga gaa ttc ttt gat aac act gta      288
   Arg Ile Asn Lys Ser Ile Glu Asp Gly Glu Phe Phe Asp Asn Thr Val
                   85                  90                  95
   tta aat aac gct gtt aaa cat gtt aaa gac aat ggc tct gcg ctt cat      336
   Leu Asn Asn Ala Val Lys His Val Lys Asp Asn Gly Ser Ala Leu His
               100                 105                 110
   gta ttc gga ttg ctt tct gat ggt ggt gta cac agt cat tat aag cat      384
   Val Phe Gly Leu Leu Ser Asp Gly Gly Val His Ser His Tyr Lys His
           115                 120                 125
   cta ttt gct att tta gaa tta gct aaa aag caa gga ata gat aaa gta      432
   Leu Phe Ala Ile Leu Glu Leu Ala Lys Lys Gln Gly Ile Asp Lys Val
       130                 135                 140
   tat gtc cac gca ttt tta gat ggt cgt gat gtt gat caa aaa tct gct      480
   Tyr Val His Ala Phe Leu Asp Gly Arg Asp Val Asp Gln Lys Ser Ala
   145                 150                 155                 160
   ttg aaa tat ata gag gaa act gaa gat aaa ttt aaa gaa tta ggt gta      528
   Leu Lys Tyr Ile Glu Glu Thr Glu Asp Lys Phe Lys Glu Leu Gly Val
                   165                 170                 175
   ggc caa ttc gct tct gtt tca gga cgt tat tat gct atg gac cgt gac      576
   Gly Gln Phe Ala Ser Val Ser Gly Arg Tyr Tyr Ala Met Asp Arg Asp
               180                 185                 190
   aag cgt tgg gat cgt gag gaa cgt gcc tat aat gct att cgt aac ttt      624
   Lys Arg Trp Asp Arg Glu Glu Arg Ala Tyr Asn Ala Ile Arg Asn Phe
           195                 200                 205
   gaa ggt cct aca ttt act tca gct aaa gca ggc gtt gaa gct aat tat      672
   Glu Gly Pro Thr Phe Thr Ser Ala Lys Ala Gly Val Glu Ala Asn Tyr
       210                 215                 220
   aaa aat gat gtg act gat gaa ttc gtc gaa ccg ttt ata gtt gaa ggc      720
   Lys Asn Asp Val Thr Asp Glu Phe Val Glu Pro Phe Ile Val Glu Gly
   225                 230                 235                 240
   caa aac gat ggt gtg aac gac gga gac gca gta atc ttt tat aat ttc      768
   Gln Asn Asp Gly Val Asn Asp Gly Asp Ala Val Ile Phe Tyr Asn Phe
                   245                 250                 255
   cgt cca gat aga gca gct caa ctt tca gaa atc ttt act aat aaa gcg      816
   Arg Pro Asp Arg Ala Ala Gln Leu Ser Glu Ile Phe Thr Asn Lys Ala
               260                 265                 270
   ttt gat gga ttt aaa gtt gaa caa gtg gac aac tta ttc tac gct aca      864
   Phe Asp Gly Phe Lys Val Glu Gln Val Asp Asn Leu Phe Tyr Ala Thr
           275                 280                 285
   ttc acg aaa tat aat gac aat gta gat gct gaa att gta ttt gaa aaa      912
   Phe Thr Lys Tyr Asn Asp Asn Val Asp Ala Glu Ile Val Phe Glu Lys
       290                 295                 300
   gtt gac tta aat aat aca atc ggt gaa gtt gct caa gat aat ggc ttg      960
   Val Asp Leu Asn Asn Thr Ile Gly Glu Val Ala Gln Asp Asn Gly Leu
   305                 310                 315                 320
   aaa caa tta cgt att gct gaa act gaa aag tat cca cat gta aca tac     1008
   Lys Gln Leu Arg Ile Ala Glu Thr Glu Lys Tyr Pro His Val Thr Tyr
                   325                 330                 335
   ttt atg agt ggt gga cga aat gaa gag ttc gaa gga gaa cgt cgt aga     1056
   Phe Met Ser Gly Gly Arg Asn Glu Glu Phe Glu Gly Glu Arg Arg Arg
               340                 345                 350
   ctc atc gat tct cca aaa gta gcg act tat gat tta aaa cct gag atg     1104
   Leu Ile Asp Ser Pro Lys Val Ala Thr Tyr Asp Leu Lys Pro Glu Met
           355                 360                 365
   agt gca tat gaa gtt aaa gat gca tta tta gaa gag tta gac aaa ggt     1152
   Ser Ala Tyr Glu Val Lys Asp Ala Leu Leu Glu Glu Leu Asp Lys Gly
       370                 375                 380
   gac tta gat tta att cta ctg aac ttt gct aac cca gat atg gtt gga     1200
   Asp Leu Asp Leu Ile Leu Leu Asn Phe Ala Asn Pro Asp Met Val Gly
   385                 390                 395                 400
   cat agt ggt atg ctt gaa cca aca att aaa gca atc gaa gca gta gat     1248
   His Ser Gly Met Leu Glu Pro Thr Ile Lys Ala Ile Glu Ala Val Asp
                   405                 410                 415
   gag tgt ctt ggt gaa gtc gtt gac aaa att att gat atg ggt ggt cat     1296
   Glu Cys Leu Gly Glu Val Val Asp Lys Ile Ile Asp Met Gly Gly His
               420                 425                 430
   gcc atc atc act gca gac cac ggt aac tca gat caa gta tta act gat     1344
   Ala Ile Ile Thr Ala Asp His Gly Asn Ser Asp Gln Val Leu Thr Asp
           435                 440                 445
   gac gac caa cct atg acg aca cac aca act aat cct gtt cca gtt att     1392
   Asp Asp Gln Pro Met Thr Thr His Thr Thr Asn Pro Val Pro Val Ile
       450                 455                 460
   gta act aaa gaa ggt gtt aca tta aga gaa act gga cgt tta ggc gat     1440
   Val Thr Lys Glu Gly Val Thr Leu Arg Glu Thr Gly Arg Leu Gly Asp
   465                 470                 475                 480
   tta gcg ccg aca tta tta gat tta tta aat gtt aaa caa cca tct gaa     1488
   Leu Ala Pro Thr Leu Leu Asp Leu Leu Asn Val Lys Gln Pro Ser Glu
                   485                 490                 495
   atg aca ggt gaa tca ctg att aaa cat                                 1515
   Met Thr Gly Glu Ser Leu Ile Lys His
               500                 505
<210> 16
   <211> 505
   <212> PRT
   <213> Staphylococcus epidermidis
<400> 16
   Met Ala Lys Gln Pro Thr Ala Leu Ile Ile Leu Asp Gly Phe Ala Asn
   1               5                   10                  15
   Arg Glu Ser Glu His Gly Asn Ala Val Lys Gln Ala His Lys Pro Asn
               20                  25                  30
   Phe Asp Arg Tyr Tyr Glu Lys Tyr Pro Thr Thr Gln Ile Glu Ala Ser
           35                  40                  45
   Gly Leu Asp Val Gly Leu Pro Glu Gly Gln Met Gly Asn Ser Glu Val
       50                  55                  60
   Gly His Met Asn Ile Gly Ala Gly Arg Ile Val Tyr Gln Ser Leu Thr
   65                  70                  75                  80
   Arg Ile Asn Lys Ser Ile Glu Asp Gly Glu Phe Phe Asp Asn Thr Val
                   85                  90                  95
   Leu Asn Asn Ala Val Lys His Val Lys Asp Asn Gly Ser Ala Leu His
               100                 105                 110
   Val Phe Gly Leu Leu Ser Asp Gly Gly Val His Ser His Tyr Lys His
           115                 120                 125
   Leu Phe Ala Ile Leu Glu Leu Ala Lys Lys Gln Gly Ile Asp Lys Val
       130                 135                 140
   Tyr Val His Ala Phe Leu Asp Gly Arg Asp Val Asp Gln Lys Ser Ala
   145                 150                 155                 160
   Leu Lys Tyr Ile Glu Glu Thr Glu Asp Lys Phe Lys Glu Leu Gly Val
                   165                 170                 175
   Gly Gln Phe Ala Ser Val Ser Gly Arg Tyr Tyr Ala Met Asp Arg Asp
               180                 185                 190
   Lys Arg Trp Asp Arg Glu Glu Arg Ala Tyr Asn Ala Ile Arg Asn Phe
           195                 200                 205
   Glu Gly Pro Thr Phe Thr Ser Ala Lys Ala Gly Val Glu Ala Asn Tyr
       210                 215                 220
   Lys Asn Asp Val Thr Asp Glu Phe Val Glu Pro Phe Ile Val Glu Gly
   225                 230                 235                 240
   Gln Asn Asp Gly Val Asn Asp Gly Asp Ala Val Ile Phe Tyr Asn Phe
                   245                 250                 255
   Arg Pro Asp Arg Ala Ala Gln Leu Ser Glu Ile Phe Thr Asn Lys Ala
               260                 265                 270
   Phe Asp Gly Phe Lys Val Glu Gln Val Asp Asn Leu Phe Tyr Ala Thr
           275                 280                 285
   Phe Thr Lys Tyr Asn Asp Asn Val Asp Ala Glu Ile Val Phe Glu Lys
       290                 295                 300
   Val Asp Leu Asn Asn Thr Ile Gly Glu Val Ala Gln Asp Asn Gly Leu
   305                 310                 315                 320
   Lys Gln Leu Arg Ile Ala Glu Thr Glu Lys Tyr Pro His Val Thr Tyr
                   325                 330                 335
   Phe Met Ser Gly Gly Arg Asn Glu Glu Phe Glu Gly Glu Arg Arg Arg
               340                 345                 350
   Leu Ile Asp Ser Pro Lys Val Ala Thr Tyr Asp Leu Lys Pro Glu Met
           355                 360                 365
   Ser Ala Tyr Glu Val Lys Asp Ala Leu Leu Glu Glu Leu Asp Lys Gly
       370                 375                 380
   Asp Leu Asp Leu Ile Leu Leu Asn Phe Ala Asn Pro Asp Met Val Gly
   385                 390                 395                 400
   His Ser Gly Met Leu Glu Pro Thr Ile Lys Ala Ile Glu Ala Val Asp
                   405                 410                 415
   Glu Cys Leu Gly Glu Val Val Asp Lys Ile Ile Asp Met Gly Gly His
               420                 425                 430
   Ala Ile Ile Thr Ala Asp His Gly Asn Ser Asp Gln Val Leu Thr Asp
           435                 440                 445
   Asp Asp Gln Pro Met Thr Thr His Thr Thr Asn Pro Val Pro Val Ile
       450                 455                 460
   Val Thr Lys Glu Gly Val Thr Leu Arg Glu Thr Gly Arg Leu Gly Asp
   465                 470                 475                 480
   Leu Ala Pro Thr Leu Leu Asp Leu Leu Asn Val Lys Gln Pro Ser Glu
                   485                 490                 495
   Met Thr Gly Glu Ser Leu Ile Lys His
               500                 505
<210> 17
   <211> 1176
   <212> DNA
   <213> Staphylococcus epidermidis
<220>
   <221> CDS
   <222> (1)..(1176)
   <223>
<400> 17
   aaa cca gat gat tca gca gaa ttt gct gct ttg gca ttt aaa gtt atg      48
   Lys Pro Asp Asp Ser Ala Glu Phe Ala Ala Leu Ala Phe Lys Val Met
   1               5                   10                  15
   act gac cct tat gtt ggt aaa tta aca ttc ttc cgt gta tac tct gga      96
   Thr Asp Pro Tyr Val Gly Lys Leu Thr Phe Phe Arg Val Tyr Ser Gly
               20                  25                  30
   aca tta tct tca ggt tca tat gtg aag aac tct tct aaa gat aaa cgt     144
   Thr Leu Ser Ser Gly Ser Tyr Val Lys Asn Ser Ser Lys Asp Lys Arg
           35                  40                  45
   gaa cgt gtt gga cgt tta tta caa atg cac gcg aac tca cgt caa gaa     192
   Glu Arg Val Gly Arg Leu Leu Gln Met His Ala Asn Ser Arg Gln Glu
       50                  55                  60
   att gac act gtt tat tca ggt gaa att gca gct gca gta ggc ctt aaa     240
   Ile Asp Thr Val Tyr Ser Gly Glu Ile Ala Ala Ala Val Gly Leu Lys
   65                  70                  75                  80
   gaa aca ggt act ggt gat act tta tgt gga gag aaa aat gac att atc     288
   Glu Thr Gly Thr Gly Asp Thr Leu Cys Gly Glu Lys Asn Asp Ile Ile
                   85                  90                  95
   ttg gaa tca atg gaa ttc cca gaa cca gtt atc cac tta tca gtt gaa     336
   Leu Glu Ser Met Glu Phe Pro Glu Pro Val Ile His Leu Ser Val Glu
               100                 105                 110
   cca aaa tct aaa gct gac caa gat aaa atg act caa gct tta gtt aaa      384
   Pro Lys Ser Lys Ala Asp Gln Asp Lys Met Thr Gln Ala Leu Val Lys
           115                 120                 125
   tta caa gaa gaa gac cca aca ttc cat gca cac aca gat gaa gaa act      432
   Leu Gln Glu Glu Asp Pro Thr Phe His Ala His Thr Asp Glu Glu Thr
       130                 135                 140
   gga caa gtt atc atc ggt ggt atg ggt gaa tta cac tta gat att tta      480
   Gly Gln Val Ile Ile Gly Gly Met Gly Glu Leu His Leu Asp Ile Leu
   145                 150                 155                 160
   gtt gac cgt atg aag aaa gaa ttc aac gtt gaa tgt aac gta ggt gct      528
   Val Asp Arg Met Lys Lys Glu Phe Asn Val Glu Cys Asn Val Gly Ala
                   165                 170                 175
   cca atg gtt tct tat cgt gaa aca ttt aaa caa cct gca caa gtt caa      576
   Pro Met Val Ser Tyr Arg Glu Thr Phe Lys Gln Pro Ala Gln Val Gln
               180                 185                 190
   ggt aaa ttc tca cgt caa tct ggt ggt cgt ggt caa tat ggt gat gtt      624
   Gly Lys Phe Ser Arg Gln Ser Gly Gly Arg Gly Gln Tyr Gly Asp Val
           195                 200                 205
   cat att gaa ttc act cct aac gaa aca ggt ggc ggt ttc gaa ttc gaa      672
   His Ile Glu Phe Thr Pro Asn Glu Thr Gly Gly Gly Phe Glu Phe Glu
       210                 215                 220
   aac gct att gtt ggt ggt gta gtt cct cgt gaa tac att cca tca gtt      720
   Asn Ala Ile Val Gly Gly Val Val Pro Arg Glu Tyr Ile Pro Ser Val
   225                 230                 235                 240
   gaa caa ggt ctt aaa gat gct atg gaa aat ggt gtc tta gct ggt tat      768
   Glu Gln Gly Leu Lys Asp Ala Met Glu Asn Gly Val Leu Ala Gly Tyr
                   245                 250                 255
   cca tta att gat gtt aaa gct aaa tta ttt gat ggt tct tat cat gat      816
   Pro Leu Ile Asp Val Lys Ala Lys Leu Phe Asp Gly Ser Tyr His Asp
               260                 265                 270
   gtc gat tca tct gaa atg gcc ttc aaa att gct gca tca tta gcg ctt      864
   Val Asp Ser Ser Glu Met Ala Phe Lys Ile Ala Ala Ser Leu Ala Leu
           275                 280                 285
   aaa gaa gct gct aaa aaa tgt gat cca gtt atc tta gaa cca atg atg      912
   Lys Glu Ala Ala Lys Lys Cys Asp Pro Val Ile Leu Glu Pro Met Met
       290                 295                 300
   aaa gtt act atc gaa atg cct gaa gaa tat atg ggt gat atc atg ggt      960
   Lys Val Thr Ile Glu Met Pro Glu Glu Tyr Met Gly Asp Ile Met Gly
   305                 310                 315                 320
   gac gtg act gct cgt cgt gga cgt gta gac ggt atg gaa cca cgt ggt      1008
   Asp Val Thr Ala Arg Arg Gly Arg Val Asp Gly Met Glu Pro Arg Gly
                   325                 330                 335
   aat gct caa gtt gtt aac gca tat gta cca ctt tca gaa atg ttt ggt      1056
   Asn Ala Gln Val Val Asn Ala Tyr Val Pro Leu Ser Glu Met Phe Gly
               340                 345                 350
   tat gca act tca tta cgt tct aac acg caa ggt cgc ggt act tac aca      1104
   Tyr Ala Thr Ser Leu Arg Ser Asn Thr Gln Gly Arg Gly Thr Tyr Thr
           355                 360                 365
   atg tac ttt gac cac tat gca gaa gtt cct aaa tca att gct gaa gaa      1152
   Met Tyr Phe Asp His Tyr Ala Glu Val Pro Lys Ser Ile Ala Glu Glu
       370                 375                 380
   atc atc aag aaa aat aaa ggt gaa                                      1176
   Ile Ile Lys Lys Asn Lys Gly Glu
   385                 390
<210> 18
   <211> 392
   <212> PRT
   <213> Staphylococcus epidermidis
<400> 18
   Lys Pro Asp Asp Ser Ala Glu Phe Ala Ala Leu Ala Phe Lys Val Met
   1               5                   10                  15
   Thr Asp Pro Tyr Val Gly Lys Leu Thr Phe Phe Arg Val Tyr Ser Gly
               20                  25                  30
   Thr Leu Ser Ser Gly Ser Tyr Val Lys Asn Ser Ser Lys Asp Lys Arg
           35                  40                  45
   Glu Arg Val Gly Arg Leu Leu Gln Met His Ala Asn Ser Arg Gln Glu
       50                  55                  60
   Ile Asp Thr Val Tyr Ser Gly Glu Ile Ala Ala Ala Val Gly Leu Lys
   65                  70                  75                  80
   Glu Thr Gly Thr Gly Asp Thr Leu Cys Gly Glu Lys Asn Asp Ile Ile
                   85                  90                  95
   Leu Glu Ser Met Glu Phe Pro Glu Pro Val Ile His Leu Ser Val Glu
               100                 105                 110
   Pro Lys Ser Lys Ala Asp Gln Asp Lys Met Thr Gln Ala Leu Val Lys
           115                 120                 125
   Leu Gln Glu Glu Asp Pro Thr Phe His Ala His Thr Asp Glu Glu Thr
       130                 135                 140
   Gly Gln Val Ile Ile Gly Gly Met Gly Glu Leu His Leu Asp Ile Leu
   145                 150                 155                 160
   Val Asp Arg Met Lys Lys Glu Phe Asn Val Glu Cys Asn Val Gly Ala
                   165                 170                 175
   Pro Met Val Ser Tyr Arg Glu Thr Phe Lys Gln Pro Ala Gln Val Gln
               180                 185                 190
   Gly Lys Phe Ser Arg Gln Ser Gly Gly Arg Gly Gln Tyr Gly Asp Val
           195                 200                 205
   His Ile Glu Phe Thr Pro Asn Glu Thr Gly Gly Gly Phe Glu Phe Glu
       210                 215                 220
   Asn Ala Ile Val Gly Gly Val Val Pro Arg Glu Tyr Ile Pro Ser Val
   225                 230                 235                 240
   Glu Gln Gly Leu Lys Asp Ala Met Glu Asn Gly Val Leu Ala Gly Tyr
                   245                 250                 255
   Pro Leu Ile Asp Val Lys Ala Lys Leu Phe Asp Gly Ser Tyr His Asp
               260                 265                 270
   Val Asp Ser Ser Glu Met Ala Phe Lys Ile Ala Ala Ser Leu Ala Leu
           275                 280                 285
   Lys Glu Ala Ala Lys Lys Cys Asp Pro Val Ile Leu Glu Pro Met Met
       290                 295                 300
   Lys Val Thr Ile Glu Met Pro Glu Glu Tyr Met Gly Asp Ile Met Gly
   305                 310                 315                 320
   Asp Val Thr Ala Arg Arg Gly Arg Val Asp Gly Met Glu Pro Arg Gly
                   325                 330                 335
   Asn Ala Gln Val Val Asn Ala Tyr Val Pro Leu Ser Glu Met Phe Gly
               340                 345                 350
   Tyr Ala Thr Ser Leu Arg Ser Asn Thr Gln Gly Arg Gly Thr Tyr Thr
           355                 360                 365
   Met Tyr Phe Asp His Tyr Ala Glu Val Pro Lys Ser Ile Ala Glu Glu
       370                 375                 380
   Ile Ile Lys Lys Asn Lys Gly Glu
   385                 390
<210> 19
   <211> 1230
   <212> DNA
   <213> Staphylococcus aureus
<220>
   <221> CDS
   <222> (1)..(1230)
   <223>
<400> 19
   gta caa gga ccc att caa gta aat agt gaa ata ggc aaa ttg aaa act       48
   Val Gln Gly Pro Ile Gln Val Asn Ser Glu Ile Gly Lys Leu Lys Thr
   1               5                   10                  15
   gtg ttg tta aaa aga cca gga aaa gaa tta gaa aat tta gta cct gat       96
   Val Leu Leu Lys Arg Pro Gly Lys Glu Leu Glu Asn Leu Val Pro Asp
               20                  25                  30
   cat tta agt ggt tta tta ttc gat gat att ccc tac tta aaa gtt gca      144
   His Leu Ser Gly Leu Leu Phe Asp Asp Ile Pro Tyr Leu Lys Val Ala
           35                  40                  45
   caa gaa gag cat gac aaa ttt gct caa act ttg aga gat gaa gga atc      192
   Gln Glu Glu His Asp Lys Phe Ala Gln Thr Leu Arg Asp Glu Gly Ile
       50                  55                  60
   gaa gta gtt tat tta gaa aaa ctt gca gca gaa tct att act gag cca      240
   Glu Val Val Tyr Leu Glu Lys Leu Ala Ala Glu Ser Ile Thr Glu Pro
   65                  70                  75                  80
   gaa gta cgc gag aac ttc ata aac gat ata tta aca gaa tct aaa aag      288
   Glu Val Arg Glu Asn Phe Ile Asn Asp Ile Leu Thr Glu Ser Lys Lys
                   85                  90                  95
   aca ata tta ggt cat gaa act gaa att aaa gaa ttc ttt tca aag tta      336
   Thr Ile Leu Gly His Glu Thr Glu Ile Lys Glu Phe Phe Ser Lys Leu
               100                 105                 110
   tct gac caa gaa ctt gta aat aaa atc atg gct ggc ata cgt aaa gaa      384
   Ser Asp Gln Glu Leu Val Asn Lys Ile Met Ala Gly Ile Arg Lys Glu
           115                 120                 125
   gaa att caa ctt gaa aca act cat tta gta gaa tat atg gat gat aga      432
   Glu Ile Gln Leu Glu Thr Thr His Leu Val Glu Tyr Met Asp Asp Arg
       130                 135                 140
   tat cca ttt tac tta gat cca atg ccc aac ctt tat ttt aca aga gat       480
   Tyr Pro Phe Tyr Leu Asp Pro Met Pro Asn Leu Tyr Phe Thr Arg Asp
   145                 150                 155                 160
   ccc caa gct tca att ggt aga gga atg aca att aac aga atg tat tgg       528
   Pro Gln Ala Ser Ile Gly Arg Gly Met Thr Ile Asn Arg Met Tyr Trp
                   165                 170                 175
   aga gca cga cgt aga gaa tct att ttt atg aca tat ata ctg aaa cat       576
   Arg Ala Arg Arg Arg Glu Ser Ile Phe Met Thr Tyr Ile Leu Lys His
               180                 185                 190
   cat cca aga ttt aaa gat aaa gat gta cca gta tgg tta gat cgt aac       624
   His Pro Arg Phe Lys Asp Lys Asp Val Pro Val Trp Leu Asp Arg Asn
           195                 200                 205
   tca cca ttt aat att gaa ggt gga gat gaa tta gta tta tcg aaa gat       672
   Ser Pro Phe Asn Ile Glu Gly Gly Asp Glu Leu Val Leu Ser Lys Asp
       210                 215                 220
   gtt tta gct att ggt ata tca gaa cgt aca tca gct caa gca ata gaa       720
   Val Leu Ala Ile Gly Ile Ser Glu Arg Thr Ser Ala Gln Ala Ile Glu
   225                 230                 235                 240
   aag tta gca cgt aat att ttc aaa gat gca aac aca agt ttt aaa aaa       768
   Lys Leu Ala Arg Asn Ile Phe Lys Asp Ala Asn Thr Ser Phe Lys Lys
                   245                 250                 255
   atc gta gct att gaa ata cct aat aca cgc aca ttt atg cac cta gat       816
   Ile Val Ala Ile Glu Ile Pro Asn Thr Arg Thr Phe Met His Leu Asp
               260                 265                 270
   aca gta cta act atg att gac tac gat aag ttt aca gta cat gca gca       864
   Thr Val Leu Thr Met Ile Asp Tyr Asp Lys Phe Thr Val His Ala Ala
           275                 280                 285
   ata ttt aaa gaa gaa aat aat atg aat ata ttt acc ata gaa caa aat       912
   Ile Phe Lys Glu Glu Asn Asn Met Asn Ile Phe Thr Ile Glu Gln Asn
       290                 295                 300
   gat ggt aag gac gat ata aaa att act cgt tct agc aag tta cgt gaa       960
   Asp Gly Lys Asp Asp Ile Lys Ile Thr Arg Ser Ser Lys Leu Arg Glu
   305                 310                 315                 320
   aca ctt gct gaa gtt tta gaa gta gaa aaa gtg gac ttt att cca aca      1008
   Thr Leu Ala Glu Val Leu Glu Val Glu Lys Val Asp Phe Ile Pro Thr
                   325                 330                 335
   ggt aat ggc gac gtt att gat ggt gca cgt gaa caa tgg aat gat ggc      1056
   Gly Asn Gly Asp Val Ile Asp Gly Ala Arg Glu Gln Trp Asn Asp Gly
               340                 345                 350
   tca aac aca tta tgt att cga cca ggg gtt gtg gtg aca tac gat cgc      1104
   Ser Asn Thr Leu Cys Ile Arg Pro Gly Val Val Val Thr Tyr Asp Arg
           355                 360                 365
   aac tat gta tca aac caa ctt tta cgc gac aaa gga att aaa gtg att      1152
   Asn Tyr Val Ser Asn Gln Leu Leu Arg Asp Lys Gly Ile Lys Val Ile
       370                 375                 380
   gaa att act ggt agt gaa ctt gta cgt gga cgc gga ggc cca aga tgt     1200
   Glu Ile Thr Gly Ser Glu Leu Val Arg Gly Arg Gly Gly Pro Arg Cys
   385                 390                 395                 400
   atg agt cag ccg tta ttt aga gaa gat att                             1230
   Met Ser Gln Pro Leu Phe Arg Glu Asp Ile
                   405                 410
<210> 20
   <211> 410
   <212> PRT
   <213> Staphylococcus aureus
<400> 20
   Val Gln Gly Pro Ile Gln Val Asn Ser Glu Ile Gly Lys Leu Lys Thr
   1               5                   10                  15
   Val Leu Leu Lys Arg Pro Gly Lys Glu Leu Glu Asn Leu Val Pro Asp
               20                  25                  30
   His Leu Ser Gly Leu Leu Phe Asp Asp Ile Pro Tyr Leu Lys Val Ala
           35                  40                  45
   Gln Glu Glu His Asp Lys Phe Ala Gln Thr Leu Arg Asp Glu Gly Ile
       50                  55                  60
   Glu Val Val Tyr Leu Glu Lys Leu Ala Ala Glu Ser Ile Thr Glu Pro
   65                  70                  75                  80
   Glu Val Arg Glu Asn Phe Ile Asn Asp Ile Leu Thr Glu Ser Lys Lys
                   85                  90                  95
   Thr Ile Leu Gly His Glu Thr Glu Ile Lys Glu Phe Phe Ser Lys Leu
               100                 105                 110
   Ser Asp Gln Glu Leu Val Asn Lys Ile Met Ala Gly Ile Arg Lys Glu
           115                 120                 125
   Glu Ile Gln Leu Glu Thr Thr His Leu Val Glu Tyr Met Asp Asp Arg
       130                 135                 140
   Tyr Pro Phe Tyr Leu Asp Pro Met Pro Asn Leu Tyr Phe Thr Arg Asp
   145                 150                 155                 160
   Pro Gln Ala Ser Ile Gly Arg Gly Met Thr Ile Asn Arg Met Tyr Trp
   165 170 175
   Arg Ala Arg Arg Arg Glu Ser Ile Phe Met Thr Tyr Ile Leu Lys His
   180 185 190
   His Pro Arg Phe Lys Asp Lys Asp Val Pro Val Trp Leu Asp Arg Asn
   195 200 205
   Ser Pro Phe Asn Ile Glu Gly Gly Asp Glu Leu Val Leu Ser Lys Asp
   210 215 220
   Val Leu Ala Ile Gly Ile Ser Glu Arg Thr Ser Ala Gln Ala Ile Glu
   225 230 235 240
   Lys Leu Ala Arg Asn Ile Phe Lys Asp Ala Asn Thr Ser Phe Lys Lys
   245 250 255
   Ile Val Ala Ile Glu Ile Pro Asn Thr Arg Thr Phe Met His Leu Asp
   260 265 270
   Thr Val Leu Thr Met Ile Asp Tyr Asp Lys Phe Thr Val His Ala Ala
   275 280 285
   Ile Phe Lys Glu Glu Asn Asn Met Asn Ile Phe Thr Ile Glu Gln Asn
   290 295 300
   Asp Gly Lys Asp Asp Ile Lys Ile Thr Arg Ser Ser Lys Leu Arg Glu
   305 310 315 320
   Thr Leu Ala Glu Val Leu Glu Val Glu Lys Val Asp Phe Ile Pro Thr
   325 330 335
   Gly Asn Gly Asp Val Ile Asp Gly Ala Arg Glu Gln Trp Asn Asp Gly
   340 345 350
   Ser Asn Thr Leu Cys Ile Arg Pro Gly Val Val Val Thr Tyr Asp Arg
   355 360 365
   Asn Tyr Val Ser Asn Gln Leu Leu Arg Asp Lys Gly Ile Lys Val Ile
   370 375 380
   Glu Ile Thr Gly Ser Glu Leu Val Arg Gly Arg Gly Gly Pro Arg Cys
   385 390 395 400
   Met Ser Gln Pro Leu Phe Arg Glu Asp Ile
                   405                 410
<210> 21
   <211> 867
   <212> DNA
   <213> Staphylococcus epidermidis
<220>
   <221> CDS
   <222> (1)..(867)
   <223>
<400> 21
   caa caa gca agt gca aca agt tct aaa act tca gaa aat cca gca acg      48
   Gln Gln Ala Ser Ala Thr Ser Ser Lys Thr Ser Glu Asn Pro Ala Thr
   1               5                   10                  15
   att gaa gaa gta tta ggt ctt agt caa gcc att tac gat aca aaa aat      96
   Ile Glu Glu Val Leu Gly Leu Ser Gln Ala Ile Tyr Asp Thr Lys Asn
               20                  25                  30
   gca tta aat ggt gaa caa cga ctt gca act gag aag agc aaa gat cta     144
   Ala Leu Asn Gly Glu Gln Arg Leu Ala Thr Glu Lys Ser Lys Asp Leu
           35                  40                  45
   aaa tta ata aaa gga tta aaa gat tta aat aaa gca caa ctt gaa gat     192
   Lys Leu Ile Lys Gly Leu Lys Asp Leu Asn Lys Ala Gln Leu Glu Asp
       50                  55                  60
   gtc aca aac aag gta aat tca gca aat act tta aca gag tta tct cag     240
   Val Thr Asn Lys Val Asn Ser Ala Asn Thr Leu Thr Glu Leu Ser Gln
   65                  70                  75                  80
   ctc act caa tca acg tta gaa tta aac gat aaa atg aaa tta ttg aga     288
   Leu Thr Gln Ser Thr Leu Glu Leu Asn Asp Lys Met Lys Leu Leu Arg
                   85                  90                  95
   gat aag ctt aaa act tta gta aat cct gtt aaa gca agt tta aat tat     336
   Asp Lys Leu Lys Thr Leu Val Asn Pro Val Lys Ala Ser Leu Asn Tyr
               100                 105                 110
   aga aac gct gat tat aat tta aaa cgt caa ttt aac aaa gct tta aaa     384
   Arg Asn Ala Asp Tyr Asn Leu Lys Arg Gln Phe Asn Lys Ala Leu Lys
           115                 120                 125
   gaa gct aaa ggc gta tta aat aaa aat agc ggt aca aat gtc aat atc     432
   Glu Ala Lys Gly Val Leu Asn Lys Asn Ser Gly Thr Asn Val Asn Ile
       130                 135                 140
   aat gac att caa cat ctt tta aca caa ata gat aat gct aaa gac caa      480
   Asn Asp Ile Gln His Leu Leu Thr Gln Ile Asp Asn Ala Lys Asp Gln
   145                 150                 155                 160
   tta aat ggt gaa cga cgt cta aaa gaa cat caa caa aaa tct gaa gta      528
   Leu Asn Gly Glu Arg Arg Leu Lys Glu His Gln Gln Lys Ser Glu Val
                   165                 170                 175
   ttt att att aaa gaa tta gat ata ctt aat aat gct caa aaa gct gca      576
   Phe Ile Ile Lys Glu Leu Asp Ile Leu Asn Asn Ala Gln Lys Ala Ala
               180                 185                 190
   ata att aat cag att aga gcg tct aaa gac att aaa ata att aat caa      624
   Ile Ile Asn Gln Ile Arg Ala Ser Lys Asp Ile Lys Ile Ile Asn Gln
           195                 200                 205
   atc gtt gat aat gca ata gaa tta aat gat gct atg caa ggt tta aaa      672
   Ile Val Asp Asn Ala Ile Glu Leu Asn Asp Ala Met Gln Gly Leu Lys
       210                 215                 220
   gaa cat gta gct caa tta aca gca act aca aaa gac aac att gaa tat      720
   Glu His Val Ala Gln Leu Thr Ala Thr Thr Lys Asp Asn Ile Glu Tyr
   225                 230                 235                 240
   tta aat gct gat gaa gac cat aaa tta caa tat gat tac gct atc aac      768
   Leu Asn Ala Asp Glu Asp His Lys Leu Gln Tyr Asp Tyr Ala Ile Asn
                   245                 250                 255
   tta gcg aat aat gtt ctt gac aaa gaa aac ggt aca aat aaa gac gct      816
   Leu Ala Asn Asn Val Leu Asp Lys Glu Asn Gly Thr Asn Lys Asp Ala
               260                 265                 270
   aat atc ata att gga atg att caa aac atg gat gat gct aga gca ctt      864
   Asn Ile Ile Ile Gly Met Ile Gln Asn Met Asp Asp Ala Arg Ala Leu
           275                 280                 285
   cta                                                                  867
   Leu
<210> 22
   <211> 289
   <212> PRT
   <213> Staphylococcus epidermidis
<400> 22
   Gln Gln Ala Ser Ala Thr Ser Ser Lys Thr Ser Glu Asn Pro Ala Thr
   1               5                   10                  15
   Ile Glu Glu Val Leu Gly Leu Ser Gln Ala Ile Tyr Asp Thr Lys Asn
               20                  25                  30
   Ala Leu Asn Gly Glu Gln Arg Leu Ala Thr Glu Lys Ser Lys Asp Leu
           35                  40                  45
   Lys Leu Ile Lys Gly Leu Lys Asp Leu Asn Lys Ala Gln Leu Glu Asp
       50                  55                  60
   Val Thr Asn Lys Val Asn Ser Ala Asn Thr Leu Thr Glu Leu Ser Gln
   65                  70                  75                  80
   Leu Thr Gln Ser Thr Leu Glu Leu Asn Asp Lys Met Lys Leu Leu Arg
                   85                  90                  95
   Asp Lys Leu Lys Thr Leu Val Asn Pro Val Lys Ala Ser Leu Asn Tyr
               100                 105                 110
   Arg Asn Ala Asp Tyr Asn Leu Lys Arg Gln Phe Asn Lys Ala Leu Lys
           115                 120                 125
   Glu Ala Lys Gly Val Leu Asn Lys Asn Ser Gly Thr Asn Val Asn Ile
       130                 135                 140
   Asn Asp Ile Gln His Leu Leu Thr Gln Ile Asp Asn Ala Lys Asp Gln
   145                 150                 155                 160
   Leu Asn Gly Glu Arg Arg Leu Lys Glu His Gln Gln Lys Ser Glu Val
                   165                 170                 175
   Phe Ile Ile Lys Glu Leu Asp Ile Leu Asn Asn Ala Gln Lys Ala Ala
               180                 185                 190
   Ile Ile Asn Gln Ile Arg Ala Ser Lys Asp Ile Lys Ile Ile Asn Gln
           195                 200                 205
   Ile Val Asp Asn Ala Ile Glu Leu Asn Asp Ala Met Gln Gly Leu Lys
       210                 215                 220
   Glu His Val Ala Gln Leu Thr Ala Thr Thr Lys Asp Asn Ile Glu Tyr
   225                 230                 235                 240
   Leu Asn Ala Asp Glu Asp His Lys Leu Gln Tyr Asp Tyr Ala Ile Asn
                   245                 250                 255
   Leu Ala Asn Asn Val Leu Asp Lys Glu Asn Gly Thr Asn Lys Asp Ala
               260                 265                 270
   Asn Ile Ile Ile Gly Met Ile Gln Asn Met Asp Asp Ala Arg Ala Leu
           275                 280                     285
   Leu
<210> 23
   <211> 621
   <212> DNA
   <213> Staphylococcus epidermidis
<220>
   <221> CDS
   <222> (1)..(621)
   <223>
<400> 23
   tca gaa act aca aac gtt gat aaa gca cac tta gta gat tta gca caa      48
   Ser Glu Thr Thr Asn Val Asp Lys Ala His Leu Val Asp Leu Ala Gln
   1               5                   10                  15
   cat aat cct gaa gaa tta aat gct aaa cca gtt caa gct ggt gct tac      96
   His Asn Pro Glu Glu Leu Asn Ala Lys Pro Val Gln Ala Gly Ala Tyr
               20                  25                  30
   gat att cat ttc gta gac aat gga tac caa tac aac ttc act tca aat     144
   Asp Ile His Phe Val Asp Asn Gly Tyr Gln Tyr Asn Phe Thr Ser Asn
           35                  40                  45
   ggt tct gaa tgg tca tgg agc tac gct gta gct ggt tca gat gct gat     192
   Gly Ser Glu Trp Ser Trp Ser Tyr Ala Val Ala Gly Ser Asp Ala Asp
       50                  55                  60
   tac aca gaa tca tca tca aac caa gaa gta agt gca aat aca caa tct     240
   Tyr Thr Glu Ser Ser Ser Asn Gln Glu Val Ser Ala Asn Thr Gln Ser
   65                  70                  75                  80
   agt aac aca aat gta caa gct gtt tca gct cca act tct tca gaa agt     288
   Ser Asn Thr Asn Val Gln Ala Val Ser Ala Pro Thr Ser Ser Glu Ser
                   85                  90                  95
   cgt agc tac agc aca tca act act tca tac tca gca cca agc cat aac     336
   Arg Ser Tyr Ser Thr Ser Thr Thr Ser Tyr Ser Ala Pro Ser His Asn
               100                 105                 110
   tac agc tct cac agt agt tca gta aga tta tca aat ggt aat act gct     384
   Tyr Ser Ser His Ser Ser Ser Val Arg Leu Ser Asn Gly Asn Thr Ala
           115                 120                 125
   ggt tct gta ggt tca tat gct gct gct caa atg gct gca cgt act ggt      432
   Gly Ser Val Gly Ser Tyr Ala Ala Ala Gln Met Ala Ala Arg Thr Gly
       130                 135                     140
   gta tct gct tca aca tgg gaa cac atc att gct aga gaa tca aat ggt      480
   Val Ser Ala Ser Thr Trp Glu His Ile Ile Ala Arg Glu Ser Asn Gly
   145                 150                     155                 160
   caa tta cat gca cgt aat gct tca ggt gct gct gga tta ttc caa act      528
   Gln Leu His Ala Arg Asn Ala Ser Gly Ala Ala Gly Leu Phe Gln Thr
                   165                     170                 175
   atg cca ggt tgg ggt tca act ggt tca gta aat gat caa atc aat gcc      576
   Met Pro Gly Trp Gly Ser Thr Gly Ser Val Asn Asp Gln Ile Asn Ala
               180                     185                 190
   gct tat aaa gca tat aaa gca caa ggt tta tct gct tgg ggt atg          621
   Ala Tyr Lys Ala Tyr Lys Ala Gln Gly Leu Ser Ala Trp Gly Met
           195                     200                 205
<210> 24
   <211> 207
   <212> PRT
   <213> Staphylococcus epidermidis
<400> 24
   Ser Glu Thr Thr Asn Val Asp Lys Ala His Leu Val Asp Leu Ala Gln
   1               5                   10                  15
   His Asn Pro Glu Glu Leu Asn Ala Lys Pro Val Gln Ala Gly Ala Tyr
               20                  25                  30
   Asp Ile His Phe Val Asp Asn Gly Tyr Gln Tyr Asn Phe Thr Ser Asn
           35                  40                  45
   Gly Ser Glu Trp Ser Trp Ser Tyr Ala Val Ala Gly Ser Asp Ala Asp
       50                  55                  60
   Tyr Thr Glu Ser Ser Ser Asn Gln Glu Val Ser Ala Asn Thr Gln Ser
   65                  70                  75                  80
   Ser Asn Thr Asn Val Gln Ala Val Ser Ala Pro Thr Ser Ser Glu Ser
                   85                  90                  95
   Arg Ser Tyr Ser Thr Ser Thr Thr Ser Tyr Ser Ala Pro Ser His Asn
               100                 105                 110
   Tyr Ser Ser His Ser Ser Ser Val Arg Leu Ser Asn Gly Asn Thr Ala
           115                 120                 125
   Gly Ser Val Gly Ser Tyr Ala Ala Ala Gln Met Ala Ala Arg Thr Gly
       130                 135                 140
   Val Ser Ala Ser Thr Trp Glu His Ile Ile Ala Arg Glu Ser Asn Gly
   145                 150                 155                 160
   Gln Leu His Ala Arg Asn Ala Ser Gly Ala Ala Gly Leu Phe Gln Thr
                   165                 170                 175
   Met Pro Gly Trp Gly Ser Thr Gly Ser Val Asn Asp Gln Ile Asn Ala
               180                 185                 190
   Ala Tyr Lys Ala Tyr Lys Ala Gln Gly Leu Ser Ala Trp Gly Met
           195                 200                 205
<210> 25
   <211> 456
   <212> DNA
   <213> Staphylococcus aureus
<220>
   <221> CDS
   <222> (1)..(456)
   <223>
<400> 25
   aag aag aaa tca cca ggt aca tta gcg gaa aat cgt aaa gca aga cat      48
   Lys Lys Lys Ser Pro Gly Thr Leu Ala Glu Asn Arg Lys Ala Arg His
   1               5                   10                  15
   gat tat aat att gaa gat acg att gaa gcg gga atc gta tta caa ggt      96
   Asp Tyr Asn Ile Glu Asp Thr Ile Glu Ala Gly Ile Val Leu Gln Gly
               20                  25                  30
   aca gaa ata aaa tcg att cgc cga ggt agt gct aac ctt aaa gat agt     144
   Thr Glu Ile Lys Ser Ile Arg Arg Gly Ser Ala Asn Leu Lys Asp Ser
           35                  40                  45
   tac gcg caa gtt aaa aac ggt gaa atg tat ttg aat aat atg cat ata     192
   Tyr Ala Gln Val Lys Asn Gly Glu Met Tyr Leu Asn Asn Met His Ile
       50                  55                  60
   gca cca tac gaa gaa ggg aat cgt ttt aat cac gat cct ctt cgt tct        240
   Ala Pro Tyr Glu Glu Gly Asn Arg Phe Asn His Asp Pro Leu Arg Ser
   65                  70                  75                  80
   cga aaa tta tta ttg cat aaa cgt gaa atc ttt aaa tta ggt gaa caa        288
   Arg Lys Leu Leu Leu His Lys Arg Glu Ile Phe Lys Leu Gly Glu Gln
                   85                  90                  95
   act cga gaa att ggt tat tcg att gtg ccg tta aag ctt tat ttg aag        336
   Thr Arg Glu Ile Gly Tyr Ser Ile Val Pro Leu Lys Leu Tyr Leu Lys
               100                 105                 110
   cat gga cat tgt aaa gta tta ctt ggt gtt gca cga ggt aag aaa aaa        384
   His Gly His Cys Lys Val Leu Leu Gly Val Ala Arg Gly Lys Lys Lys
           115                 120                 125
   tat gat aaa cgt caa gct ttg aaa gaa aaa gca gtc aaa cga gat gtt        432
   Tyr Asp Lys Arg Gln Ala Leu Lys Glu Lys Ala Val Lys Arg Asp Val
       130                 135                 140
   gcg cgc gat atg aaa gcc cgt tat                                        456
   Ala Arg Asp Met Lys Ala Arg Tyr
   145                 150
<210> 26
   <211> 152
   <212> PRT
   <213> Staphylococcus aureus
<400> 26
   Lys Lys Lys Ser Pro Gly Thr Leu Ala Glu Asn Arg Lys Ala Arg His
   1               5                   10                  15
   Asp Tyr Asn Ile Glu Asp Thr Ile Glu Ala Gly Ile Val Leu Gln Gly
               20                  25                  30
   Thr Glu Ile Lys Ser Ile Arg Arg Gly Ser Ala Asn Leu Lys Asp Ser
           35                  40                  45
   Tyr Ala Gln Val Lys Asn Gly Glu Met Tyr Leu Asn Asn Met His Ile
       50                  55                  60
   Ala Pro Tyr Glu Glu Gly Asn Arg Phe Asn His Asp Pro Leu Arg Ser
   65                  70                  75                  80
   Arg Lys Leu Leu Leu His Lys Arg Glu Ile Phe Lys Leu Gly Glu Gln
                   85                  90                  95
   Thr Arg Glu Ile Gly Tyr Ser Ile Val Pro Leu Lys Leu Tyr Leu Lys
               100                 105                 110
   His Gly His Cys Lys Val Leu Leu Gly Val Ala Arg Gly Lys Lys Lys
           115                 120                 125
   Tyr Asp Lys Arg Gln Ala Leu Lys Glu Lys Ala Val Lys Arg Asp Val
       130                 135                 140
   Ala Arg Asp Met Lys Ala Arg Tyr
   145                 150
<210> 27
   <211> 1932
   <212> DNA
   <213> Staphylococcus aureus
<220>
   <221> CDS
   <222> (1)..(1932)
   <223>
<400> 27
   tcc aaa gat aaa gaa att aat aat act att gat gca att gaa gat aaa       48
   Ser Lys Asp Lys Glu Ile Asn Asn Thr Ile Asp Ala Ile Glu Asp Lys
   1               5                   10                  15
   aat ttc aaa caa gtt tat aaa gat agc agt tat att tct aaa agc gat       96
   Asn Phe Lys Gln Val Tyr Lys Asp Ser Ser Tyr Ile Ser Lys Ser Asp
               20                  25                  30
   aat ggt gaa gta gaa atg act gaa cgt ccg ata aaa ata tat aat agt      144
   Asn Gly Glu Val Glu Met Thr Glu Arg Pro Ile Lys Ile Tyr Asn Ser
           35                  40                  45
   tta ggc gtt aaa gat ata aac att cag gat cgt aaa ata aaa aaa gta      192
   Leu Gly Val Lys Asp Ile Asn Ile Gln Asp Arg Lys Ile Lys Lys Val
       50                  55                  60
   tct aaa aat aaa aaa cga gta gat gct caa tat aaa att aaa aca aac      240
   Ser Lys Asn Lys Lys Arg Val Asp Ala Gln Tyr Lys Ile Lys Thr Asn
   65                  70                  75                  80
   tac ggt aac att gat cgc aac gtt caa ttt aat ttt gtt aaa gaa gat      288
   Tyr Gly Asn Ile Asp Arg Asn Val Gln Phe Asn Phe Val Lys Glu Asp
                   85                  90                  95
   ggt atg tgg aag tta gat tgg gat cat agc gtc att att cca gga atg      336
   Gly Met Trp Lys Leu Asp Trp Asp His Ser Val Ile Ile Pro Gly Met
               100                 105                 110
   cag aaa gac caa agc ata cat att gaa aat tta aaa tca gaa cgt ggt      384
   Gln Lys Asp Gln Ser Ile His Ile Glu Asn Leu Lys Ser Glu Arg Gly
           115                 120                 125
   aaa att tta gac cga aac aat gtg gaa ttg gcc aat aca gga aca gca      432
   Lys Ile Leu Asp Arg Asn Asn Val Glu Leu Ala Asn Thr Gly Thr Ala
       130                 135                 140
   tat gag ata ggc atc gtt cca aag aat gta tct aaa aaa gat tat aaa      480
   Tyr Glu Ile Gly Ile Val Pro Lys Asn Val Ser Lys Lys Asp Tyr Lys
   145                 150                 155                 160
   gca atc gct aaa gaa cta agt att tct gaa gac tat atc aaa caa caa      528
   Ala Ile Ala Lys Glu Leu Ser Ile Ser Glu Asp Tyr Ile Lys Gln Gln
                   165                 170                 175
   atg gat caa aat tgg gta caa gat gat acc ttc gtt cca ctt aaa acc      576
   Met Asp Gln Asn Trp Val Gln Asp Asp Thr Phe Val Pro Leu Lys Thr
               180                 185                 190
   gtt aaa aaa atg gat gaa tat tta agt gat ttc gca aaa aaa ttt cat      624
   Val Lys Lys Met Asp Glu Tyr Leu Ser Asp Phe Ala Lys Lys Phe His
           195                 200                 205
   ctt aca act aat gaa aca gaa agt cgt aac tat cct cta gga aaa gcg      672
   Leu Thr Thr Asn Glu Thr Glu Ser Arg Asn Tyr Pro Leu Gly Lys Ala
       210                 215                 220
   act tca cat cta tta ggt tat gtt ggt ccc att aac tct gaa gaa tta      720
   Thr Ser His Leu Leu Gly Tyr Val Gly Pro Ile Asn Ser Glu Glu Leu
   225                 230                 235                 240
   aaa caa aaa gaa tat aaa ggc tat aaa gat gat gca gtt att ggt aaa      768
   Lys Gln Lys Glu Tyr Lys Gly Tyr Lys Asp Asp Ala Val Ile Gly Lys
                   245                 250                 255
   aag gga ctc gaa aaa ctt tac gat aaa aag ctc caa cat gaa gat ggc      816
   Lys Gly Leu Glu Lys Leu Tyr Asp Lys Lys Leu Gln His Glu Asp Gly
               260                 265                 270
   tat cgt gtc aca atc gtt gac gat aat agc aat aca atc gca cat aca      864
   Tyr Arg Val Thr Ile Val Asp Asp Asn Ser Asn Thr Ile Ala His Thr
           275                 280                 285
   tta ata gag aaa aag aaa aaa gat ggc aaa gat att caa cta act att      912
   Leu Ile Glu Lys Lys Lys Lys Asp Gly Lys Asp Ile Gln Leu Thr Ile
       290                 295                 300
   gat gct aaa gtt caa aag agt att tat aac aac atg aaa aat gat tat      960
   Asp Ala Lys Val Gln Lys Ser Ile Tyr Asn Asn Met Lys Asn Asp Tyr
   305                 310                 315                 320
   ggc tca ggt act gct atc cac cct caa aca ggt gaa tta tta gca ctt     1008
   Gly Ser Gly Thr Ala Ile His Pro Gln Thr Gly Glu Leu Leu Ala Leu
                   325                 330                 335
   gta agc aca cct tca tat gac gtc tat cca ttt atg tat ggc atg agt      1056
   Val Ser Thr Pro Ser Tyr Asp Val Tyr Pro Phe Met Tyr Gly Met Ser
               340                 345                 350
   aac gaa gaa tat aat aaa tta acc gaa gat aaa aaa gaa cct ctg ctc      1104
   Asn Glu Glu Tyr Asn Lys Leu Thr Glu Asp Lys Lys Glu Pro Leu Leu
           355                 360                 365
   aac aag ttc cag att aca act tca cca ggt tca act caa aaa ata tta      1152
   Asn Lys Phe Gln Ile Thr Thr Ser Pro Gly Ser Thr Gln Lys Ile Leu
       370                 375                 380
   aca gca atg att ggg tta aat aac aaa aca tta gac gat aaa aca agt      1200
   Thr Ala Met Ile Gly Leu Asn Asn Lys Thr Leu Asp Asp Lys Thr Ser
   385                 390                 395                 400
   tat aaa atc gat ggt aaa ggt tgg caa aaa gat aaa tct tgg ggt ggt      1248
   Tyr Lys Ile Asp Gly Lys Gly Trp Gln Lys Asp Lys Ser Trp Gly Gly
                   405                 410                 415
   tac aac gtt aca aga tat gaa gtg gta aat ggt aat atc gac tta aaa      1296
   Tyr Asn Val Thr Arg Tyr Glu Val Val Asn Gly Asn Ile Asp Leu Lys
               420                 425                 430
   caa gca ata gaa tca tca gat aac att ttc ttt gct aga gta gca ctc      1344
   Gln Ala Ile Glu Ser Ser Asp Asn Ile Phe Phe Ala Arg Val Ala Leu
           435                 440                 445
   gaa tta ggc agt aag aaa ttt gaa aaa ggc atg aaa aaa cta ggt gtt      1392
   Glu Leu Gly Ser Lys Lys Phe Glu Lys Gly Met Lys Lys Leu Gly Val
       450                 455                 460
   ggt gaa gat ata cca agt gat tat cca ttt tat aat gct caa att tca      1440
   Gly Glu Asp Ile Pro Ser Asp Tyr Pro Phe Tyr Asn Ala Gln Ile Ser
   465                 470                 475                 480
   aac aaa aat tta gat aat gaa ata tta tta gct gat tca ggt tac gga      1488
   Asn Lys Asn Leu Asp Asn Glu Ile Leu Leu Ala Asp Ser Gly Tyr Gly
                   485                 490                 495
   caa ggt gaa ata ctg att aac cca gta cag atc ctt tca atc tat agc      1536
   Gln Gly Glu Ile Leu Ile Asn Pro Val Gln Ile Leu Ser Ile Tyr Ser
               500                 505                 510
   gca tta gaa aat aat ggc aat att aac gca cct cac tta tta aaa gac      1584
   Ala Leu Glu Asn Asn Gly Asn Ile Asn Ala Pro His Leu Leu Lys Asp
           515                 520                 525
   acg aaa aac aaa gtt tgg aag aaa aat att att tcc aaa gaa aat atc      1632
   Thr Lys Asn Lys Val Trp Lys Lys Asn Ile Ile Ser Lys Glu Asn Ile
       530                 535                 540
   aat cta tta act gat ggt atg caa caa gtc gta aat aaa aca cat aaa      1680
   Asn Leu Leu Thr Asp Gly Met Gln Gln Val Val Asn Lys Thr His Lys
   545                 550                 555                 560
   gaa gat att tat aga tct tat gca aac tta att ggc aaa tcc ggt act      1728
   Glu Asp Ile Tyr Arg Ser Tyr Ala Asn Leu Ile Gly Lys Ser Gly Thr
                   565                 570                 575
   gca gaa ctc aaa atg aaa caa gga gaa act ggc aga caa att ggg tgg      1776
   Ala Glu Leu Lys Met Lys Gln Gly Glu Thr Gly Arg Gln Ile Gly Trp
               580                 585                 590
   ttt ata tca tat gat aaa gat aat cca aac atg atg atg gct att aat      1824
   Phe Ile Ser Tyr Asp Lys Asp Asn Pro Asn Met Met Met Ala Ile Asn
           595                 600                 605
   gtt aaa gat gta caa gat aaa gga atg gct agc tac aat gcc aaa atc      1872
   Val Lys Asp Val Gln Asp Lys Gly Met Ala Ser Tyr Asn Ala Lys Ile
       610                 615                 620
   tca ggt aaa gtg tat gat gag cta tat gag aac ggt aat aaa aaa tac      1920
   Ser Gly Lys Val Tyr Asp Glu Leu Tyr Glu Asn Gly Asn Lys Lys Tyr
   625                 630                 635                 640
   gat ata gat gaa                                                      1932
   Asp Ile Asp Glu
<210> 28
   <211> 644
   <212> PRT
   <213> Staphylococcus aureus
<400> 28
   Ser Lys Asp Lys Glu Ile Asn Asn Thr Ile Asp Ala Ile Glu Asp Lys
   1               5                   10                  15
   Asn Phe Lys Gln Val Tyr Lys Asp Ser Ser Tyr Ile Ser Lys Ser Asp
               20                  25                  30
   Asn Gly Glu Val Glu Met Thr Glu Arg Pro Ile Lys Ile Tyr Asn Ser
           35                  40                  45
   Leu Gly Val Lys Asp Ile Asn Ile Gln Asp Arg Lys Ile Lys Lys Val
       50                  55                  60
   Ser Lys Asn Lys Lys Arg Val Asp Ala Gln Tyr Lys Ile Lys Thr Asn
   65                  70                  75                  80
   Tyr Gly Asn Ile Asp Arg Asn Val Gln Phe Asn Phe Val Lys Glu Asp
                   85                  90                  95
   Gly Met Trp Lys Leu Asp Trp Asp His Ser Val Ile Ile Pro Gly Met
               100                 105                 110
   Gln Lys Asp Gln Ser Ile His Ile Glu Asn Leu Lys Ser Glu Arg Gly
           115                 120                 125
   Lys Ile Leu Asp Arg Asn Asn Val Glu Leu Ala Asn Thr Gly Thr Ala
       130                 135                 140
   Tyr Glu Ile Gly Ile Val Pro Lys Asn Val Ser Lys Lys Asp Tyr Lys
   145                 150                 155                 160
   Ala Ile Ala Lys Glu Leu Ser Ile Ser Glu Asp Tyr Ile Lys Gln Gln
                   165                 170                 175
   Met Asp Gln Asn Trp Val Gln Asp Asp Thr Phe Val Pro Leu Lys Thr
               180                 185                 190
   Val Lys Lys Met Asp Glu Tyr Leu Ser Asp Phe Ala Lys Lys Phe His
           195                 200                 205
   Leu Thr Thr Asn Glu Thr Glu Ser Arg Asn Tyr Pro Leu Gly Lys Ala
       210                 215                 220
   Thr Ser His Leu Leu Gly Tyr Val Gly Pro Ile Asn Ser Glu Glu Leu
   225                 230                 235                 240
   Lys Gln Lys Glu Tyr Lys Gly Tyr Lys Asp Asp Ala Val Ile Gly Lys
                   245                 250                 255
   Lys Gly Leu Glu Lys Leu Tyr Asp Lys Lys Leu Gln His Glu Asp Gly
               260                 265                 270
   Tyr Arg Val Thr Ile Val Asp Asp Asn Ser Asn Thr Ile Ala His Thr
           275                 280                 285
   Leu Ile Glu Lys Lys Lys Lys Asp Gly Lys Asp Ile Gln Leu Thr Ile
       290                 295                 300
   Asp Ala Lys Val Gln Lys Ser Ile Tyr Asn Asn Met Lys Asn Asp Tyr
   305                 310                 315                 320
   Gly Ser Gly Thr Ala Ile His Pro Gln Thr Gly Glu Leu Leu Ala Leu
                   325                 330                 335
   Val Ser Thr Pro Ser Tyr Asp Val Tyr Pro Phe Met Tyr Gly Met Ser
               340                 345                 350
   Asn Glu Glu Tyr Asn Lys Leu Thr Glu Asp Lys Lys Glu Pro Leu Leu
           355                 360                 365
   Asn Lys Phe Gln Ile Thr Thr Ser Pro Gly Ser Thr Gln Lys Ile Leu
       370                 375                 380
   Thr Ala Met Ile Gly Leu Asn Asn Lys Thr Leu Asp Asp Lys Thr Ser
   385                 390                 395                 400
   Tyr Lys Ile Asp Gly Lys Gly Trp Gln Lys Asp Lys Ser Trp Gly Gly
                   405                 410                 415
   Tyr Asn Val Thr Arg Tyr Glu Val Val Asn Gly Asn Ile Asp Leu Lys
               420                 425                 430
   Gln Ala Ile Glu Ser Ser Asp Asn Ile Phe Phe Ala Arg Val Ala Leu
           435                 440                 445
   Glu Leu Gly Ser Lys Lys Phe Glu Lys Gly Met Lys Lys Leu Gly Val
       450                 455                 460
   Gly Glu Asp Ile Pro Ser Asp Tyr Pro Phe Tyr Asn Ala Gln Ile Ser
   465                 470                 475                 480
   Asn Lys Asn Leu Asp Asn Glu Ile Leu Leu Ala Asp Ser Gly Tyr Gly
                   485                 490                 495
   Gln Gly Glu Ile Leu Ile Asn Pro Val Gln Ile Leu Ser Ile Tyr Ser
               500                 505                 510
   Ala Leu Glu Asn Asn Gly Asn Ile Asn Ala Pro His Leu Leu Lys Asp
           515                 520                 525
   Thr Lys Asn Lys Val Trp Lys Lys Asn Ile Ile Ser Lys Glu Asn Ile
       530                 535                 540
   Asn Leu Leu Thr Asp Gly Met Gln Gln Val Val Asn Lys Thr His Lys
   545                 550                 555                 560
   Glu Asp Ile Tyr Arg Ser Tyr Ala Asn Leu Ile Gly Lys Ser Gly Thr
                   565                 570                 575
   Ala Glu Leu Lys Met Lys Gln Gly Glu Thr Gly Arg Gln Ile Gly Trp
               580                 585                 590
   Phe Ile Ser Tyr Asp Lys Asp Asn Pro Asn Met Met Met Ala Ile Asn
           595                 600                 605
   Val Lys Asp Val Gln Asp Lys Gly Met Ala Ser Tyr Asn Ala Lys Ile
       610                 615                 620
   Ser Gly Lys Val Tyr Asp Glu Leu Tyr Glu Asn Gly Asn Lys Lys Tyr
   625                 630                 635                 640
   Asp Ile Asp Glu
<210> 29
   <211> 675
   <212> DNA
   <213> Staphylococcus aureus
<220>
   <221> CDS
   <222> (1)..(675)
   <223>
<400> 29
   ttt gat gga agt ttt gtg ctt tat aat gaa cgg gag caa gct tat tct       48
   Phe Asp Gly Ser Phe Val Leu Tyr Asn Glu Arg Glu Gln Ala Tyr Ser
   1               5                   10                  15
   att tat aat gaa cca gaa agt aaa caa cga tat tca cct aat tct act       96
   Ile Tyr Asn Glu Pro Glu Ser Lys Gln Arg Tyr Ser Pro Asn Ser Thr
               20                  25                  30
   tac aaa att tat tta gcg tta atg gca ttc gac caa aat tta ctc tca      144
   Tyr Lys Ile Tyr Leu Ala Leu Met Ala Phe Asp Gln Asn Leu Leu Ser
           35                  40                  45
   tta aat cat act gaa caa caa tgg gat aaa cat caa tat cca ttt aaa      192
   Leu Asn His Thr Glu Gln Gln Trp Asp Lys His Gln Tyr Pro Phe Lys
       50                  55                  60
   gaa tgg aac caa gat caa aat tta aat tct tca atg aaa tat tca gta      240
   Glu Trp Asn Gln Asp Gln Asn Leu Asn Ser Ser Met Lys Tyr Ser Val
   65                  70                  75                  80
   aat tgg tat tac gaa aat tta aac aaa cat tta aga caa gat gag gtt      288
   Asn Trp Tyr Tyr Glu Asn Leu Asn Lys His Leu Arg Gln Asp Glu Val
                   85                  90                  95
   aaa tct tat tta gat cta att gaa tat ggt aat gaa gaa ata tca ggg      336
   Lys Ser Tyr Leu Asp Leu Ile Glu Tyr Gly Asn Glu Glu Ile Ser Gly
               100                 105                 110
   aat gaa aat tat tgg aat gaa tct tca tta aaa att tct gca ata gaa      384
   Asn Glu Asn Tyr Trp Asn Glu Ser Ser Leu Lys Ile Ser Ala Ile Glu
           115                 120                 125
   cag gtt aat ttg ttg aaa aat atg aaa caa cat aac atg cat ttt gat      432
   Gln Val Asn Leu Leu Lys Asn Met Lys Gln His Asn Met His Phe Asp
       130                 135                 140
   aat aag gct att gaa aaa gtt gaa aat agt atg act ttg aaa caa aaa      480
   Asn Lys Ala Ile Glu Lys Val Glu Asn Ser Met Thr Leu Lys Gln Lys
   145                 150                 155                 160
   gat act tat aaa tat gta ggt aaa act gga aca gga atc gtg aat cac      528
   Asp Thr Tyr Lys Tyr Val Gly Lys Thr Gly Thr Gly Ile Val Asn His
                   165                 170                 175
   aaa gaa gca aat gga tgg ttc gta ggt tat gtt gaa acg aaa gat aat      576
   Lys Glu Ala Asn Gly Trp Phe Val Gly Tyr Val Glu Thr Lys Asp Asn
               180                 185                 190
   acg tat tat ttt gct aca cat tta aaa ggc gaa gac aat gcg aat ggc      624
   Thr Tyr Tyr Phe Ala Thr His Leu Lys Gly Glu Asp Asn Ala Asn Gly
           195                 200                 205
   gaa aaa gca caa caa att tct gag cgt att tta aaa gaa atg gag tta      672
   Glu Lys Ala Gln Gln Ile Ser Glu Arg Ile Leu Lys Glu Met Glu Leu
       210                 215                 220
   ata                                                                  675
   Ile
   225
<210> 30
   <211> 225
   <212> PRT
   <213> Staphylococcus aureus
<400> 30
   Phe Asp Gly Ser Phe Val Leu Tyr Asn Glu Arg Glu Gln Ala Tyr Ser
   1               5                   10                  15
   Ile Tyr Asn Glu Pro Glu Ser Lys Gln Arg Tyr Ser Pro Asn Ser Thr
               20                  25                  30
   Tyr Lys Ile Tyr Leu Ala Leu Met Ala Phe Asp Gln Asn Leu Leu Ser
           35                  40                  45
   Leu Asn His Thr Glu Gln Gln Trp Asp Lys His Gln Tyr Pro Phe Lys
       50                  55                  60
   Glu Trp Asn Gln Asp Gln Asn Leu Asn Ser Ser Met Lys Tyr Ser Val
   65                  70                  75                  80
   Asn Trp Tyr Tyr Glu Asn Leu Asn Lys His Leu Arg Gln Asp Glu Val
                   85                  90                  95
   Lys Ser Tyr Leu Asp Leu Ile Glu Tyr Gly Asn Glu Glu Ile Ser Gly
               100                 105                 110
   Asn Glu Asn Tyr Trp Asn Glu Ser Ser Leu Lys Ile Ser Ala Ile Glu
           115                 120                 125
   Gln Val Asn Leu Leu Lys Asn Met Lys Gln His Asn Met His Phe Asp
       130                 135                 140
   Asn Lys Ala Ile Glu Lys Val Glu Asn Ser Met Thr Leu Lys Gln Lys
   145                 150                 155                 160
   Asp Thr Tyr Lys Tyr Val Gly Lys Thr Gly Thr Gly Ile Val Asn His
                   165                 170                 175
   Lys Glu Ala Asn Gly Trp Phe Val Gly Tyr Val Glu Thr Lys Asp Asn
               180                 185                 190
   Thr Tyr Tyr Phe Ala Thr His Leu Lys Gly Glu Asp Asn Ala Asn Gly
           195                 200                 205
   Glu Lys Ala Gln Gln Ile Ser Glu Arg Ile Leu Lys Glu Met Glu Leu
       210                 215                 220
   Ile
   225
<210> 31
   <211> 840
   <212> DNA
   <213> Staphylococcus epidermidis
<220>
   <221> CDS
   <222> (1)..(840)
   <223>
<400> 31
   aaa aag tta ata ctt tta att gca att gct tta gtt tta agt gca tgt        48
   Lys Lys Leu Ile Leu Leu Ile Ala Ile Ala Leu Val Leu Ser Ala Cys
   1               5                   10                  15
   aat tca acc agt tca cat gct aaa gag tta aat aat tta gaa aag aaa        96
   Asn Ser Thr Ser Ser His Ala Lys Glu Leu Asn Asn Leu Glu Lys Lys
               20                  25                  30
   tat aat gct aat att ggt gtc tat gca tta gat act aaa agt ggt aag       144
   Tyr Asn Ala Asn Ile Gly Val Tyr Ala Leu Asp Thr Lys Ser Gly Lys
           35                  40                  45
   gaa gta aaa ttt aat gcc gat aag aga ttt gcc tat gct tca act tca       192
   Glu Val Lys Phe Asn Ala Asp Lys Arg Phe Ala Tyr Ala Ser Thr Ser
       50                  55                  60
   aaa gcg ata aat agt gct att ttg tta gaa caa gca cct tat aat aag       240
   Lys Ala Ile Asn Ser Ala Ile Leu Leu Glu Gln Ala Pro Tyr Asn Lys
   65                  70                  75                  80
   tta aat aaa aaa gta cat att aac aaa gat gat ata gtt gct tat tct       288
   Leu Asn Lys Lys Val His Ile Asn Lys Asp Asp Ile Val Ala Tyr Ser
                   85                  90                  95
   cct att tta gaa aaa tat gta gga aaa gat atc act tta aaa gaa ctt       336
   Pro Ile Leu Glu Lys Tyr Val Gly Lys Asp Ile Thr Leu Lys Glu Leu
               100                 105                 110
   att gag gct tca atg aag tat agt gat aat aca gca aac aat aaa att       384
   Ile Glu Ala Ser Met Lys Tyr Ser Asp Asn Thr Ala Asn Asn Lys Ile
           115                 120                 125
   ata aac gaa atc ggt gga atc aaa aaa att aaa aaa cgt tta aaa aaa       432
   Ile Asn Glu Ile Gly Gly Ile Lys Lys Ile Lys Lys Arg Leu Lys Lys
       130                 135                 140
   ttg gga gat aaa gta aca aat cca gtt aga tat gaa ata gaa tta aat       480
   Leu Gly Asp Lys Val Thr Asn Pro Val Arg Tyr Glu Ile Glu Leu Asn
   145                 150                 155                 160
   tac tat tca cca aag agc aaa aaa gat act tca acg cct gct gct ttc       528
   Tyr Tyr Ser Pro Lys Ser Lys Lys Asp Thr Ser Thr Pro Ala Ala Phe
                   165                 170                 175
   ggc aag act tta aat aaa ctt atc gca aat gga aaa tta agc aaa aaa       576
   Gly Lys Thr Leu Asn Lys Leu Ile Ala Asn Gly Lys Leu Ser Lys Lys
               180                 185                 190
   aat aaa aat ttc tta ctt gat tta atg tta aat aat aaa aac gga gac       624
   Asn Lys Asn Phe Leu Leu Asp Leu Met Leu Asn Asn Lys Asn Gly Asp
           195                 200                 205
   act tta att aaa gat ggt gtt cca aaa gac tat aag gtt gct gat aaa       672
   Thr Leu Ile Lys Asp Gly Val Pro Lys Asp Tyr Lys Val Ala Asp Lys
       210                 215                 220
   agt ggt caa gca ata aca tat gct tct aga aat gat gta gct ttt att       720
   Ser Gly Gln Ala Ile Thr Tyr Ala Ser Arg Asn Asp Val Ala Phe Ile
   225                 230                 235                 240
   tat cct aag aac caa tct gaa cct att att tta gtc att ttt acg aat       768
   Tyr Pro Lys Asn Gln Ser Glu Pro Ile Ile Leu Val Ile Phe Thr Asn
                   245                 250                 255
   aaa gac aat aaa agt gat aaa cct aat gat aaa ttg ata agt gaa acc       816
   Lys Asp Asn Lys Ser Asp Lys Pro Asn Asp Lys Leu Ile Ser Glu Thr
               260                 265                 270
   gcc aag aat gta ata aac aaa ttt                                       840
   Ala Lys Asn Val Ile Asn Lys Phe
           275                 280
<210> 32
   <211> 280
   <212> PRT
   <213> Staphylococcus epidermidis
<400> 32
   Lys Lys Leu Ile Leu Leu Ile Ala Ile Ala Leu Val Leu Ser Ala Cys
   1               5                   10                  15
   Asn Ser Thr Ser Ser His Ala Lys Glu Leu Asn Asn Leu Glu Lys Lys
               20                  25                  30
   Tyr Asn Ala Asn Ile Gly Val Tyr Ala Leu Asp Thr Lys Ser Gly Lys
           35                  40                  45
   Glu Val Lys Phe Asn Ala Asp Lys Arg Phe Ala Tyr Ala Ser Thr Ser
       50                  55                  60
   Lys Ala Ile Asn Ser Ala Ile Leu Leu Glu Gln Ala Pro Tyr Asn Lys
   65                  70                  75                  80
   Leu Asn Lys Lys Val His Ile Asn Lys Asp Asp Ile Val Ala Tyr Ser
                   85                  90                  95
   Pro Ile Leu Glu Lys Tyr Val Gly Lys Asp Ile Thr Leu Lys Glu Leu
               100                 105                 110
   Ile Glu Ala Ser Met Lys Tyr Ser Asp Asn Thr Ala Asn Asn Lys Ile
           115                 120                 125
   Ile Asn Glu Ile Gly Gly Ile Lys Lys Ile Lys Lys Arg Leu Lys Lys
       130                 135                 140
   Leu Gly Asp Lys Val Thr Asn Pro Val Arg Tyr Glu Ile Glu Leu Asn
   145                 150                 155                 160
   Tyr Tyr Ser Pro Lys Ser Lys Lys Asp Thr Ser Thr Pro Ala Ala Phe
                   165                 170                 175
   Gly Lys Thr Leu Asn Lys Leu Ile Ala Asn Gly Lys Leu Ser Lys Lys
               180                 185                 190
   Asn Lys Asn Phe Leu Leu Asp Leu Met Leu Asn Asn Lys Asn Gly Asp
           195                 200                 205
   Thr Leu Ile Lys Asp Gly Val Pro Lys Asp Tyr Lys Val Ala Asp Lys
       210                 215                 220
   Ser Gly Gln Ala Ile Thr Tyr Ala Ser Arg Asn Asp Val Ala Phe Ile
   225                 230                 235                 240
   Tyr Pro Lys Asn Gln Ser Glu Pro Ile Ile Leu Val Ile Phe Thr Asn
                   245                 250                 255
   Lys Asp Asn Lys Ser Asp Lys Pro Asn Asp Lys Leu Ile Ser Glu Thr
               260                 265                 270
   Ala Lys Asn Val Ile Asn Lys Phe
           275                 280
<210> 33
   <211> 714
   <212> DNA
   <213> Staphylococcus epidermidis
<220>
   <221> CDS
   <222> (1)..(714)
   <223>
<400> 33
   tta cca gaa ggc gac ttc cct gaa act aca gaa aaa ata cct gca atg      48
   Leu Pro Glu Gly Asp Phe Pro Glu Thr Thr Glu Lys Ile Pro Ala Met
   1               5                   10                  15
   cgc aaa gca att gct aaa gca atg gtt aat tct aaa cac act gca cct      96
   Arg Lys Ala Ile Ala Lys Ala Met Val Asn Ser Lys His Thr Ala Pro
               20                  25                  30
   cat gtt aca tta atg gat gaa att gat gtg caa gaa tta tgg gat cac      144
   His Val Thr Leu Met Asp Glu Ile Asp Val Gln Glu Leu Trp Asp His
           35                  40                  45
   cgt aag aaa ttt aaa gaa att gct gct gaa caa ggt aca aaa ctt act      192
   Arg Lys Lys Phe Lys Glu Ile Ala Ala Glu Gln Gly Thr Lys Leu Thr
       50                  55                  60
   ttc tta cca tat gtt gtt aaa gca tta gtt tct gca ctt aaa aaa tat      240
   Phe Leu Pro Tyr Val Val Lys Ala Leu Val Ser Ala Leu Lys Lys Tyr
   65                  70                  75                  80
   cca gca ctt aat act tct ttc aat gaa gaa gct gga gag gtt gta cac      288
   Pro Ala Leu Asn Thr Ser Phe Asn Glu Glu Ala Gly Glu Val Val His
                   85                  90                  95
   aaa cat tac tgg aat att ggt att gct gca gat acg gat aaa gga tta      336
   Lys His Tyr Trp Asn Ile Gly Ile Ala Ala Asp Thr Asp Lys Gly Leu
               100                 105                 110
   tta gta cca gta gtt aaa cat gcc gat cgt aaa tca ata ttc gaa att      384
   Leu Val Pro Val Val Lys His Ala Asp Arg Lys Ser Ile Phe Glu Ile
           115                 120                 125
   tct gat gaa att aat gaa cta gct gta aaa gca cgt gat ggt aaa tta      432
   Ser Asp Glu Ile Asn Glu Leu Ala Val Lys Ala Arg Asp Gly Lys Leu
       130                 135                 140
   act tca gaa gaa atg aaa ggt gca aca tgc aca att agt aat atc ggt      480
   Thr Ser Glu Glu Met Lys Gly Ala Thr Cys Thr Ile Ser Asn Ile Gly
   145                 150                 155                 160
   tcc gct ggt gga caa tgg ttc act cca gtt atc aat cac cca gaa gta      528
   Ser Ala Gly Gly Gln Trp Phe Thr Pro Val Ile Asn His Pro Glu Val
                   165                 170                 175
   gct atc tta gga att ggc cgt atc gct caa aaa cct atc gtt aaa gat      576
   Ala Ile Leu Gly Ile Gly Arg Ile Ala Gln Lys Pro Ile Val Lys Asp
               180                 185                 190
   gga gaa att gta gct gca cca gtg tta gct tta tca tta agc ttt gac      624
   Gly Glu Ile Val Ala Ala Pro Val Leu Ala Leu Ser Leu Ser Phe Asp
           195                 200                 205
   cat aga caa atc gat ggt gct act gga caa aat gct atg aat cac att      672
   His Arg Gln Ile Asp Gly Ala Thr Gly Gln Asn Ala Met Asn His Ile
       210                 215                 220
   aaa cgc tta tta aat aat cca gaa tta tta tta atg gag ggg              714
   Lys Arg Leu Leu Asn Asn Pro Glu Leu Leu Leu Met Glu Gly
   225                 230                 235
<210> 34
   <211> 238
   <212> PRT
   <213> Staphylococcus epidermidis
<400> 34
   Leu Pro Glu Gly Asp Phe Pro Glu Thr Thr Glu Lys Ile Pro Ala Met
   1               5                   10                  15
   Arg Lys Ala Ile Ala Lys Ala Met Val Asn Ser Lys His Thr Ala Pro
               20                  25                  30
   His Val Thr Leu Met Asp Glu Ile Asp Val Gln Glu Leu Trp Asp His
           35                  40                  45
   Arg Lys Lys Phe Lys Glu Ile Ala Ala Glu Gln Gly Thr Lys Leu Thr
       50                  55                  60
   Phe Leu Pro Tyr Val Val Lys Ala Leu Val Ser Ala Leu Lys Lys Tyr
   65                  70                  75                  80
   Pro Ala Leu Asn Thr Ser Phe Asn Glu Glu Ala Gly Glu Val Val His
                   85                  90                  95
   Lys His Tyr Trp Asn Ile Gly Ile Ala Ala Asp Thr Asp Lys Gly Leu
               100                 105                 110
   Leu Val Pro Val Val Lys His Ala Asp Arg Lys Ser Ile Phe Glu Ile
           115                 120                 125
   Ser Asp Glu Ile Asn Glu Leu Ala Val Lys Ala Arg Asp Gly Lys Leu
       130                 135                 140
   Thr Ser Glu Glu Met Lys Gly Ala Thr Cys Thr Ile Ser Asn Ile Gly
   145                 150                 155                 160
   Ser Ala Gly Gly Gln Trp Phe Thr Pro Val Ile Asn His Pro Glu Val
                   165                 170                 175
   Ala Ile Leu Gly Ile Gly Arg Ile Ala Gln Lys Pro Ile Val Lys Asp
               180                 185                 190
   Gly Glu Ile Val Ala Ala Pro Val Leu Ala Leu Ser Leu Ser Phe Asp
           195                 200                 205
   His Arg Gln Ile Asp Gly Ala Thr Gly Gln Asn Ala Met Asn His Ile
       210                 215                 220
   Lys Arg Leu Leu Asn Asn Pro Glu Leu Leu Leu Met Glu Gly
   225                 230                 235
<210> 35
   <211> 1296
   <212> DNA
   <213> Staphylococcus epidermidis
<220>
   <221> CDS
   <222> (1) .. (1296)
   <223>
<400> 35
   gca ttt gaa ttt aga tta ccc gat atc ggg gaa ggt atc cac gaa ggt       48
   Ala Phe Glu Phe Arg Leu Pro Asp Ile Gly Glu Gly Ile His Glu Gly
   1               5                   10                  15
   gaa att gtt aaa tgg ttt att aaa gcc ggc gat aca att gaa gaa gat       96
   Glu Ile Val Lys Trp Phe Ile Lys Ala Gly Asp Thr Ile Glu Glu Asp
               20                  25                  30
   gat gta tta gca gaa gtt caa aat gat aaa tct gta gta gaa att cct      144
   Asp Val Leu Ala Glu Val Gln Asn Asp Lys Ser Val Val Glu Ile Pro
           35                  40                  45
   tct cca gta agt ggt act gtt gaa gaa gtg tta gta gat gaa gga aca      192
   Ser Pro Val Ser Gly Thr Val Glu Glu Val Leu Val Asp Glu Gly Thr
       50                  55                  60
   gtg gca gta gta gga gat gtc atc gtt aaa att gat gca cct gat gca      240
   Val Ala Val Val Gly Asp Val Ile Val Lys Ile Asp Ala Pro Asp Ala
   65                  70                  75                  80
   gaa gaa atg caa ttt aaa ggt cat ggc gat gat gag gat tct aag aaa      288
   Glu Glu Met Gln Phe Lys Gly His Gly Asp Asp Glu Asp Ser Lys Lys
                   85                  90                  95
   gaa gaa aaa gaa caa gaa tca cca gtg caa gaa gaa gct tca tca act      336
   Glu Glu Lys Glu Gln Glu Ser Pro Val Gln Glu Glu Ala Ser Ser Thr
               100                 105                 110
   caa tca caa gaa aag aca gaa gta gat gaa agt aaa act gtt aaa gcg      384
   Gln Ser Gln Glu Lys Thr Glu Val Asp Glu Ser Lys Thr Val Lys Ala
           115                 120                 125
   atg ccg tca gtg cgt aag tat gca cgt gaa aat ggt gtc aat att aaa      432
   Met Pro Ser Val Arg Lys Tyr Ala Arg Glu Asn Gly Val Asn Ile Lys
       130                 135                 140
   gct gta aat ggt tct ggt aaa aat gga cga atc aca aaa gaa gac atc      480
   Ala Val Asn Gly Ser Gly Lys Asn Gly Arg Ile Thr Lys Glu Asp Ile
   145                 150                 155                 160
   gat gca tac tta aat ggt ggt agt tcc gaa gaa ggt tca aac act agc      528
   Asp Ala Tyr Leu Asn Gly Gly Ser Ser Glu Glu Gly Ser Asn Thr Ser
                   165                 170                 175
   gta gca tct gaa tca act tct agt gat gtc gtt aat gct tct gca aca      576
   Val Ala Ser Glu Ser Thr Ser Ser Asp Val Val Asn Ala Ser Ala Thr
               180                 185                 190
   caa gca tta cca gaa ggc gac ttc cct gaa act aca gaa aaa ata cct      624
   Gln Ala Leu Pro Glu Gly Asp Phe Pro Glu Thr Thr Glu Lys Ile Pro
           195                 200                 205
   gca atg cgc aaa gca att gct aaa gca atg gtt aat tct aaa cac act      672
   Ala Met Arg Lys Ala Ile Ala Lys Ala Met Val Asn Ser Lys His Thr
       210                 215                 220
   gca cct cat gtt aca tta atg gat gaa att gat gtg caa gaa tta tgg      720
   Ala Pro His Val Thr Leu Met Asp Glu Ile Asp Val Gln Glu Leu Trp
   225                 230                 235                 240
   gat cac cgt aag aaa ttt aaa gaa att gct gct gaa caa ggt aca aaa      768
   Asp His Arg Lys Lys Phe Lys Glu Ile Ala Ala Glu Gln Gly Thr Lys
                   245                 250                 255
   ctt act ttc tta cca tat gtt gtt aaa gca tta gtt tct gca ctt aaa      816
   Leu Thr Phe Leu Pro Tyr Val Val Lys Ala Leu Val Ser Ala Leu Lys
               260                 265                 270
   aaa tat cca gca ctt aat act tct ttc aat gaa gaa gct gga gag gtt      864
   Lys Tyr Pro Ala Leu Asn Thr Ser Phe Asn Glu Glu Ala Gly Glu Val
           275                 280                 285
   gta cac aaa cat tac tgg aat att ggt att gct gca gat acg gat aaa      912
   Val His Lys His Tyr Trp Asn Ile Gly Ile Ala Ala Asp Thr Asp Lys
       290                 295                 300
   gga tta tta gta cca gta gtt aaa cat gcc gat cgt aaa tca ata ttc      960
   Gly Leu Leu Val Pro Val Val Lys His Ala Asp Arg Lys Ser Ile Phe
   305                 310                 315                 320
   gaa att tct gat gaa att aat gaa cta gct gta aaa gca cgt gat ggt     1008
   Glu Ile Ser Asp Glu Ile Asn Glu Leu Ala Val Lys Ala Arg Asp Gly
                   325                 330                 335
   aaa tta act tca gaa gaa atg aaa ggt gca aca tgc aca att agt aat      1056
   Lys Leu Thr Ser Glu Glu Met Lys Gly Ala Thr Cys Thr Ile Ser Asn
               340                 345                 350
   atc ggt tcc gct ggt gga caa tgg ttc act cca gtt atc aat cac cca      1104
   Ile Gly Ser Ala Gly Gly Gln Trp Phe Thr Pro Val Ile Asn His Pro
           355                 360                 365
   gaa gta gct atc tta gga att ggc cgt atc gct caa aaa cct atc gtt      1152
   Glu Val Ala Ile Leu Gly Ile Gly Arg Ile Ala Gln Lys Pro Ile Val
       370                 375                 380
   aaa gat gga gaa att gta gct gca cca gtg tta gct tta tca tta agc      1200
   Lys Asp Gly Glu Ile Val Ala Ala Pro Val Leu Ala Leu Ser Leu Ser
   385                 390                 395                 400
   ttt gac cat aga caa atc gat ggt gct act gga caa aat gct atg aat      1248
   Phe Asp His Arg Gln Ile Asp Gly Ala Thr Gly Gln Asn Ala Met Asn
                   405                 410                 415
   cac att aaa cgc tta tta aat aat cca gaa tta tta tta atg gag ggg      1296
   His Ile Lys Arg Leu Leu Asn Asn Pro Glu Leu Leu Leu Met Glu Gly
               420                 425                 430
<210> 36
   <211> 432
   <212> PRT
   <213> Staphylococcus epidermidis
<400> 36
   Ala Phe Glu Phe Arg Leu Pro Asp Ile Gly Glu Gly Ile His Glu Gly
   1               5                   10                  15
   Glu Ile Val Lys Trp Phe Ile Lys Ala Gly Asp Thr Ile Glu Glu Asp
               20                  25                  30
   Asp Val Leu Ala Glu Val Gln Asn Asp Lys Ser Val Val Glu Ile Pro
           35                  40                  45
   Ser Pro Val Ser Gly Thr Val Glu Glu Val Leu Val Asp Glu Gly Thr
       50                  55                  60
   Val Ala Val Val Gly Asp Val Ile Val Lys Ile Asp Ala Pro Asp Ala
   65                  70                  75                  80
   Glu Glu Met Gln Phe Lys Gly His Gly Asp Asp Glu Asp Ser Lys Lys
                   85                  90                  95
   Glu Glu Lys Glu Gln Glu Ser Pro Val Gln Glu Glu Ala Ser Ser Thr
   100 105 110
   Gln Ser Gln Glu Lys Thr Glu Val Asp Glu Ser Lys Thr Val Lys Ala
   115 120 125
   Met Pro Ser Val Arg Lys Tyr Ala Arg Glu Asn Gly Val Asn Ile Lys
   130 135 140
   Ala Val Asn Gly Ser Gly Lys Asn Gly Arg Ile Thr Lys Glu Asp Ile
   145 150 155 160
   Asp Ala Tyr Leu Asn Gly Gly Ser Ser Glu Glu Gly Ser Asn Thr Ser
   165 170 175
   Val Ala Ser Glu Ser Thr Ser Ser Asp Val Val Asn Ala Ser Ala Thr
   180 185 190
   Gln Ala Leu Pro Glu Gly Asp Phe Pro Glu Thr Thr Glu Lys Ile Pro
   195 200 205
   Ala Met Arg Lys Ala Ile Ala Lys Ala Met Val Asn Ser Lys His Thr
   210 215 220
   Ala Pro His Val Thr Leu Met Asp Glu Ile Asp Val Gln Glu Leu Trp
   225 230 235 240
   Asp His Arg Lys Lys Phe Lys Glu Ile Ala Ala Glu Gln Gly Thr Lys
   245 250 255
   Leu Thr Phe Leu Pro Tyr Val Val Lys Ala Leu Val Ser Ala Leu Lys
   260 265 270
   Lys Tyr Pro Ala Leu Asn Thr Ser Phe Asn Glu Glu Ala Gly Glu Val
   275 280 285
   Val His Lys His Tyr Trp Asn Ile Gly Ile Ala Ala Asp Thr Asp Lys
   290 295 300
   Gly Leu Leu Val Pro Val Val Lys His Ala Asp Arg Lys Ser Ile Phe
   305 310 315 320
   Glu Ile Ser Asp Glu Ile Asn Glu Leu Ala Val Lys Ala Arg Asp Gly
   325 330 335
   Lys Leu Thr Ser Glu Glu Met Lys Gly Ala Thr Cys Thr Ile Ser Asn
               340                 345                 350
   Ile Gly Ser Ala Gly Gly Gln Trp Phe Thr Pro Val Ile Asn His Pro
           355                 360                 365
   Glu Val Ala Ile Leu Gly Ile Gly Arg Ile Ala Gln Lys Pro Ile Val
       370                 375                 380
   Lys Asp Gly Glu Ile Val Ala Ala Pro Val Leu Ala Leu Ser Leu Ser
   385                 390                 395                 400
   Phe Asp His Arg Gln Ile Asp Gly Ala Thr Gly Gln Asn Ala Met Asn
                   405                 410                 415
   His Ile Lys Arg Leu Leu Asn Asn Pro Glu Leu Leu Leu Met Glu Gly
               420                 425                 430